# EUROPEAN PATENT SPECIFICATION

(11) **EP 3 371 217 B1**
(45) Date of publication and mention of the grant of the patent: **11.06.2025**
(21) Application number: 16798062.2
(22) Date of filing: 08.11.2016
(51) Int. Cl.: C07K 16/22, C07K 16/28, C07K 16/46, C07K 16/40

(54) **METHODS OF SCREENING FOR MULTISPECIFIC ANTIBODIES**
SCREENING VERFAHREN FÜR MULTISPEZIFISCHE ANTIKÖRPER
DES PROCÉDÉS DE CRIBLAGE D'ANTICORPS MULTISPÉCIFIQUES

(30) Priority: 08.11.2015 US 201562252549 P
(43) Date of publication of application: 12.09.2018
(73) Proprietor: F. Hoffmann-La Roche AG, 4070 Basel (CH)
(72) Inventor: DUNSHEE, Diana Ronai, South San Francisco, CA 94080-4990 (US); HONGO, Joanne, South San Francisco, CA 94080-4990 (US); KIM, Hok Seon, South San Francisco, CA 94080-4990 (US); SONODA, Junichiro, South San Francisco, CA 94080-4990 (US); SPIESS, Christoph, South San Francisco, CA 94080-4990 (US); YEE, Angie Grace, South San Francisco, CA 94080-4990 (US)
(74) Representative: Cueni, Leah Noëmi
(86) International application number: PCT/US2016/060986
(87) International publication number: WO 2017/079768

(56) References cited:
- WO-A1-2010/112194
- WO-A1-2012/006633
- SPIESS CHRISTOPH ET AL: "Alternative molecular formats and therapeutic applications for bispecific antibodies", MOLECULAR IMMUNOLOGY, PERGAMON, GB, vol. 67, no. 2, 27 January 2015 (2015-01-27), pages 95 - 106, XP029246892, ISSN: 0161-5890, DOI: 10.1016/J.MOLIMM.2015.01.003
- KIM YU-JIN ET AL: "Improving the productivity of single-chain Fv antibody against c-Met by rearranging the order of its variable domains", JOURNAL OF MICROBIOLOGY AND BIOTECHNOLOGY, KOREAN SOCIETY FOR APPLIED MICROBIOLOGY, SEOUL, KR, vol. 18, no. 6, 1 June 2008 (2008-06-01), pages 1186 - 1190, XP009121999, ISSN: 1017-7825
- LUO D ET AL: "VL-LINKER-VH ORIENTATION-DEPENDENT EXPRESSION OF SINGLE CHAIN FV CONTAINING AN ENGINEERED DISULFIDE-STABILIZED BOND IN THE FRAMEWORK REGIONS", JOURNAL OF BIOCHEMISTRY, OXFORD UNIVERSITY PRESS, GB, vol. 118, no. 4, 1 October 1995 (1995-10-01), pages 825 - 831, XP002008023, ISSN: 0021-924X
- ZUO ZHUANG ET AL: "An efficient route to the production of an IgG-like bispecific antibody", PROTEIN ENGINEERING, OXFORD UNIVERSITY PRESS, SURREY, GB, vol. 13, no. 5, 1 May 2000 (2000-05-01), pages 361 - 367, XP002206534, ISSN: 0269-2139, DOI: 10.1093/PROTEIN/13.5.361
- R. ASANO ET AL: "Highly Effective Recombinant Format of a Humanized IgG-like Bispecific Antibody for Cancer Immunotherapy with Retargeting of Lymphocytes to Tumor Cells", JOURNAL OF BIOLOGICAL CHEMISTRY, vol. 282, no. 38, 1 January 2007 (2007-01-01), pages 27659 - 27665, XP055043823, ISSN: 0021-9258, DOI: 10.1074/jbc.M704719200
- CHEN XIAOYING ET AL: "Fusion protein linkers: Property, design and functionality", ADVANCED DRUG DELIVERY REVIEWS, vol. 65, no. 10, 15 October 2013 (2013-10-15), pages 1357 - 1369, XP028737352, ISSN: 0169-409X, DOI: 10.1016/J.ADDR.2012.09.039
- KLEIN CHRISTIAN ET AL: "Progress in overcoming the chain association issue in bispecific heterodimeric IgG antibodies", MABS, LANDES BIOSCIENCE, US, vol. 4, no. 6, 1 November 2012 (2012-11-01), pages 653 - 663, XP009170958, ISSN: 1942-0862, DOI: 10.4161/MABS.21379

## Description

### FIELD OF INVENTION

The presently disclosed subject matter relates to multispecific antibodies, e.g., bispecific antibodies, and methods of screening for such antibodies. The presently disclosed subject matter further provides antibodies that bind to Klotho-beta, and these antibodies for use in methods of treating diseases.

### BACKGROUND

Multispecific antibodies, such as bispecific antibodies, are important as research tools, diagnostic tools and as therapeutics. This is due, in large part, to the fact that such antibodies can be selected to bind with high specificity and affinity to two or more antigens or two or more epitopes present on an antigen. For example, in the case of cancer therapeutics, multispecific antibodies can be used to target a cancer cell, *e.g.,* by binding an antigen present on the cancer cell, to an immune cell to trigger an immune response. In addition, multispecific antibodies can be used as ligands for heterodimeric receptors that are normally activated by their cognate ligand when it binds to and promotes interaction between the components of the receptor.

Multispecific antibodies have traditionally been identified and produced by chemically linking fragments of antibodies such as monoclonal antibodies that possess the desired binding properties. This procedure can require the generation and recovery of specific antibody fragments, coupling the fragments using cross-linking agents or other moieties that interact and linking the antibody fragments to generate heterodimers. Recombinant DNA techniques have also been used, by coexpressing two heavy chain-light chain pairs, where the two heavy chain-light chains have different binding specificities. However, because of the random assortment of immunoglobulin heavy and light chains, this approach produces a potential mixture of different antibody molecules, of which only a small portion of the molecules have the desired multispecific, e.g., bispecific, structure. For example, using these methods, homodimers may be produced and heavy chains may pair with the wrong light chains to produce antibodies that do not have the desired specificity for the antigens or epitopes of interest. A way of providing bispecificity to IgG-like antibodies while avoiding chain mispairing is to attach single-chain Fv or Fab fragments of different specificity to them, which results in non-natural antibody structures, however (see e.g. Zuo et al. (2000) Protein Engineering 13(5):361-367; Asano et al. (2007) Journal of Biological Chemistry 282(38):27659-27665; Spiess et al. (2015) Molecular Immunology 67:95-106; or WO 2010/112194). Accordingly, there is a need in the art for improved methods for the identification and selection of multispecific antibodies, e.g., bispecific and biepitopic antibodies, against certain antigens and/or epitopes of interest.

Multispecific antibodies, e.g., bispecific antibodies, have been found to be useful in functioning as agonists of receptors *(see, e.g.,* Weidle et al. (2013) Cancer Genomics Proteomics 10(1):1-18). One such receptor that can benefit from such antibodies is Klotho-beta (KLB). KLB is a 114-kDa type 1 transmembrane protein with a short intracellular domain and two extracellular glycosidase domains that lack a characteristic glutamic acid residue essential for enzymatic activity (Ito et al. (2000) Mech. Dev. 98:115-119). Of the 7 primary isoforms of the fibroblast growth factor receptors (FGFRs) encoded by mammalian species (1b, 2b, 3b, 1c, 2c, 3c, and 4), FGFR1c, 2c and 3c can interact with Klotho-beta, which acts as a co-receptor, to form functional receptor complexes for certain FGF ligands. For example, FGFR1c present in complexes with Klotho-beta appears to play a predominant role in mediating the metabolic effect of Fibroblast growth factor 21 (FGF21) (Ogawa et al. (2007) Proc. Natl. Acad. Sci. USA 104(18):7432-37; US2010/0184665). FGF21 was identified as a potent disease-modifying protein agent to reverse obesity and type 2 diabetes in animal disease models (Kharitonenkov et al. (2005) J. Clin. Invest. 115(6): 1627-35). Recombinant FGF21 has been shown to reduce hepatic lipids, improves insulin sensitivity, and normalize glycemic control in leptin-signaling-deficient (ob/ob or db/db) mice or high-fat diet (HFD)-fed mice. Reduction in blood glucose and improvements in various cardiovascular risk factors have also been observed in obese and diabetic rhesus monkeys treated daily with recombinant FGF21.

Therapeutic agents targeting FGFR1c and KLB has been an area of intense research. An antibody antagonist specific for FGFR1c was reported to induce weight loss in mice and monkeys (WO2005/037235) and agonistic antibody-mediated selective activation of FGFR1c was sufficient to recapitulate the insulin sensitization by FGF21 in diabetic mice (WO2012/158704; Wu et al. (2011) Sci. Trans. Med. 3(113):1-10). Antibodies that bind to the KLB/FGFR1c complex have been proposed as activators/therapeutic agents (US 7,537,903; WO2011/071783; WO2012/158704). Others have investigated two alternative approaches to selectively activate FGFR1c/ Klotho-beta complex. Foltz et al. (2012) Sci. Transl. Med. 4: 162ra153 discloses a high affinity anti-Klotho-beta antibody called mimAb1 and US Patent No. 8,372,952 discloses a bispecific anti-FGFR1/Klotho-beta Avimer polypeptide C3201 linked to human serum albumin (HSA). Given the significant role for Klotho-beta in glucose metabolism and in metabolic diseases, there remains a need in the art to develop therapeutic antibodies and methods to modulate KLB activity.

### SUMMARY

The presently disclosed subject matter provides multispecific antibodies, e.g., bispecific and biepitopic antibodies, and methods of screening for such compounds. The present disclosure further provides methods for producing and analyzing such antibodies. In certain aspect, the presently disclosed subject matter further relates to anti-KLB antibodies. In particular, the present disclosure provides bispecific antibodies that specifically bind to at least two different epitopes present on KLB, and the bispecific antibodies or mixtures of each monospecific antibody for use in treating diseases in a subject. The present invention is defined by the appended claims. The disclosure herein may in some respects go beyond the disclosure of the invention per se, and may also provide technical background for related technical developments. It will be appreciated that the additional technical background provided is not intended to define the present invention as such (which is defined by the appended claims), but rather to place it in a broader technical context. Accordingly, it will be appreciated that the terms "embodiments" or "aspects" reflect specific details of the disclosure, but insofar as they refer to a part of the additional technical background are not intended to define as part of the invention subject-matter that does not fall within the scope of the appended claims.

In certain embodiments, an isolated multispecific antibody, e.g., bispecific antibody, of the present disclosure comprises a first antigen-binding polypeptide, where the first antigen-binding polypeptide comprises a VL domain, a linker, a VH domain, a CH1 domain, a CH2 domain and a CH3 domain positioned in an N-terminal to C-terminal direction. In certain embodiments, the bispecific antibody comprises a VLfH (variable light chain full heavy) format. In certain embodiments, the components of the first antigen-binding polypeptide are positioned in an N-terminal to C-terminal direction in the following sequential order: VL-linker-VH-CH1-CH2-CH3. In certain embodiments, the multispecific antibody does not comprise a CL domain. Alternatively, in certain embodiments, the multispecific antibody can further comprise a CL domain. In certain embodiments, the bispecific antibody comprises a tcIgG (tri-chain IgG) format. In certain embodiments, the CL domain can be linked to the first antigen-binding polypeptide by one or more disulfide bridges. In certain embodiments, the disulfide bridge between the CL domain and the first antigen-binding polypeptide links the CL domain with the CH1 domain of the first antigen-binding polypeptide.

In certain embodiments, a multispecific antibody, e.g., a bispecific antibody, of the present disclosure can further comprise a second antigen-binding polypeptide, where the second antigen-binding polypeptide comprises a VL domain, a linker, a VH domain, a CH1 domain, a CH2 domain and a CH3 domain positioned in an N-terminal to C-terminal direction. In certain embodiments, the components of the second antigen-binding polypeptide are positioned in an N-terminal to C-terminal direction in the following sequential order: VL-linker-VH-CH1-CH2-CH3. In certain embodiments, the second antigen-binding polypeptide does not comprise a CL domain.

In certain embodiments, the multispecific antibody comprises two CL domains. In certain embodiments, the two CL domains are the same. In certain embodiments, one of the two CL domains is linked to the first antigen-binding polypeptide by one or more disulfide bridges, and the second of the two CL domains is linked to the second antigen-binding polypeptide by one or more disulfide bridges. In certain embodiments, the disulfide bridges link the CL domains with the CH1 domains of the first antigen-binding polypeptide and second antigen-binding polypeptide.

In certain embodiments where a multispecific antibody of the present disclosure comprises a first antigen-binding polypeptide and a second antigen-binding polypeptide, the first and second antigen-binding polypeptides bind to two different epitopes on the same antigen. Alternatively, the first and second antigen-binding polypeptides bind two different antigens. In certain embodiments, the multispecific antibody comprises a tcBsIgG format. In certain embodiments, the multispecific antibody can be a multispecific agonist antibody or multispecific antagonist antibody.

The presently disclosed antibodies can be multispecific agonist antibodies or multispecific antagonist antibodies. In certain embodiments, a multispecific antibody of the presently disclosed subject matter is a bispecific antibody, e.g., a biepitopic antibody for a target or bispecific antibody for two different targets. In certain embodiments, the bispecific antibody is an agonistic biepitopic antibody. In certain embodiments, the bispecific antibody is an antagonistic biepitopic antibody.

In certain embodiments, the presently disclosed subject matter provides an isolated biepitopic agonist antibody comprising a first antigen-binding polypeptide and a second antigen-binding polypeptide, wherein each of the first and second antigen-binding polypeptides comprise a VL domain, a linker, a VH domain, a CH1 domain, a CH2 domain and a CH3 domain positioned in an N-terminal to C-terminal direction in the following sequence VL-linker-VH-CH1-CH2-CH3. In certain embodiments, the first and second antigen-binding polypeptides of the biepitopic agonist antibody bind to two different epitopes on the same antigen.

In certain embodiments, the biepitopic agonist antibody does not comprise a CL domain. In certain embodiments, the biepitopic agonist antibody further comprises two CL domains. In certain embodiments, the CL domain is not covalently linked to the first or second antigen-binding polypeptide. In certain embodiments, the CL domain is not covalently linked to the VL domain. In certain embodiments, the VL and CL are separated. In certain embodiments, the separation can be at any junction between VL and CL. In certain embodiments, the junction can be at T109 *(i.e.,* CL can start at T109, and VL can end at R108). In certain other embodiments, the CL can start from V110.

In certain embodiments, the two CL domains are the same. In certain embodiments, the two CL domains of the biepitopic agonist antibody is linked to the first antigen-binding polypeptide by one or more disulfide bridges, and the second of the two CL domains is linked to the second antigen-binding polypeptide by one or more disulfide bridges. In certain embodiments, the disulfide bridges link the CL domains with the CH1 domains of the first antigen-binding polypeptide and second antigen-binding polypeptide.

In certain embodiments, the linker present within an antigen-binding polypeptide of a disclosed antibody can comprise glycine (G) and serine (S) residues. In certain embodiments, the linker has a length from about 1 to about 50 amino acids. In certain embodiments, the linker is about 20 amino acids in length. In certain embodiments, the linker comprises G4S repeats. In certain embodiments, the linker comprises the amino acid sequence of SEQ ID NO: 68. In certain embodiments, the linker is cleavable.

In certain embodiments, the CH3 domain of the first antigen-binding polypeptide and the CH3 domain of the second antigen-binding polypeptide of a disclosed multispecific antibody meet at an interface that is altered to promote the formation of a multispecific antibody. In certain embodiments, one or more amino acid residues of the CH3 domain of the first antigen-binding polypeptide is replaced with one or more amino acid residues having a larger side chain volume to generate a protuberance on a surface of the CH3 domain of the first antigen-binding polypeptide. In certain embodiments, one or more amino acid residues of the CH3 domain of the second antigen-binding polypeptide is replaced with one or more amino acid residues having a smaller side chain volume to generate a cavity on the surface of a CH3 domain of the second antigen-binding polypeptide that interacts with the protuberance on the surface of the CH3 domain of the first antigen-binding polypeptide. In certain embodiments, the amino acid residues having a larger side chain volume can include arginine (R), phenylalanine (F), tyrosine (Y) or tryptophan (W). In certain embodiments, the amino acid residue having a smaller side chain volume can include alanine (A), serine (S), threonine (T) or valine (V). In certain embodiments, the knob, *e.g.,* protuberance, mutation can comprise T366W (EU numbering). In certain embodiments, the hole, *e.g.,* cavity, mutation(s) can comprise at least one, at least two or all of T366S, L368A and Y407V (EU numbering). In certain embodiments, the multispecific antibody or biepitopic antibody of the presently disclosed subject matter is of an IgG, IgA or IgE isotype. In certain embodiments, the multispecific antibody or biepitopic antibody of the presently disclosed subject matter is of an IgG isotype. In certain embodiments, the multispecific antibody or biepitopic antibody is of an IgG₁, IgG₂ or IgG₄ isotype. In certain embodiments, the biepitopic antibody is a biepitopic agonist antibody.

The presently disclosed subject matter further provides an isolated nucleic acid comprising a sequence that encodes a multispecific antibody or biepitopic antibody of the present disclosure. In certain embodiments, the biepitopic antibody is a biepitopic agonist antibody. The presently disclosed subject matter further provides a vector comprising the disclosed nucleic acids.

The presently disclosed subject matter further provides a host cell that expresses a multispecific antibody or biepitopic antibody of the present disclosure. In certain embodiments, the present disclosure provides host cells comprising one or more nucleic acids that encode the multispecific antibody or biepitopic antibody. For example, and not by way of limitation, the host cell can comprise a nucleic acid that encodes a first antigen-binding polypeptide and a separate nucleic acid that encodes a second antigen-binding polypeptide. Alternatively, the host cell can comprise a nucleic acid that encodes a first antigen-binding polypeptide and a second antigen-binding polypeptide. In certain embodiments, the host cell further comprises a separate nucleic acid, *e.g.,* a second nucleic acid or a third nucleic acid, that encodes a CL domain. In certain embodiments, the biepitopic antibody is a biepitopic agonist antibody.

The presently disclosed subject matter further provides a method of producing a multispecific antibody or biepitopic antibody, *e.g.,* a biepitopic agonist antibody. In certain embodiments, the method comprises culturing a host cell under conditions sufficient for producing the multispecific antibody or biepitopic antibody. In certain embodiments, the method can further include recovering the multispecific antibody or biepitopic antibody from the culture, *e.g.,* by purification techniques. In certain embodiments, the biepitopic antibody is a biepitopic agonist antibody.

In another aspect, the presently disclosed subject matter further provides a pharmaceutical composition comprising a multispecific antibody or biepitopic antibody disclosed herein. In certain embodiments, the composition further comprises a second therapeutic agent. The presently disclosed subject matter further provides a kit comprising a multispecific antibody or biepitopic antibody disclosed herein or a composition thereof. In certain embodiments, the biepitopic antibody is a biepitopic agonist antibody.

The presently disclosed subject matter further provides a library comprising a plurality of the disclosed multispecific antibodies or biepitopic antibodies. In certain embodiments, the library can comprise a plurality of polynucleotides encoding a plurality of multispecific antibodies or biepitopic antibodies disclosed herein.

In another aspect, the presently disclosed subject matter further provides a method of screening for a multispecific antibody. For example, and not by way of limitation, the methods disclosed herein can be used to screen for a biepitopic antibody, where the antibody binds to two epitopes on the same antigen. In certain embodiments, the methods disclosed herein can be used to identify a multispecific antibody, *e.g.,* a bispecific or biepitopic antibody, that exhibits agonistic activity or antagonistic activity. In certain embodiments, the method can include (a) obtaining a plurality of multispecific antibodies from a library, (b) assaying for binding of the plurality of multispecific antibodies to a first and second antigen or a first and second epitope on the same antigen and (c) identifying the multispecific antibody that binds to the first and second antigen or the first and second epitope on the same antigen.

In certain embodiments, a method of screening for a multispecific antibody can comprise (a) expressing a multispecific antibody in a cell, (b) contacting the multispecific antibody of step (a) with a first antigen and a second antigen or a first epitope and a second epitope of the same antigen and (c) identifying the multispecific antibody that binds to the first antigen and the second antigen or the first epitope and the second epitope of the same antigen.

In a further aspect, the presently disclosed subject matter further provides a method of screening for a biepitopic agonist or antagonist antibody. In certain embodiments, the method can comprise (a) obtaining a plurality of multispecific antibodies from a library, (b) assaying for binding of the plurality of multispecific antibodies to a first and second epitope of the same antigen, (c) identifying one or more biepitopic antibodies that binds to the first and second epitope of the same antigen and (d) identifying a biepitopic antibody from the one or more biepitopic antibodies that exhibits agonistic or antagonistic activity to obtain a biepitopic agonist or antagonist antibody. In certain embodiments, the method can further comprise comparing the agonistic or antagonistic activity of the biepitopic agonist or antagonist antibody to one or more monospecific parental antibodies, singly or in combination, from which the biepitopic agonist or antagonist antibody was derived.

In certain embodiments, a method of screening for a biepitopic agonist or antagonist antibody can comprise (a) expressing a multispecific antibody in a cell, (b) contacting the multispecific antibody of step (a) with a first epitope and a second epitope of the same antigen, (c) identifying the multispecific antibody that binds to the first epitope and the second epitope of the same antigen to obtain a biepitopic antibody and (d) determining whether the biepitopic antibody exhibits agonistic or antagonistic activity to obtain a biepitopic agonist or antagonist antibody. In certain embodiments, the method can further comprise comparing the agonistic or antagonistic activity of the biepitopic agonist or antagonist antibody to one or more monospecific parental antibodies, either singly or in combination, from which the biepitopic agonist or antagonist antibody was derived.

In certain embodiments, expressing the multispecific antibody or biepitopic antibody in the cell can comprise introducing one or more nucleic acids encoding the multispecific antibody or biepitopic antibody into the cell. In certain embodiments, the multispecific antibody or biepitopic antibody is contacted with the first and second antigens or the first and second epitope of the same antigen simultaneously in step (b). In certain embodiments, the multispecific antibody or biepitopic antibody of step (b) is purified before contacting the multispecific antibody or biepitopic antibody with the first and second antigens or the first and second epitopes of the same antigen. In certain embodiments, the multispecific antibody or biepitopic antibody is purified by protein A chromatography. In certain embodiments, the antigen is present within a biological complex. In certain embodiments, the cells are prokaryotic cells, *e.g.,* Escherichia coli cells. In certain embodiments, the cells are eukaryotic cells, *e.g.,* yeast cells or mammalian cells. In certain embodiments, the mammalian cells are Chinese hamster ovary (CHO) cells. In certain embodiments, the binding of the multispecific antibody to the first and/or second antigens is analyzed by ELISA. In certain embodiments, the biepitopic antibody is a biepitopic agonist antibody.

In another aspect, the present disclosure provides bispecific, *e.g.,* biepitopic, antibodies that specifically bind to at least two epitopes present on KLB and/or mixtures of anti-KLB antibodies that specifically bind to at least two epitopes present on KLB, and the antibodies and/or mixtures for use in treating diseases in a subject. In certain embodiments, a bispecific anti-KLB antibody includes a first antibody or antigen binding portion thereof, and a second antibody or antigen binding portion thereof. For example, and not by way of limitation, the first antibody, or antigen binding portion thereof, can include a heavy chain variable region and a light chain variable region, and the second antibody, or antigen binding portion thereof, can include a heavy chain variable region and a light chain variable region, wherein the first antibody and the second antibody, or antigen binding portions thereof, bind to different epitopes present on KLB.

In certain embodiments, a bispecific anti-KLB antibody includes a heavy chain variable region that includes CDR1, CDR2 and CDR3 domains, and a light chain variable region that includes CDR1, CDR2 and CDR3 domains. In certain embodiments, a bispecific anti-KLB antibody, or an antigen-binding portion thereof, comprises a heavy chain variable region and a light chain variable region. In certain embodiments, the heavy chain variable region includes amino acids having a sequence that is at least 90%, 91%, 92%, 93%, 94%, 95%, 96%, 97%, 98%, 99% or 100% identical to the sequence set forth in SEQ ID NO: 34, 36, 38, 40 or 42. In certain embodiments, the light chain variable region includes amino acids having a sequence that is at least 90%, 91%, 92%, 93%, 94%, 95%, 96%, 97%, 98%, 99% or 100% identical to the sequence set forth in SEQ ID NO: 33, 35, 37, 39 or 41.

In certain embodiments, a bispecific anti-KLB antibody can include a heavy chain variable region having a sequence that is about 95% identical to the sequence set forth in SEQ ID NO: 38, and a light chain variable region having a sequence that is about 95% identical to the sequence set forth in SEQ ID NO: 37. In certain embodiments, the bispecific anti-KLB antibody can include a heavy chain variable region having a sequence that is about 95% identical to the sequence set forth in SEQ ID NO: 42, and a light chain variable region having a sequence that is about 95% identical to the sequence set forth in SEQ ID NO: 41.

In certain embodiments, the present disclosure provides pharmaceutical compositions that include a disclosed bispecific anti-KLB antibody and a pharmaceutically acceptable carrier. In certain embodiments, the pharmaceutical composition can include an additional therapeutic agent.

In certain embodiments, the presently disclosed subject matter provides bispecific anti-KLB antibodies for use in methods of treating metabolic disorders, *e.g.,* polycystic ovary syndrome (PCOS), metabolic syndrome (MetS), obesity, non-alcoholic steatohepatitis (NASH), non-alcoholic fatty liver disease (NAFLD), hyperlipidemia, hypertension, type 2 diabetes, non-type 2 diabetes, type 1 diabetes, latent autoimmune diabetes (LAD), and maturity onset diabetes of the young (MODY), and aging and related diseases such as Alzheimer's disease, Parkinson's disease and ALS. In certain embodiments, the bispecific anti-KLB antibody is used to treat type 2 diabetes. The method can include administering to the individual a therapeutically effective amount an antibody of the presently disclosed subject matter. In certain embodiments, the disease is diabetes, *e.g.,* type 2 diabetes. In certain embodiments, the method further includes administering an additional therapeutic agent to the individual.

In certain embodiments, the presently disclosed subject matter provides isolated nucleic acids encoding a bispecific anti-KLB antibody of the presently disclosed subject matter. In certain embodiments, the presently disclosed subject matter provides a host cell comprising a nucleic acid disclosed herein. In certain embodiments, the presently disclosed subject matter further provides a hybridoma cell that expresses a bispecific anti-KLB antibody, disclosed herein, or antigen-binding portion thereof. In certain embodiments, the presently disclosed subject matter provides a method of producing a bispecific antibody in a host cell, including transforming a host cell with a nucleic acid of the present disclosure, culturing the host cell under conditions to produce the bispecific antibody. In certain embodiments, this method further comprises recovering the antibody from the host cell.

In certain embodiments, the presently disclosed subject matter provides methods of activating a KLB-FGFR1c receptor complex in an individual, including administering to the individual an effective amount of a disclosed bispecific anti-KLB antibody.

### BRIEF DESCRIPTION OF THE FIGURES

Figures 1A-1B depict the variable light chain full heavy chain (VLfH) format used in an exemplary method in accordance with one non-limiting embodiment of the disclosed subject matter. VLfH can also be referred to herein as "Tri-chain IgG" or "tcIgG". When a tcIgG is a bispecific antibody, the tcIgG can be more specifically referred to as "tcBsIgG."
Figures 2A-2B depict liquid chromatography-mass spectrometry (LC-MS) graphs showing the heterodimerization of VLfH-formatted bispecific antibodies.
Figure 3 depicts induction of KLB-FGFR1c receptor activity by various VLfH-formatted bispecific anti-KLB antibodies that bind to two epitopes present on KLB in a GAL4-Elk1 based luciferase assay in comparison to the corresponding parental monospecific VLfH-formatted antibodies.
Figure 4 depicts induction of KLB-FGFR1c receptor activity by various bispecific anti-KLB antibodies in IgG format that bind to two epitopes present on KLB in a GAL4-Elk1 based luciferase assay in comparison to the corresponding parental monospecific antibodies.
Figure 5 depicts induction of KLB-FGFR1c receptor activity by various bispecific anti-KLB antibodies that bind to two epitopes present on KLB in a GAL4-Elk1 based luciferase assay in comparison to the corresponding parental monoclonal antibodies, an anti-KLB/anti-FGFR1c bispecific antibody (BsAb2), and trastuzumab, an isotype-matched control antibody.
Figure 6 depicts induction of KLB-FGFR1c receptor activity by various bispecific anti-KLB antibodies that bind to two epitopes present on KLB in a GAL4-Elk1 based luciferase assay in comparison to a 1:1 mixture of the corresponding parental monospecific antibodies.
Figure 7 depicts induction of KLB-FGFR1c receptor activity, but not activity of a closely-related receptor complex, KLB-FGFR2c, by anti-KLB agonistic monoclonal antibodies, using a GAL4-Elk1-based luciferase reporter assay.
Figure 8 depicts that bispecific anti-KLB antibodies induced ERK phosphorylation in human primary adipocytes.
Figure 9 depicts KLB binding of the monoclonal antibodies used to generate the presently disclosed bispecific anti-KLB antibodies using flow cytometry.
Figure 10 depicts induction of KLB/FGFR1c receptor activity by monoclonal antibodies used to generate the presently disclosed bispecific anti-KLB antibodies, using chimeric KLB and FGFR proteins.
Figures 11A-11B. (A) A summary of epitope binning for anti-KLB monoclonal antibodies used to generate bispecific antibodies and a control, anti-KLB monoclonal antibody, 8C5. (B) FACS binding with HEK293T cells expressing human/rat KLB chimeric proteins.
Figure 12 depicts an example biolayer inferometry experiment used to determine the epitope bins summarized in Figure 7. In this example, 2C12 (human IgG1) competes with 23B3 for binding to recombinant KLB, but does not compete with 28B7 or 8C5.
Figure 13 discloses the light chain variable region and heavy chain variable region sequences for the anti-KLB antibody, clone 12B8.
Figure 14 discloses the light chain variable region and heavy chain variable region sequences for the anti-KLB antibody, clone 2C12.
Figure 15 discloses the light chain variable region and heavy chain variable region sequences for the anti-KLB antibody, clone 4H7.
Figure 16 discloses the light chain variable region and heavy chain variable region sequences for the anti-KLB antibody, clone 23B3.
Figure 17 discloses the light chain variable region and heavy chain variable region sequences for the anti-KLB antibody, clone 28B7.

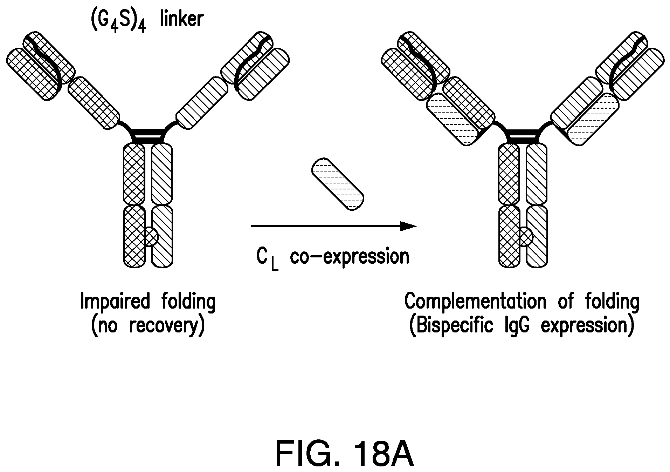

Figures 18A-18D disclose that tcBsIgG format enables bispecific antibody generation in a single cell. (A) Schematic representation of the tcBsIgG format. The antibody VL domain is tethered via a (G₄S)₄ linker to the antibody heavy chain (left). The folding defect of CH1 is complemented by CL expression in trans from a separate plasmid (right). (B) Capillary electrophoresis of expression of anti-KLB antibody, clone 28B7 monoepitopic tcIgG in the isotypes of IgG1, 2 and 4 as well as the aglycosylated (N₂₉₇G) version of IgG1 and IgG4 after protein A affinity column purification recovery. Expression as VLfH alone without the C_{L} in HECK293 cells with trace amount of or no VLfH expression (lane 1) ; VLfH co-expressed with C_{L} to result in tcIgG expression (lane 2) that is comparable to a standard IgG expression (lane 3). (C) Capillary electrophoresis of expression of anti-KLB antibody, clone 28B7 monoepitopic tcIgG after protein A affinity column purification recovery for standard IgG1, 2 and 4 and the tcIgG1, 2, and 4 counterparts as the knob half IgG (lane 1); hole half IgG (lane 2); knob and hole co-expressed IgG in single-cell. (D) Intact LC-MS/MS of anti-KLB antibody, clone 28B7 monoepitopic tcIgG after protein A and size-exclusion chromatography.
Figures 19A-19C disclose the production of anti-KLB biepitopic antibodies in the tcBsIgG format. (A) Capillary electrophoresis of co-expression of positive and negative expression control tcIgGs and five anti-KLB tcIgG half antibodies in 7x7 Knob and Hole combinations to produce tcBsIgG in a single-cell. (B) Capillary electrophoresis of parental tcIgG expression of the respective tcBsIgGs expressed in single-cell. (C) Analytical size-exclusion chromatography (SEC) of tcIgG after ProA chromatography.
Figures 20A-20C disclose the impact of antibody isotype on antibody agonist activity. (A) luciferase reporter assay showing activities of the different isotypes. (B) Impact of different antibody isotypes on ERK1/2 phosphorylation in human primary adipocytes. (C) Impact of different antibody isotypes and combinations thereof on ERK1/2 phosphorylation in human primary adipocytes.
Figure 21 discloses the binding affinity difference between the anti-KLB antibody clones 28B7 and 4H7.

### DETAILED DESCRIPTION

For clarity and not by way of limitation the detailed description of the presently disclosed subject matter is divided into the following subsections:
I. Definitions;
II. Antibodies;
III. Methods of Screening and Production;
IV. Methods of Use;
V. Pharmaceutical formulations; and
VI. Articles of Manufacture and Kits.

### I. DEFINITIONS

An "acceptor human framework," as used herein, is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In certain embodiments, the number of amino acid changes are about 10 or less, about 9 or less, about 8 or less, about 7 or less, about 6 or less, about 5 or less, about 4 or less, about 3 or less or about 2 or less. In certain embodiments, the VL acceptor human framework is identical in sequence to the VL human immunoglobulin framework sequence or human consensus framework sequence.

"Affinity," as used herein, refers to the strength of the sum total of noncovalent interactions between a single binding site of a molecule *(e.g.,* an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (K_{d}). Affinity can be measured by common methods known in the art, including those described herein. Specific illustrative and exemplary embodiments for measuring binding affinity are described in the following.

An "affinity matured" antibody refers to an antibody with one or more alterations in one or more hypervariable regions (HVRs), compared to a parent antibody, which does not possess such alterations, such alterations resulting in an improvement in the affinity of the antibody for antigen.

"Klotho-beta," "KLB" and "beta-Klotho," as used herein, refers to any native Klotho-beta from any vertebrate source, including mammals such as primates (e.g., humans) and rodents (e.g., mice and rats), unless otherwise indicated. The term encompasses "full-length," unprocessed KLB as well as any form of KLB that results from processing in the cell. The term also encompasses mutations and naturally occurring variants of KLB, *e.g.,* splice variants or allelic variants.

A non-limiting example of a human KLB amino acid sequence targeted by a bispecific antibody of the present disclosure, excluding the signal sequence, is as follows:

FSGDGRAIWSKNPNFTPVNESQLFLYDTFPKNFFWGIGTGALQVEGSWKKDGKG
PSIWDHFIHTHLKNVSSTNGSSDSYIFLEKDLSALDFIGVSFYQFSISWPRLFPDGI
VTVANAKGLQYYSTLLDALVLRNIEPIVTLYHWDLPLALQEKYGGWKNDTIIDI
FNDYATYCFQMFGDRVKYWITIHNPYLVAWHGYGTGMHAPGEKGNLAAVYT
VGHNLIKAHSKVWHNYNTHFRPHQKGWLSITLGSHWIEPNRSENTMDIFKCQQS
MVSVLGWFANPIHGDGDYPEGMRKKLFSVLPIFSEAEKHEMRGTADFFAFSFGP
NNFKPLNTMAKMGQNVSLNLREALNWIKLEYNNPRILIAENGWFTDSRVKTED
TTAIYMMKNFLSQVLQAIRLDEIRVFGYTAWSLLDGFEWQDAYTIRRGLFYVDF

NSKQKERKPKSSAHYYKQIIRENGFSLKESTPDVQGQFPCDFSWGVTESVLKPES
VASSPQFSDPHLYVWNATGNRLLHRVEGVRLKTRPAQCTDFVNIKKQLEMLAR
MKVTHYRFALDWASVLPTGNLSAVNRQALRYYRCVVSEGLKLGISAMVTLYYP
THAHLGLPEPLLHADGWLNPSTAEAFQAYAGLCFQELGDLVKLWITINEPNRLS
DIYNRSGNDTYGAAHNLLVAHALAWRLYDRQFRPSQRGAVSLSLHADWAEPA
NPYADSHWRAAERFLQFEIAWFAEPLFKTGDYPAAMREYIASKHRRGLSSSALP
RLTEAERRLLKGTVDFCALNHFTTRFVMHEQLAGSRYDSDRDIQFLQDITRLSSP
TRLAVIPWGVRKLLRWVRRNYGDMDIYITASGIDDQALEDDRLRKYYLGKYLQ
EVLKAYLIDKVRIKGYYAFKLAEEKSKPRFGFFTSDFKAKSSIQFYNKVISSRGFP
FENSSSRCSQTQENTECTVCLFLVQKKPLIFLGCCFFSTLVLLLSIAIFQRQKRRKF
WKAKNLQHIPLKKGKRVVS (SEQ ID NO: 1).

The term "C-terminal domain of KLB" refers to the carboxy-terminal glycosidase-like domain of KLB. For example, the C-terminal domain of the exemplary KLB protein shown in SEQ ID NO: 1 includes the following amino acid sequence:

FPCDFSWGVTESVLKPESVASSPQFSDPHLYVWNATGNRLLHRVEGVRLKTRPA
QCTDFVNIKKQLEMLARMKVTHYRFALDWASVLPTGNLSAVNRQALRYYRCV
VSEGLKLGISAMVTLYYPTHAHLGLPEPLLHADGWLNPSTAEAFQAYAGLCFQE
LGDLVKLWITINEPNRLSDIYNRSGNDTYGAAHNLLVAHALAWRLYDRQFRPSQ
RGAVSLSLHADWAEPANPYADSHWRAAERFLQFEIAWFAEPLFKTGDYPAAMR
EYIASKHRRGLSSSALPRLTEAERRLLKGTVDFCALNHFTTRFVMHEQLAGSRY
DSDRDIQFLQDITRLSSPTRLAVIPWGVRKLLRWVRRNYGDMDIYITASGIDDQA
LEDDRLRKYYLGKYLQEVLKAYLIDKVRIKGYYAFKLAEEKSKPRFGFFTSDFK
AKSSIQFYNKVISSRGFPFENSSSR (SEQ ID NO: 2).

An "antigen-binding polypeptide," as referred to herein, is a protein or polypeptide that comprises an antigen-binding region or antigen-binding portion, that has a strong affinity to another molecule to which it binds. Antigen-binding polypeptides encompass antibodies, or antigen-binding fragments thereof, and fusion proteins.

The term "antibody" is used herein in the broadest sense and encompasses various antibody structures, including, but not limited to, multispecific antibodies, e.g., bispecific antibodies, and antibody fragments that exhibit the desired antigen-binding activity.

An "antibody fragment," "antigen-binding portion" of an antibody (or simply "antibody portion") or "antigen-binding fragment" of an antibody, as used herein, refers to a molecule other than an intact antibody that comprises a portion of an intact antibody that binds the antigen and/or epitope to which the intact antibody binds. Examples of antibody fragments include but are not limited to Fv, Fab, Fab', Fab'-SH, F(ab')₂; diabodies; linear antibodies; single-chain antibody molecules (e.g., scFv); and antibody fragments formed from multispecific, *e.g.,* bispecific antibodies.

In certain embodiments, the terms "agonist" and "agonistic" can refer to a molecule (*e.g.,* an antigen-binding polypeptide and/or an antibody and/or antigen-binding antibody fragment) which interacts with, *e.g.,* binds to, a receptor (*e.g.,* an antigen) and is capable of initiating, mimicking and/or stimulating a reaction or activity that is similar to or the same as that initiated, mimicked and/or stimulated by the receptor's natural ligand. In certain embodiments, an agonist as described herein is capable of inducing, augmenting, enhancing and/or stimulating the activation of a signal transduction pathway associated with the receptor.

An "antibody that competes for binding" with a reference antibody refers to an antibody that blocks binding of the reference antibody to its antigen, e.g., KLB, in a competition assay by 50% or more, and conversely, the reference antibody blocks binding of the antibody to its antigen, e.g., KLB, in a competition assay by 50% or more. An exemplary competition assay is described in "Antibodies," Harlow and Lane (Cold Spring Harbor Press, Cold Spring Harbor, NY).

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively. In certain embodiments, the multispecific antibodies, e.g., bispecific antibodies, of the present disclosure can be any one of the disclosed classes or subclasses (isotypes) of antibodies.

The term "cytotoxic agent" as used herein refers to a substance that inhibits or prevents a cellular function and/or causes cell death or destruction. Cytotoxic agents include, but are not limited to, radioactive isotopes (e.g., At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors *(e.g.,* B cell receptor); and B cell activation.

The term "Fc region" herein is used to define a C-terminal region of an immunoglobulin heavy chain that contains at least a portion of the constant region. The term includes native sequence Fc regions and variant Fc regions. In certain embodiments, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full length antibody," "intact antibody," and "whole antibody," as used interchangeably herein, refers to an antibody having a structure substantially similar to a native antibody structure or having heavy chains that contain an Fc region as defined herein.

The terms "host cell," "host cell line," and "host cell culture," as used interchangeably, refers to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody," as used herein, refers to an antibody that possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "human consensus framework," as used herein, refers to a framework, which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), Vols. 1-3. In certain embodiments, for the VL, the subgroup is subgroup kappa I as in Kabat et al., *supra.* In certain embodiments, for the VH, the subgroup is subgroup III as in Kabat et al., *supra.*

A "humanized" antibody, as used herein, refers to a chimeric antibody comprising amino acid residues from non-human HVRs and amino acid residues from human FRs. In certain embodiments, a humanized antibody can comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "variable region" or "variable domain" refers to the domain of an antibody heavy or light chain that is involved in binding the antibody to an antigen. The variable domains of the heavy chain and light chain (VH and VL, respectively) of a native antibody generally have similar structures, with each domain comprising four conserved framework regions (FRs) and three hypervariable regions (HVRs). (See, *e.g.,* Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. *See, e.g.,* Portolano et al., J. Immunol. 150:880-887 (1993); Clarkson et al., Nature 352:624-628 (1991).

The term "hypervariable region" or "HVR," as used herein, refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (H1, H2, H3), and three in the VL (L1, L2, L3). Exemplary HVRs herein include:
(a) hypervariable loops occurring at amino acid residues 26-32 (L1), 50-52 (L2), 91-96 (L3), 26-32 (H1), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (L1), 46-55 (L2), 89-96 (L3), 30-35b (H1), 47-58 (H2) and 93-101 (H3) (MacCallum et al. J. Mol. Biol. 262: 732-745 (1996)); and
(d) combinations of (a), (b) and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).
Unless otherwise indicated, HVR residues and other residues in the variable domain (*e.g.,* FR residues) are numbered herein according to Kabat et al., *supra.*

An "immunoconjugate," as used herein, refers to an antibody conjugated to one or more heterologous molecule(s), including but not limited to a cytotoxic agent.

An "individual," "patient" or "subject," as used interchangeably herein, refers to a mammal. Mammals include, but are not limited to, domesticated animals (*e.g.,* cows, sheep, cats, dogs, and horses), primates (*e.g.,* humans and non-human primates such as monkeys), rabbits, and rodents (*e.g.,* mice and rats). In certain embodiments, the individual or subject is a human.

An "isolated" antibody, as used herein, refers to an antibody which has been separated from a component of its natural environment. In certain embodiments, an antibody is purified to greater than 95% or 99% purity as determined by, for example, electrophoretic (*e.g.,* SDS-PAGE, isoelectric focusing (IEF), capillary electrophoresis) or chromatographic (*e.g.,* ion exchange or reverse phase HPLC). For review of methods for assessment of antibody purity, *see, e.g.,* Flatman et al., J. Chromatogr. B 848:79-87 (2007).

An "isolated" nucleic acid, as used herein, refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an antibody" (including references to a specific antibody, *e.g.,* an anti-KLB antibody) refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

The term "monoclonal antibody," as used herein, refers to an antibody obtained from a population of substantially homogeneous antibodies, *i.e.,* the individual antibodies comprising the population are identical and/or bind the same epitope, except for possible variant antibodies, *e.g.,* containing naturally occurring mutations or arising during production of a monoclonal antibody preparation, such variants generally being present in minor amounts, or multispecific antibodies *e.g.,* antibodies that bind to at least two different epitopes. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present disclosure may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked antibody," as used herein, refers to an antibody that is not conjugated to a heterologous moiety (*e.g.,* a cytotoxic moiety) or radiolabel. The naked antibody may be present in a pharmaceutical formulation.

"Native antibodies," as used herein, refers to naturally occurring immunoglobulin molecules with varying structures. For example, but not by way of limitation, native IgG antibodies are heterotetrameric glycoproteins of about 150,000 daltons, composed of two identical light chains and two identical heavy chains that are disulfide-bonded. From N-to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain. In certain embodiments, a "CH2 domain" of a human IgG Fc region extends from about residues 231 to about 340 of the IgG. In certain embodiments, the CH3 domain comprises the stretch of residues C-terminal to a CH2 domain in an Fc region (*i.e.,* from about amino acid residue 341 to about amino acid residue 447 of an IgG).

"Hinge region," as used herein, generally refers to the amino acids Glu216 to Pro230 of human IgGl (*see* Burton, Molec. Immunol. 22: 161-206 (1985)). In certain embodiments, hinge regions of other IgG isotypes can be aligned with the IgG1 sequence by placing the first and last cysteine residues forming inter-heavy chain S-S bonds in the same positions.

The term "interface" comprises those "contact" amino acid residues (or other non-amino acid groups such as carbohydrate groups, NADH, biotin, FAD or haem group) in the first antigen-binding polypeptide which interact with one or more "contact" amino acid residues (or other non-amino acid groups) in the interface of the second antigen-binding polypeptide.

The term "package insert," as used herein, refer to instructions customarily included in commercial packages of therapeutic products, that contain information about the indications, usage, dosage, administration, combination therapy, contraindications and/or warnings concerning the use of such therapeutic products.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and
where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "pharmaceutical formulation," as used herein, refers to a preparation which is in such form as to permit the biological activity of an active ingredient contained therein to be effective, and which contains no additional components which are unacceptably toxic to a subject to which the formulation would be administered.

A "pharmaceutically acceptable carrier," as used herein, refers to an ingredient in a pharmaceutical formulation, other than an active ingredient, which is nontoxic to a subject. A pharmaceutically acceptable carrier includes, but is not limited to, a buffer, excipient, stabilizer or preservative.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In certain embodiments, antibodies of the present disclosure are used to delay development of a disease or to slow the progression of a disease.

A "therapeutically effective amount" or "effective amount" of an agent, *e.g.,* a pharmaceutical formulation, refers to an amount effective, at dosages and for periods of time necessary, to achieve the desired therapeutic or prophylactic result. For example, and not by way of limitation, a "therapeutically effective amount" or "effective amount" can refer to a dosage amount of a bispecific antibody that results in the diminishment of any direct or indirect pathological consequences of the disease, a reduction in the rate of disease progression, the amelioration of the disease state, the remission or improved prognosis, prevention of the occurrence or recurrence of the disease and/or alleviation of symptoms.

As used herein, the term "about" or "approximately" means within an acceptable error range for the particular value as determined by one of ordinary skill in the art, which will depend in part on how the value is measured or determined, *i.e.,* the limitations of the measurement system. For example, "about" can mean within 3 or more than 3 standard deviations, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value.

As described herein, any concentration range, percentage range, ratio range or integer range is to be understood to include the value of any integer within the recited range and, when appropriate, fractions thereof (such as one tenth and one hundredth of an integer), unless otherwise indicated.

### II. ANTIBODIES

The presently disclosed subject matter provides multispecific antibodies, *e.g.,* bispecific antibodies. A multispecific antibody, *e.g.,* a bispecific antibody, of the present disclosure has at least two different binding specificities. *See, e.g.,* U.S. Patent Nos. 5,922,845 and 5,837,243; Zeilder (1999) J. Immunol. 163:1246-1252; Somasundaram (1999) Hum. Antibodies 9:47-54; Keler (1997) Cancer Res. 57:4008-4014. In certain embodiments, the presently disclosed multispecific antibodies can bind to at least two different epitopes on an antigen or bind to at least two epitopes that overlap on an antigen, *e.g.,* a biepitopic antibody. Alternatively, in certain embodiments, the multispecific antibodies of the present disclosure can bind to at least two different antigens.

The term "epitope," as used herein, refers to a protein determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

### A. Multispecific antibodies

The presently disclosed subject matter provides multispecific antibodies, *e.g.,* bispecific antibodies and biepitopic antibodies, that bind to at least two epitopes, *e.g.,* overlapping or non-overlapping epitopes, present on an antigen. The presently disclosed subject matter further provides multispecific antibodies that bind to at least one epitope on a first antigen and at least one epitope on a second antigen. The presently disclosed multispecific antibodies can be agonistic antibodies or antagonistic antibodies.

In certain embodiments, the multispecific antibodies, *e.g.,* bispecific antibodies, of the present disclosure can comprise one or more antigen-binding polypeptides. For example, and not by way of limitation, a multispecific antibody of the present disclosure can include a first antigen-binding polypeptide and a second antigen-binding polypeptide. In certain embodiments, the first antigen-binding polypeptide and the second antigen-binding polypeptide have different binding specificities. For example, and not by way of limitation, the first antigen-binding polypeptide can bind to a first epitope on an antigen and the second antigen-binding polypeptide can bind to a second epitope on the antigen. Alternatively, in certain embodiments, the first antigen-binding polypeptide can bind to a first antigen and the second antigen-binding polypeptide can bind to a second antigen.

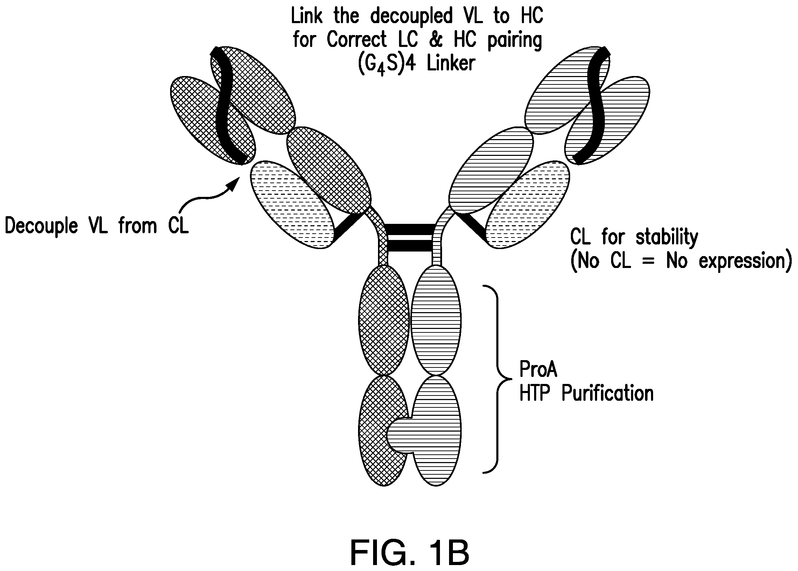

In certain embodiments, and as shown in Figure 1B, the first antigen-binding polypeptide and/or the second antigen-binding polypeptide can comprise a light chain variable region (VL), a heavy chain variable region (VH), a CH1 domain, a CH2 domain and/or a CH3 domain. In certain embodiments, an antigen-binding polypeptide of a disclosed multispecific antibody comprises a VL, a VH, a CH1 domain, a CH2 domain and a CH3 domain, where the components are positioned relative to each other in an N-terminal to C-terminal direction in the following sequence: VL-linker-VH-CH1-CH2-CH3. In certain embodiments, the first and/or second antigen-binding polypeptides can further comprise a hinge region between the CH1 and CH2 domains.

In certain embodiments, a VL domain present within an antigen-binding polypeptide (*e.g.,* a first antigen-binding polypeptide) can be joined to the VH present within the same antigen-binding polypeptide by a linker. For example, and not by way of limitation, the C-terminus of the VL domain can be linked to the N-terminus of the VH domain. In certain embodiments, where the multispecific antibody of the present disclosure comprises at least two antigen-binding polypeptides, the linkers present within each of the antigen-binding polypeptides can be the same. Alternatively, the linkers present within each of the antigen-binding polypeptides can be different.

In certain embodiments, the linker can comprise neutral, polar or nonpolar amino acids. In certain embodiments, the linker can be about 1 to about 100 amino acids in length, *e.g.,* a length between about 1 and 50 amino acids in length. For example, and not by way of limitation, a linker present within the first and/or second antigen-binding polypeptide can comprise about 1 or more, about 2 or more, about 3 or more, about 4 or more, about 5 or more, about 6 or more, about 7 or more, about 8 or more, about 9 or more, about 10 or more, about 15 or more, about 20 or more, about 25 or more, about 30 or more, about 35 or more, about 40 or more or about 45 or more amino acids. Non-limiting examples of linkers are disclosed in Shen et al., Anal. Chem. 80(6):1910-1917 (2008) and WO 2014/087010. In certain embodiments, the linker is a Gly(G)4Ser(S) linker (SEQ ID NO:70). In certain embodiments, the linker includes GGGGS repeats, *e.g.,* about 2 repeats ((GGGGS)₂) (SEQ ID NO:73), about 3 repeats ((GGGGS)₃) (SEQ ID NO:74), about 4 repeats ((GGGGS)₄) (SEQ ID NO:68), about 5 repeats ((GGGGS)₅) (SEQ ID NO:75), about 6 repeats ((GGGGS)₆) (SEQ ID NO:76) or about 7 repeats ((GGGGS)₇) (SEQ ID NO:77). Additional non-limiting examples of linkers are disclosed in Table 7. In certain embodiments, the linker comprises the amino acid sequence set forth in SEQ ID NO: 68.

In certain embodiments, the linker can be a cleavable linker, *e.g.,* auto-cleavable, enzymatically cleavable or a chemically cleavable linker. *See* WO 2011/034605. For example, and not by way of limitation, enzymatic cleavage of a linker can include the use of an endopeptidase or an exopeptidase. Non-limiting examples of endopeptidases include urokinase, Lys-C, Asp-N, Arg-C, V8, Glu-C, chymotrypsin, trypsin, pepsin, papain, thrombin, Genenase, Factor Xa, TEV (tobacco etch virus cysteine protease), Enterokinase, HRV C3 (human rhinovirus C3 protease), ininogenase, subtilisin-like proprotein convertases (*e.g.,* Furin (PC1), PC2 or PC3) and N-arginine dibasic convertase. Non-limiting examples of exopeptidases include Carboxypeptidase A, Carboxypeptidase B, Carboxypeptidase D, Carboxypeptidase E (also called Carboxypeptidase H), Carboxypeptidase M, Carboxypeptidase N or Carboxypeptidase Z. In certain embodiments, chemical cleavage can be performed by using hydroxylamine, N-chlorosuccinimide, N-bromosuccinimide or cyanogen bromide.

**Table 7**

| **Linker** | **SEQ ID NO:** |
|---|---|
| GGGGS | SEQ ID NO: 70 |
| SGGSGGS | SEQ ID NO: 71 |
| GGGGSGGGS | SEQ ID NO: 72 |
| GGGGSGGGGS | SEQ ID NO: 73 |
| GGGGSGGGGSGGGGS | SEQ ID NO: 74 |
| GGGGSGGGGSGGGGSGGGGS | SEQ ID NO: 68 |
| GGGGSGGGGSGGGGSGGGGSGGGGS | SEQ ID NO: 75 |
| GGGGSGGGGSGGGGSGGGGSGGGGSGGGGS | SEQ ID NO: 76 |
| GGGGSGGGGSGGGGSGGGGSGGGGSGGGGSGGGGS | SEQ ID NO: 77 |
| EPKSCDKTHTCPPCP | SEQ ID NO: 78 |
| GGGGSGGGSEPKSCDKTHTCPPCP | SEQ ID NO: 79 |
| ELKTPLGDTTHTCPRCPEPKSCDTPPPCPRCPEPKSCDTPPPCPRC PEPKSCDTPPPCPRCP | SEQ ID NO: 80 |
| GSGSGS | SEQ ID NO: 81 |
| AAA | SEQ ID NO: 82 |

In certain embodiments, *e.g.,* where a multispecific antibody of the present disclosure comprises a first antigen-binding polypeptide and a second antigen-binding polypeptide, the first antigen-binding polypeptide and second antigen-binding polypeptide can interact by one or more disulfide bridges. For example, and not by way of limitation, in certain embodiments, the hinge regions of the first and second antigen-binding polypeptides can interact by one or more disulfide bridges, *e.g.,* by 2 disulfide bridges.

In certain embodiments, the multispecific, *e.g.,* bispecific, antibodies can include a heterodimerization domain within each of the antigen-binding polypeptides of the antibody, as disclosed herein. In certain embodiments, the CH3 domains of the first and second antigen-binding polypeptide of a disclosed multispecific antibody can be altered to promote heterodimerization of the first and second antigen-binding polypeptides. For example, and not by way of limitation, the first and/or second antigen-binding polypeptides can include one or more heterodimerization domains using knob-in-hole technology (*see, e.g.,* U.S. Patent Nos. 5,731,168 and 8,216,805) to promote the association and/or interaction between the first antigen-binding polypeptide and the second antigen-binding polypeptide.

In certain embodiments, the first and/or second antigen-binding polypeptide can be altered to generate a protuberance or a cavity on the surface of the antigen-binding polypeptide. For example, and not by way of limitation, the first antigen-binding polypeptide can be altered to generate a protuberance on the surface of the first antigen-binding polypeptide and the second antigen-binding polypeptide can be altered to generate a cavity on the second antigen-binding polypeptide, where the protuberance of the first antigen-binding polypeptide interacts with the cavity of the second antigen-binding polypeptide to promote heterodimerization. In certain embodiments, a multispecific, e.g., bispecific, antibody of the present disclosure can comprise a first antigen-binding polypeptide and a second antigen-binding polypeptide which interact at an interface, where the first antigen-binding polypeptide has a protuberance at the interface which is positionable in a cavity at the interface of the second antigen-binding polypeptide. *See,* for example, U.S. Patent No. 8,216,805.

In certain embodiments, the region of the antigen-binding polypeptide that is altered to generate a protuberance or a cavity can be a portion of the CH3 domain. Protuberances, *e.g.,* within a CH3 domain of a first antigen-binding polypeptide, can be generated by replacing amino acid with small side chains, *e.g.,* glycine (G), alanine (A), serine (S), threonine (T) or valine (V), with amino acids with large side chains, *e.g.,* arginine (R), phenylalanine (F), tyrosine (Y) or tryptophan (W). For example, and not by way of limitation, a mutation that can be introduced into an antigen-binding polypeptide to generate a protuberance (*e.g.,* "knob") can include T366W (EU numbering). Compensatory cavities of identical or similar size to the protuberance can be generated, *e.g.,* within a CH3 domain of a second antigen-binding polypeptide, by replacing amino acids, *e.g.,* with large side chains, with amino acid with smaller side chains. For example, and not by way of limitation, mutations that can be introduced into an antigen-binding polypeptide to generate a cavity (*e.g.,* "hole") can include T366S, L368A and/or Y407V (EU numbering).

In certain embodiments, a multispecific antibody, *e.g.,* a bispecific or biepitopic antibody, can comprise a first antigen-binding polypeptide and a second antigen-binding polypeptide, where one or more amino acid residues of the CH3 domain of the first antigen-binding polypeptide is replaced with one or more amino acid residues having a larger side chain volume to generate a protuberance on a surface of the CH3 domain of the first antigen-binding polypeptide that interacts with the CH3 domain of the second antigen-binding polypeptide. In certain embodiments, one or more amino acid residues of the CH3 domain of the second antigen-binding polypeptide is replaced with one or more amino acid residues having a smaller side chain volume to generate a cavity on the surface of a CH3 domain of the second antigen-binding polypeptide that interacts with the CH3 domain of the first antigen-binding polypeptide *(see,* for example, Figure 1B).

In certain embodiments, a multispecific antibody of the present disclosure does not include a light chain constant domain (CL). Alternatively, a multispecific antibody disclosed herein can include one or more CL domains. For example, and not by way of limitation, where the multispecific antibody comprises a CL domain, the CL domain interacts with the CH1 domain by one or more disulfide bridges. In certain embodiments, where a multispecific antibody of the present disclosure comprises a first antigen-binding polypeptide and a second antigen-binding polypeptide, the multispecific antibody can further comprise two CL domains. In certain embodiments, one of the CL domains interacts with the first antigen-binding polypeptide and the other CL domain interacts with the first antigen-binding polypeptide, *e.g.,* through one or more disulfide bridges. For example, and not by way of limitation, the CL domains can interact with the CH1 domains of the antigen-binding polypeptides. In certain embodiments, the two CL domains can be the same or can be different. In certain embodiments, the CL domain is not covalently linked to the first or second antigen-binding polypeptide. In certain embodiments, the CL domain is not covalently bonded to the CH1 domain. In certain embodiments, the CL domain is not covalently bonded to the VL domain. In certain embodiments, the VL and CL are separated. In certain embodiments, the separation can be at any junction between VL and CL. In certain embodiments, the junction can be at T109 (*i.e.,* CL can start at T109, and VL can end at R108). In certain other embodiments, the CL can start from V110.

The presently disclosed subject matter further provides antagonistic and agonistic antibodies. For example, and not by way of limitation, an agonistic antibody of the present disclosure is a biepitopic antibody, where the antibody binds to two epitopes on the same antigen. In certain embodiments, the epitopes to which the biepitopic antibody binds are non-overlapping or are at least partially overlapping on the antigen. In certain embodiments, a biepitopic antibody, *e.g.,* an agonistic biepitopic antibody, of the present disclosure can include a first antigen-binding polypeptide and a second antigen-binding polypeptide, as disclosed herein. In certain embodiments, the first antigen-binding polypeptide and the second antigen-binding polypeptide have different binding specificities, where the first antigen-binding polypeptide can bind to a first epitope on an antigen and the second antigen-binding polypeptide can bind to a second epitope on the antigen. In certain embodiments, the antigen-binding polypeptides of a biepitopic agonist antibody of the presently disclosed subject matter can comprise a VL, a linker, a VH, a CH1 domain, a CH2 domain and a CH3 domain, where the components are positioned relative to each other in an N-terminal to C-terminal direction in the following sequence: VL-linker-VH-CH1-CH2-CH3. The antigen-binding polypeptides of an agonistic biepitopic antibody of the present disclosure can further comprise a heterodimerization domain within each of the polypeptides, as disclosed herein. In certain embodiments, the heterodimerization domains are generated by knob-in-hole techniques. For example, and not by way of limitation, the first antigen-binding polypeptide can have a protuberance, *e.g.,* within its CH3 domain, and the second antigen-binding polypeptide can have a cavity, *e.g.,* within its CH3 domain, which interact to promote heterodimerization.

In certain embodiments, the multispecific agnostic antibodies, *e.g.,* biepitopic agonist antibodies, of the present disclosure exhibit greater agonistic activity than the parental monospecific antibodies, individually or combined, from which the biepitopic agonist antibody is derived. For example, and not by way of limitation, a biepitopic agonist antibody, disclosed herein, exhibits greater agonistic activity than a monospecific antibody that has identical VL and VH sequences. In certain embodiments, a biepitopic agonist antibody of the present disclosure exhibits greater agonistic activity than a monospecific antibody that has identical VL, VH, CH1, CH2 and/or CH3 sequences.

In certain embodiments, the multispecific antagonistic antibodies, *e.g.,* biepitopic antagonist antibodies, of the present disclosure exhibit greater antagonistic activity than the parental monospecific antibodies, individually or combined, from which the biepitopic antagonist antibody is derived. For example, and not by way of limitation, a biepitopic antagonist antibody, disclosed herein, exhibits greater antagonistic activity than a monospecific antibody that has identical VL and VH sequences. In certain embodiments, a biepitopic antagonist antibody of the present disclosure exhibits greater antagonistic activity than a monospecific antibody that has identical VL, VH, CH1, CH2 and/or CH3 sequences.

### B. Bispecific anti-KLB antibodies

The presently disclosed subject matter further provides multispecific antibodies, e.g., bispecific and biepitopic antibodies, that bind Klotho-beta (KLB) and can function to modulate the activity of the FGFR1c/KLB receptor complex. The disclosed anti-KLB antibodies can function as agonists of the FGFR1c/KLB receptor complex, *e.g.,* to simulate the effect of ligand and/or activate the FGFR1c/KLB receptor complex.

The presently disclosed subject matter provides anti-KLB antibodies, or antigen-binding portions thereof, that bind to at least two epitopes present on a KLB protein, *e.g.,* bispecific or multispecific antibodies. For example, and not by way of limitation, the presently disclosed subject matter provides bispecific antibodies, *e.g.,* biepitopic antibodies, having one binding site (*e.g.,* antigen binding site) for a first epitope present on KLB and a second binding site for a second epitope present on KLB. In certain embodiments, the presently disclosed subject matter provides multispecific antibodies, which have at least three binding sites.

In certain embodiments, a disclosed bispecific antibody of the presently disclosed subject matter binds to two epitopes present within the glycosidase-like domain, KL1, of KLB. In certain embodiments, a disclosed bispecific antibody binds to two epitopes present within the glycosidase-like domain, KL2, of KLB. In certain embodiments, a disclosed bispecific antibody binds to two epitopes present on KLB, where one of the epitopes is present within KL1 and the second epitope is present within KL2.

In certain embodiments, a disclosed bispecific antibody of the presently disclosed subject matter binds to a fragment of KLB comprising the amino acid sequence IQFYNKVISSRGFPFENSSSRCSQTQENTECTVCLFLVQKKPLIFLGCCFFSTLVLL LSIAIFQRQKRRKFWKAKNLQHIPLKKGKRVVS (SEQ ID NO: 43) or a fragment thereof.

In certain embodiments, a disclosed bispecific antibody of the presently disclosed subject matter binds to a fragment of KLB comprising the amino acid sequence ADSHWRAAERFLQFEIAWFAEPLFKTGDYPAAMREYIASKHRRGLSSSALPRLT EAERRLLKGTVDFCALNHFTTRFVMHEQLAGSRYDSDRDI (SEQ ID NO: 44) or a fragment thereof.

In certain embodiments, a disclosed bispecific antibody of the presently disclosed subject matter binds to a fragment of KLB comprising the amino acid sequence FSGDGRAIWSKNPNFTPVNESQLFLYDTFPKNFFWGIGTGALQVEGSWKKDGKG PSIWDHFIHTHLKNVSSTNGSSDSYIFLEKDLSALDFIGVSFYQFSISWPRLFPDGI VTVANAKGLQYYSTLLDALVLRNIEPIVTLYHWDLPLALQEKYGGWKNDTIIDI FNDYATYCFQMFGDRVKYWITIHNPYLVAWHGYGTGMHAPGEKGNLAAVYT VGHNLIKAHSKVWHNYNTHFRPHQKGWLSITLGSHWIEPNRSENTMDIFKCQQS MVSVLGWFANPIHGDGDYPEGMRKKLFSVLPIFSEAEKHEMRGTADFFAFSFGP NNFKPLNTMAKMGQNVSLNLREALNWIKLEYNNPRILIAENGWFTDSRVKTED TTAIYMMKNFLSQVLQAIRLDEIRVFGYTAWSLLDGFEWQDAYTIRRGLFYVDF NSKQKERKPKSSAHYYKQIIRENGFSLKESTPDVQGQFPCDFSWGVTESVLKPES VASSPQFSDPHLYVWNATGNRLLHRVEGVRLKTRPAQCTDFVNIKKQLEMLAR MKVTHYRFALDWASVLPTGNLSAVNRQA (SEQ ID NO: 45) or a fragment thereof.

In certain embodiments, a disclosed bispecific antibody of the presently disclosed subject matter binds to one or more fragments of KLB having the amino acid sequence set forth in SEQ ID NO: 42, 43, 44 and/or 45. For example, and not by way of limitation, a bispecific anti-KLB antibody of the present disclosure can bind to one fragment of KLB that comprises the amino acid sequence set forth in SEQ ID NO: 45, or a fragment thereof, and can bind to a second fragment of KLB that comprises the amino acid sequence set forth in SEQ ID NO: 44, or a fragment thereof.

In certain embodiments, a disclosed bispecific antibody of the presently disclosed subject matter modulates KLB/FGFR1c complex activity, where FGFR1c of the complex comprises the amino acid sequence KTVALGSNVEFMCKVYSDPQPHIQWLKHIEVNGSKIGPDNLPYVQILKTAGVNT TDKEMEVLHLRNVSFEDAGEYTCLAGNSIGLSHHSAWLTVLEALEERPAVMT (SEQ ID NO: 46) or a fragment thereof.

In certain embodiments, the bispecific antibodies disclosed herein can be antibody fragments or diabodies. Diabodies are bivalent, bispecific antibodies in which the VH and VL domains are expressed on a single polypeptide chain, but using a linker that is too short to allow for pairing between the two domains on the same chain, thereby forcing the domains to pair with complementary domains of another chain and creating two antibody, *e.g.,* antigen, binding sites (*see, e.g.,* Holliger, P., et al. (1993) Proc. Natl. Acad. Sci. USA 90:6444-6448; Poljak, R.J., et al.. (1994) Structure 2:1121-1123).

In certain embodiments, a bispecific anti-KLB antibody, as disclosed herein, is an antibody that is capable of binding KLB with sufficient affinity such that the antibody is useful as a diagnostic and/or therapeutic agent in targeting KLB. In certain embodiments, the extent of binding of an anti-KLB antibody to an unrelated, non-KLB protein is less than about 10% of the binding of the antibody to KLB as measured, e.g., by a radioimmunoassay (RIA).

In certain embodiments, an anti-KLB antibody disclosed herein refers to an antibody that modulates KLB/FGFR1c complex activity. For example, the bispecific anti-KLB antibody can function as an agonist and activate the KLB/FGFR 1c complex. In certain embodiments, the bispecific anti-KLB antibody is an antibody that increases the activity of the KLB/FGFR1c complex by at least 10%, 20%, 30%, 40%, 50%, 60%, 70%, 80%, 90%, 99% or 99.9%. In certain embodiments, the bispecific anti-KLB antibody can be an antibody that results in the phosphorylation of the downstream targets of the KLB/FGFR1c complex, *e.g.,* MAPK and/or ERK.

In certain embodiments, a bispecific anti-KLB antibody, or an antigen-binding portion thereof, includes a heavy chain variable region and a light chain variable region. In certain embodiments, the heavy chain variable region includes amino acids having a sequence that is at least 90%, 91%, 92%, 93%, 94%, 95%, 96%, 97%, 98%, 99%, or 100% identical to the sequence set forth in SEQ ID NO: 34, 36, 38, 40 or 42. In certain embodiments, the light chain variable region includes amino acids having a sequence that is at least 90%, 91%, 92%, 93%, 94%, 95%, 96%, 97%, 98%, 99%, or 100% identical to the sequence set forth in SEQ ID NO: 33, 35, 37, 39 or 41.

In certain embodiments, a bispecific anti-KLB antibody can include a heavy chain variable region having a sequence that is about 95% identical to the sequence set forth in SEQ ID NO: 34 and a light chain variable region having a sequence that is about 95% identical to the sequence set forth in SEQ ID NO: 33.

In certain embodiments, a bispecific anti-KLB antibody can include a heavy chain variable region having a sequence that is about 95% identical to the sequence set forth in SEQ ID NO: 36 and a light chain variable region having a sequence that is about 95% identical to the sequence set forth in SEQ ID NO: 35.

In certain embodiments, a bispecific anti-KLB antibody can include a heavy chain variable region having a sequence that is about 95% identical to the sequence set forth in SEQ ID NO: 38 and a light chain variable region having a sequence that is about 95% identical to the sequence set forth in SEQ ID NO: 37.

In certain embodiments, a bispecific anti-KLB antibody can include a heavy chain variable region having a sequence that is about 95% identical to the sequence set forth in SEQ ID NO: 40 and a light chain variable region having a sequence that is about 95% identical to the sequence set forth in SEQ ID NO: 39.

In certain embodiments, a bispecific anti-KLB antibody can include a heavy chain variable region having a sequence that is about 95% identical to the sequence set forth in SEQ ID NO: 42 and a light chain variable region having a sequence that is about 95% identical to the sequence set forth in SEQ ID NO: 41.

In certain embodiments, a bispecific anti-KLB antibody includes a heavy chain variable region that comprises CDR1, CDR2, and CDR3 domains, and a light chain variable region that comprises CDR1, CDR2, and CDR3 domains. In certain embodiments, the heavy chain variable region CDR1 domain includes an amino acid sequence having a sequence set forth in SEQ ID NO: 3, 4, 5, 6 or 7. In certain embodiments, the heavy chain variable region CDR2 domain includes an amino acid sequence having a sequence set forth in SEQ ID NO: 8, 9, 10, 11 or 12. In certain embodiments, the heavy chain variable region CDR3 domain includes an amino acid sequence having a sequence set forth in SEQ ID NO: 13, 14, 15, 16 or 17. In certain embodiments, the light chain variable region CDR1 domain includes an amino acid sequence having a sequence set forth in SEQ ID NO: 18, 19, 20, 21 and 22. In certain embodiments, the light chain variable region CDR2 domain includes an amino acid sequence having a sequence set forth in SEQ ID NO: 23, 24, 25, 26 or 27. In certain embodiments, the light chain variable region CDR3 domain includes an amino acid sequence having a sequence set forth in SEQ ID NO: 28, 29, 30, 31 or 32.

In certain embodiments, a bispecific anti-KLB antibody includes a heavy chain variable region CDR1 having the sequence set forth in SEQ ID NO: 7; a heavy chain variable region CDR2 having the sequence set forth in SEQ ID NO: 12; a heavy chain variable region CDR3 having the sequence set forth in SEQ ID NO: 17; a light chain variable region CDR1 having the sequence set forth in SEQ ID NO: 22; a light chain variable region CDR2 having the sequence set forth in SEQ ID NO: 27; and a light chain variable region CDR3 having the sequence set forth in SEQ ID NO: 32.

In certain embodiments, a bispecific anti-KLB antibody includes a first antibody, or antigen binding portion thereof, and a second antibody, or antigen binding portion thereof, where the first antibody, or antigen binding portion thereof, and the second antibody, or antigen binding portion thereof, bind to different epitopes present on KLB. For example, and not by way of limitation, the first antibody, or antigen binding portion thereof, can include a heavy chain variable region and a light chain variable region; and the second antibody, or antigen binding portion thereof, can include a heavy chain variable region and a light chain variable region. In certain embodiments, the heavy chain variable region of the first and/or second antibody or antigen binding portion thereof includes amino acids having a sequence that is at least 90%, 91%, 92%, 93%, 94%, 95%, 96%, 97%, 98%, 99%, or 100% identical to the sequence set forth in SEQ ID NO: 34, 36, 38, 40 or 42. In certain embodiments, the light chain variable region of the first and/or second antibody, or antigen binding portions thereof, includes amino acids having a sequence set forth in SEQ ID NO: 33, 35, 37, 39 or 41.

For example, but not by way of limitation, a bispecific anti-KLB antibody can include a first antibody, or antigen binding portion thereof, comprising a heavy chain variable region having an amino acid sequence that is at least 95% identical to the sequence set forth in SEQ ID NO: 40 and a light chain variable region having an amino acid sequence that is at least 95% identical to the sequence set forth in SEQ ID NO: 39; and a second antibody, or antigen binding portion thereof, comprising a heavy chain variable region having an amino acid sequence that is at least 95% identical to the sequence set forth in SEQ ID NO: 42 and a light chain variable region having an amino acid sequence that is at least 95% identical to the sequence set forth in SEQ ID NO: 41.

In certain embodiments, a bispecific anti-KLB antibody can include a first antibody, or antigen binding portion thereof, comprising a heavy chain variable region having a sequence that is about 95% identical to the sequence set forth in SEQ ID NO: 34 and a light chain variable region having a sequence that is about 95% identical to the sequence set forth in SEQ ID NO: 33; and a second antibody, or antigen binding portion thereof, comprising a heavy chain variable region having an amino acid sequence that is at least 95% identical to the sequence set forth in SEQ ID NO: 40 and a light chain variable region having an amino acid sequence that is at least 95% identical to the sequence set forth in SEQ ID NO: 39.

### 1. Antibody Affinity

In certain embodiments, the multispecific and bispecific antibodies provided herein can have a dissociation constant (K_{d}) from about 0.001 nM to about 1µM or from about 10⁻⁸ M to about 10⁻¹³ M. For example, and not by way of limitation, a multispecific antibody, *e.g.,* a bispecific antibody, can have a K_{d} ≤ 10 nM, ≤ 1 nM, ≤ 0.1 nM, ≤ 0.01 nM or ≤ 0.001 nM. In certain embodiments, the multispecific antibody can have a K_{d} of about 10⁻⁸ M or less, from about 10⁻⁹ M or less, about 10⁻¹⁰ M or less, about 10⁻¹¹ M or less, about 10⁻¹² M or about 10⁻¹³ M or less to.

In certain embodiments, K_{d} can be measured by a radiolabeled antigen-binding assay (RIA). In certain embodiments, an RIA is performed with the F(ab)₂ version of a bispecific antibody of interest and its two antigens. For example, solution binding affinity of F(ab)₂ for antigen is measured by equilibrating F(ab)₂ with a minimal concentration of (¹²⁵I)-labeled antigen in the presence of a titration series of unlabeled antigen, then capturing bound antigen with an anti-Fab antibody-coated plate (*see, e.g.,* Chen et al., J. Mol. Biol. 293:865-881(1999)). To establish conditions for the assay, MICROTITER^{®} multi-well plates (Thermo Scientific) are coated overnight with 5 µg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [¹²⁵I]-antigen are mixed with serial dilutions of a Fab of interest (*e.g.,* consistent with assessment of the anti-VEGF antibody, Fab-12, in Presta et al., Cancer Res. 57:4593-4599 (1997)). The Fab of interest is then incubated overnight; however, the incubation may continue for a longer period (*e.g.,* about 65 hours) to ensure that equilibrium is reached. Thereafter, the mixtures are transferred to the capture plate for incubation at room temperature (*e.g.,* for one hour). The solution is then removed and the plate washed eight times with 0.1% polysorbate 20 (TWEEN-20^{®}) in PBS. When the plates have dried, 150 µl/well of scintillant (MICROSCINT-20 ^{™}; Packard) is added, and the plates are counted on a TOPCOUNT ^{™} gamma counter (Packard) for ten minutes. Concentrations of each Fab that give less than or equal to 20% of maximal binding are chosen for use in competitive binding assays.

In certain embodiments, K_{d} can be measured using a BIACORE^{®} surface plasmon resonance assay. For example, an assay using a BIACORE^{®}-2000 or a BIACORE ^{®}-3000 (BIAcore, Inc., Piscataway, NJ) is performed at 25°C with immobilized antigen CM5 chips at ~10 response units (RU). In certain embodiments, carboxymethylated dextran biosensor chips (CM5, BIACORE, Inc.) are activated with N-ethyl-N'-(3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 µg/ml (~0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20^{™}) surfactant (PBST) at 25°C at a flow rate of approximately 25 µl/min. Association rates (kₒₙ) and dissociation rates (k_{off}) are calculated using a simple one-to-one Langmuir binding model (BIACORE ^{®} Evaluation Software version 3.2) by simultaneously fitting the association and dissociation sensorgrams. The equilibrium dissociation constant (K_{d}) is calculated as the ratio k_{off}/ kₒₙ. *See, e.g.,* Chen et al., J. Mol. Biol. 293:865-881 (1999). If the on-rate exceeds 10⁶ M⁻¹s⁻¹ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25^{O}C of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO ^{™} spectrophotometer (ThermoSpectronic) with a stirred cuvette.

### 2. Antibody Fragments

In certain embodiments, an antibody provided herein is an antibody fragment. Antibody fragments include, but are not limited, to F(ab')₂, diabodies and other fragments described below. For a review of certain antibody fragments, *see* Hudson et al. Nat. Med. 9:129-134 (2003). For a review of scFv fragments, *see, e.g.,* Pluckthün, in The Pharmacology of Monoclonal Antibodies, vol. 113, Rosenburg and Moore eds., (Springer-Verlag, New York), pp. 269-315 (1994); *see* also PCT Application No. WO 93/16185; and U.S. Patent Nos. 5,571,894 and 5,587,458.

Diabodies are antibody fragments with two antigen-binding sites that may be bivalent or bispecific. *See, for example,* EP Patent Application No. 404,097; PCT Application No. WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Additional non-limiting examples of antibody fragments include Fab, Fab', Fab'-SH, Fv and scFv fragments. Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; *see,* e.g., U.S. Patent No. 6,248,516 B1).

For discussion of Fab and F(ab')₂ fragments comprising salvage receptor binding epitope residues and having increased *in vivo* half-life, *see* U.S. Patent No. 5,869,046.

Antibody fragments can be made by various techniques, including but not limited to proteolytic digestion of an intact antibody as well as production by recombinant host cells (*e.g., E. coli* or phage), as described herein.

### 3. Chimeric and Humanized Antibodies

In certain embodiments, the multispecific and bispecific antibodies provided herein are chimeric antibodies. Certain chimeric antibodies are described, *e.g.,* in U.S. Patent No. 4,816,567; and Morrison et al., Proc. Natl. Acad. Sci. USA, 81:6851-6855 (1984)). In one example, a chimeric antibody comprises a non-human variable region (*e.g.,* a variable region derived from a mouse, rat, hamster, rabbit or non-human primate, such as a monkey) and a human constant region. In certain embodiments, a chimeric antibody can be a "class switched" antibody in which the class or subclass has been changed from that of the parent antibody. Chimeric antibodies include antigen-binding fragments thereof.

In certain embodiments, a chimeric antibody is a humanized antibody. A non-human antibody can be humanized to reduce immunogenicity to humans, while retaining the specificity and affinity of the parental non-human antibody. A humanized antibody can include one or more variable domains in which HVRs, e.g., CDRs, or portions thereof, are derived from a non-human antibody, and FRs, or portions thereof, are derived from human antibody sequences. A humanized antibody optionally can also include at least a portion of a human constant region. In certain embodiments, some FR residues in a humanized antibody are substituted with corresponding residues from a non-human antibody (*e.g.,* the antibody from which the HVR residues are derived), *e.g.,* to restore or improve antibody specificity or affinity.

Humanized antibodies and methods of making them are reviewed, *e.g.,* in Almagro and Fransson, Front. Biosci. 13:1619-1633 (2008), and are further described, *e.g.,* in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall' Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (*see, e.g.,* Sims et al. J. Immunol. 151:2296 (1993)); framework regions derived from the consensus sequence of human antibodies of a particular subgroup of light or heavy chain variable regions *(see, e.g.,* Carter et al. Proc. Natl. Acad. Sci. USA, 89:4285 (1992); and Presta et al. J. Immunol., 151:2623 (1993)); human mature (somatically mutated) framework regions or human germline framework regions (see, *e.g.,* Almagro and Fransson, Front. Biosci. 13:1619-1633 (2008)); and framework regions derived from screening FR libraries (see, *e.g.,* Baca et al., J. Biol. Chem. 272:10678-10684 (1997) and Rosok et al., J. Biol. Chem. 271:22611-22618 (1996)).

### 4. Human Antibodies

In certain embodiments, the multispecific and bispecific antibodies provided herein are human antibodies. Human antibodies can be produced using various techniques known in the art. Human antibodies are described, generally, in van Dijk and van de Winkel, Curr. Opin. Pharmacol. 5: 368-74 (2001) and Lonberg, Curr. Opin. Immunol. 20:450-459 (2008).

Human antibodies can be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). *See* also, *e.g.,* U.S. Patent Nos. 6,075,181 and 6,150,584 describing XENOMOUSE^{™} technology; U.S. Patent No. 5,770,429 describing HUMAB^{®} technology; U.S. Patent No. 7,041,870 describing K-M MOUSE^{®} technology, and U.S. Patent Application Publication No. US 2007/0061900, describing VELOCIMOUSE^{®} technology). Human variable regions from intact antibodies generated by such animals can be further modified, *e.g.,* by combining with a different human constant region.

In certain embodiments, human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

In certain embodiments, human antibodies can also be generated by isolating Fv clone variable domain sequences selected from human-derived phage display libraries. Such variable domain sequences may then be combined with a desired human constant domain. Techniques for selecting human antibodies from antibody libraries are described below.

### 5. Library-Derived Antibodies

Multispecific and bispecific antibodies, e.g., bispecific anti-KLB antibodies, of the present disclosure can be isolated by screening combinatorial libraries for antibodies with the desired activity or activities. For example, but not by way of limitation, a variety of methods are known in the art for generating phage display libraries and screening such libraries for antibodies possessing the desired binding characteristics. Such methods are reviewed, *e.g.,* in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned *(e.g.,* from human) to provide a single source of antibodies to a wide range of non-self and also self-antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example, US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### 6. Antibody Variants

In certain embodiments, amino acid sequence variants of the disclosed antibodies are provided herein. For example, it may be desirable to improve the binding affinity and/or other biological properties of an antibody by generating an amino acid sequence variant of the antibody. Amino acid sequence variants of a multispecific antibody of the present disclosure, e.g., a bispecific anti-KLB antibody, can be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion and substitution can be made to arrive at the final construct, provided that the final construct possesses the desired characteristics, e.g., antigen-binding.

### a) Substitution, Insertion, and Deletion Variants

In certain embodiments, antibody variants having one or more amino acid substitutions are provided. Sites of interest for substitutional mutagenesis include the HVRs and FRs. Non-limiting examples of conservative substitutions are shown in Table 1 under the heading of "preferred substitutions." Non-limiting examples of more substantial changes are provided in Table 1 under the heading of "exemplary substitutions," and as further described below in reference to amino acid side chain classes. Amino acid substitutions can be introduced into an antibody of interest and the products screened for a desired activity, *e.g.,* retained/improved antigen binding, decreased immunogenicity or improved complement dependent cytotoxicity or antibody-dependent cell-mediated cytotoxicity.

Amino acids can be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

In certain embodiments, non-conservative substitutions will entail exchanging a member of one of these classes for another class.

**Table 1**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

In certain embodiments, a type of substitutional variant involves substituting one or more hypervariable region residues of a parent antibody, *e.g.,* a humanized or human antibody. Generally, the resulting variant(s) selected for further study will have modifications, *e.g.,* improvements, in certain biological properties such as, but not limited to, increased affinity, reduced immunogenicity, relative to the parent antibody and/or will have substantially retained certain biological properties of the parent antibody. A non-limiting example of a substitutional variant is an affinity matured antibody, which may be conveniently generated, *e.g.,* using phage display-based affinity maturation techniques such as those described herein. Briefly, one or more HVR residues are mutated and the variant antibodies displayed on phage and screened for a particular biological activity (*e.g.,* binding affinity).

In certain embodiments, alterations (*e.g.,* substitutions) can be made in HVRs, *e.g.,* to improve antibody affinity. Such alterations may be made in HVR "hotspots," *i.e.,* residues encoded by codons that undergo mutation at high frequency during the somatic maturation process *(see, e.g.,* Chowdhury, Methods Mol. Biol. 207:179-196 (2008)), and/or residues that contact antigen, with the resulting variant VH or VL being tested for binding affinity. Affinity maturation by constructing and reselecting from secondary libraries has been described, *e.g.,* in Hoogenboom et al. in Methods in Molecular Biology 178:1-37 (O'Brien et al., ed., Human Press, Totowa, NJ, (2001). In certain embodiments of affinity maturation, diversity can be introduced into the variable genes chosen for maturation by any of a variety of methods (*e.g.,* error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (*e.g.,* 4-6 residues at a time) are randomized. HVR residues involved in antigen binding can be specifically identified, *e.g.,* using alanine scanning mutagenesis or modeling. CDR-H3 and CDR-L3 in particular are often targeted.

In certain embodiments, substitutions, insertions or deletions can occur within one or more HVRs so long as such alterations do not substantially reduce the ability of the antibody to bind antigen. For example, conservative alterations (e.g., conservative substitutions as provided herein) that do not substantially reduce binding affinity can be made in HVRs. Such alterations may, for example, be outside of antigen contacting residues in the HVRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid *(e.g.,* alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants can be screened to determine whether they contain the desired properties as described above.

Amino acid sequence insertions can include amino- and/or carboxyl-terminal fusions ranging in length from one residue to polypeptides containing a hundred or more residues, as well as intrasequence insertions of single or multiple amino acid residues. Non-limiting examples of terminal insertions include an antibody with an N-terminal methionyl residue. Other insertional variants of the antibody molecule can include the fusion to the N- or C-terminus of the antibody to an enzyme (*e.g.,* for ADEPT) or a polypeptide, which increases the serum half-life of the antibody.

### b) Glycosylation variants

In certain embodiments, multispecific and bispecific antibodies provided herein can be altered to increase or decrease the extent to which the antibody is glycosylated. Addition or deletion of glycosylation sites to an antibody can be conveniently accomplished by altering the amino acid sequence such that one or more glycosylation sites is created or removed.

In certain embodiments where the antibody comprises an Fc region, the carbohydrate attached thereto can be altered. Native antibodies produced by mammalian cells typically comprise a branched, biantennary oligosaccharide that is generally attached by an N-linkage to Asn297 of the CH2 domain of the Fc region. *See, e.g.,* Wright et al. TIBTECH 15:26-32 (1997). The oligosaccharide may include various carbohydrates, *e.g.,* mannose, N-acetyl glucosamine (GlcNAc), galactose, and sialic acid, as well as a fucose attached to a GlcNAc in the "stem" of the biantennary oligosaccharide structure. In certain embodiments, modifications of the oligosaccharide in an antibody of the present disclosure can be made in order to create antibody variants with certain improved properties.

In certain embodiments, antibody variants having a carbohydrate structure that lacks fucose attached (directly or indirectly) to an Fc region are provided herein. For example, the amount of fucose in such antibody can be from about 1% to about 80%, from about 1% to about 65%, from about 5% to about 65% or from about 20% to about 40% and values in between.

The amount of fucose can be determined by calculating the average amount of fucose within the sugar chain at Asn297, relative to the sum of all glycostructures attached to Asn 297 (e.g., complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 can also be located about ± 3 amino acids upstream or downstream of position 297, *i.e.,* between positions 294 and 300, due to minor sequence variations in antibodies. Such fucosylation variants may have improved ADCC function. *See, e.g.,* US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004).

Defucosylated antibodies can be produced in any cell line that is deficient in protein fucosylation. Non-limiting examples of cell lines include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams *et al.,* especially at Example 11), and knockout cell lines, such as alpha-1,6-fucosyltransferase gene, *FUT8,* knockout CHO cells (see, *e.g.,* Yamane-Ohnuki et al. Biotech. Bioeng. 87: 614 (2004); Kanda, Y. et al., Biotechnol. Bioeng., 94(4):680-688 (2006); and WO2003/085107).

Antibodies variants are further provided with bisected oligosaccharides, *e.g.,* in which a biantennary oligosaccharide attached to the Fc region of the antibody is bisected by GlcNAc. Such antibody variants may have reduced fucosylation and/or improved ADCC function. Non-limiting examples of such antibody variants are described, *e.g.,* in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants can have improved CDC function. Such antibody variants are described, *e.g.,* in WO 1997/30087 (Patel et al.); WO 1998/58964 (Raju, S.); and WO 1999/22764 (Raju, S.).

### c) Fc region variants

In certain embodiments, one or more amino acid modifications may be introduced into the Fc region of an antibody provided herein, thereby generating an Fc region variant. The Fc region variant may comprise a human Fc region sequence (*e.g.,* a human IgG₁, IgG₂, IgG₃ or IgG₄ Fc region) comprising an amino acid modification (*e.g*., a substitution) at one or more amino acid positions.

In certain embodiments, the present disclosure contemplates an antibody variant that possesses some but not all effector functions, which make it a desirable candidate for applications in which the half-life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express Fc(RIII only, whereas monocytes express FcyRI, FcyRII and FcγRIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Patent No. 5,500,362 (see, *e.g.,* Hellstrom, I. et al. Proc. Nat'lAcad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACTI^{™} non-radioactive cytotoxicity assay for flow cytometry (CellTechnology, Inc. Mountain View, CA; and CytoTox 96^{®} non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, *e.g.,* C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half-life determinations can also be performed using methods known in the art (see, *e.g.,* Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described (*see, e.g.,* U.S. Patent No. 6,737,056; WO 2004/056312, and Shields et al., J. Biol. Chem. 9(2): 6591-6604 (2001). In certain embodiments, an antibody variant includes an Fc region with one or more amino acid substitutions which improve ADCC, e.g., substitutions at positions 298, 333, and/or 334 of the Fc region (EU numbering of residues). In certain embodiments, alterations are made in the Fc region that result in altered (*i.e.,* either improved or diminished) C1q binding and/or Complement Dependent Cytotoxicity (CDC), *e.g.,* as described in US Patent No. 6,194,551, WO 99/51642, and Idusogie et al. J. Immunol. 164: 4178-4184 (2000).

In certain embodiments, alteration made in the Fc region of a bispecific antibody, disclosed herein, can produce a variant antibody with an increased half-life and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, *e.g.,* substitution of Fc region residue 434 (US Patent No. 7,371,826).

*See also* Duncan & Winter, Nature 322:738-40 (1988); U.S. Patent No. 5,648,260; U.S. Patent No. 5,624,821; and WO 94/29351 concerning other examples of Fc region variants.

### d) Cysteine engineered antibody variants

In certain embodiments, it may be desirable to create cysteine engineered antibodies, *e.g.,* "thioMAbs," in which one or more residues of an antibody are substituted with cysteine residues. In certain embodiments, the substituted residues can occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described herein. Cysteine engineered antibodies may be generated as described, *e.g.,* in U.S. Patent No. 7,521,541.

### e) Antibody Derivatives

In certain embodiments, a multispecific, *e.g.,* bispecific, antibody provided herein can be further modified to contain additional non-proteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, polypropylene oxide/ethylene oxide co-polymers, polyoxyethylated polyols (*e.g.,* glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In certain embodiments, conjugates of an antibody and non-proteinaceous moiety that can be selectively heated by exposure to radiation are provided herein. In certain embodiments, the non-proteinaceous moiety is a carbon nanotube (Kam et al., Proc. Natl. Acad. Sci. USA 102: 11600-11605 (2005)). In certain embodiments, the radiation can be of any wavelength, and includes, but is not limited to, wavelengths that do not harm ordinary cells, but which heat the non-proteinaceous moiety to a temperature at which cells proximal to the antibody-non-proteinaceous moiety are killed.

### C. Immunoconjugates

The presently disclosed subject matter also provides immunoconjugates, which include a multispecific antibody, *e.g.,* a bispecific antibody, disclosed herein, conjugated to one or more cytotoxic agents, such as chemotherapeutic agents or drugs, growth inhibitory agents, proteins, peptides, toxins (*e.g.,* protein toxins, enzymatically active toxins of bacterial, fungal, plant, or animal origin, or fragments thereof), or radioactive isotopes. For example, an antibody or antigen-binding portion of the disclosed subject matter can be functionally linked (*e.g.,* by chemical coupling, genetic fusion, noncovalent association or otherwise) to one or more other binding molecules, such as another antibody, antibody fragment, peptide or binding mimetic.

In certain embodiments, an immunoconjugate is an antibody-drug conjugate (ADC) in which an antibody is conjugated to one or more drugs, including but not limited to, a maytansinoid (*see* U.S. Patent Nos. 5,208,020, 5,416,064 and European Patent EP 0 425 235 B1); an auristatin such as monomethylauristatin drug moieties DE and DF (MMAE and MMAF) (*see* U.S. Patent Nos. 5,635,483 and 5,780,588, and 7,498,298); a dolastatin; a calicheamicin or derivative thereof (*see* U.S. Patent Nos. 5,712,374, 5,714,586, 5,739,116, 5,767,285, 5,770,701, 5,770,710, 5,773,001, and 5,877,296; Hinman et al., Cancer Res. 53:3336-3342 (1993); and Lode et al., Cancer Res. 58:2925-2928 (1998)); an anthracycline such as daunomycin or doxorubicin *(see* Kratz et al., Current Med. Chem. 13:477-523 (2006); Jeffrey et al., Bioorganic & Med. Chem. Letters 16:358-362 (2006); Torgov et al., Bioconj. Chem. 16:717-721 (2005); Nagy et al., Proc. Natl. Acad. Sci. USA 97:829-834 (2000); Dubowchik et al., Bioorg. & Med. Chem. Letters 12:1529-1532 (2002); King et al., J. Med. Chem. 45:4336-4343 (2002); and U.S. Patent No. 6,630,579); methotrexate; vindesine; a taxane such as docetaxel, paclitaxel, larotaxel, tesetaxel, and ortataxel; a trichothecene; and CC1065.

In certain embodiments, an immunoconjugate includes an antibody as described herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes.

In certain embodiments, an immunoconjugate includes multispecific antibody, as described herein, conjugated to a radioactive atom to form a radioconjugate. A variety of radioactive isotopes are available for the production of radioconjugates. Non-limiting examples include At^{211,} I¹³¹, I¹²⁵, Y⁹⁰, Re¹⁸⁶, Re¹⁸⁸, Sm¹⁵³, Bi²¹², P³², Pb²¹² and radioactive isotopes of Lu. When a radioconjugate is used for detection, it can include a radioactive atom for scintigraphic studies, for example tc99m or I¹²³, or a spin label for nuclear magnetic resonance (NMR) imaging (also known as magnetic resonance imaging, mri), such as iodine-123, iodine-131, indium-111, fluorine-19, carbon-13, nitrogen-15, oxygen-17, gadolinium, manganese or iron.

Conjugates of an antibody and cytotoxic agent can be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO 94/11026. The linker may be a "cleavable linker" facilitating release of a cytotoxic drug in the cell. For example, an acid-labile linker, peptidase-sensitive linker, photolabile linker, dimethyl linker or disulfide-containing linker (Chari et al., Cancer Res. 52:127-131 (1992); U.S. Patent No. 5,208,020) may be used. Non-limiting examples of linkers are disclosed above.

The immunuoconjugates or ADCs herein expressly contemplate, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A).

### IV. METHODS OF SCREENING AND PRODUCTION

The multispecific antibodies of the presently disclosed subject matter can be identified, screened for or characterized for their physical/chemical properties and/or biological activities by various assays known in the art and provided herein.

### A. Multispecific antibody screening assays

In certain embodiments, a multispecific antibody, *e.g.,* a bispecific antibody or a biepitopic antibody, of the present disclosure can be identified by known methods. For example, and not by way of limitation, bispecific antibodies or biepitopic antibodies can be identified using the "knob-into-hole" heterodimerization technique, discussed above. *See,* also, Ridgway et al., Protein Engineering, Vol. 9:7, p617-621 (1996); Atwell et al. J. Mol. Biol. 270, 26-35 (1997); and Spiess et al., Nat. Biotech. 31, 753-759 (2013).

The presently disclosed subject matter further provides a method of screening for a multispecific antibody, *e.g.,* a bispecific antibody or a biepitopic antibody. In certain embodiments, the methods disclosed herein can be used to identify a multispecific antibody, *e.g.,* a bispecific or biepitopic antibody, that exhibits agonistic activity or antagonistic activity.

In certain embodiments, the method comprises expressing a multispecific antibody in one or more cells. For example, and not by way of limitation, the multispecific antibody can comprise a first antigen-binding polypeptide, a second antigen-binding polypeptide and a CL domain. The first antigen-binding polypeptide and the second antigen-binding polypeptide can have distinct binding specificities. For example, and not by way of limitation, the first antigen-binding polypeptide can bind to a first epitope of an antigen and the second antigen-binding polypeptide can bind to a second epitope on the antigen. Alternatively, the first antigen-binding polypeptide can bind to a first antigen and the second antigen-binding polypeptide can bind to a second antigen. In certain embodiments, each of the antigen-binding polypeptides comprise a VL, a VH, a CH1 domain, a CH2 domain and a CH3 domain, where the components are positioned relative to each other in an N-terminal to C-terminal direction in the following sequential order: VL-linker-VH-CH1-CH2-CH3. In certain embodiments, the first and/or second antigen-binding polypeptides can further comprise a hinge region between the CH1 and CH2 domains. In certain embodiments, the C-terminus of the VL domain can be linked to the N-terminus of the VH domain via the linker. The first and/or second antigen-binding polypeptides can further comprise a heterodimerization domain as disclosed herein, *e.g.,* a protuberance or a cavity in the CH3 domain.

In certain embodiments, a nucleic acid, *e.g.,* a first nucleic acid, that encodes a first antigen-binding polypeptide, or a vector that comprises the nucleic acid, can be introduced into the cells. A second nucleic acid that encodes a second antigen-binding polypeptide, or a vector that comprises the second nucleic acid, can be introduced into the cells. In certain embodiments, a third nucleic acid encoding a CL domain, or a vector comprising the third nucleic acid, can be introduced into the cell. The nucleic acids can be introduced into the cells by any methods known in the art. For example, and not by way of limitation, the nucleic acids can be introduced into the cells by transformation or transfection.

The cells used in the disclosed methods can be any of the cells disclosed herein, *e.g.,* the host cells disclosed herein. For example, and not by way of limitation, the cells can be prokaryotic cells, *e.g.,* Escherichia coli (E. coli). In certain embodiments, the cells are eukaryotic cells, *e.g.,* yeast cells or mammalian cells. In certain embodiments, the mammalian cells are Chinese hamster ovary (CHO) cells.

In certain embodiments, the method can further include contacting the multispecific antibody obtained from the one or more cells with two or more antigens, *e.g.,* a first and a second antigen, or two or more epitopes present on the same antigen, *e.g.,* a first and a second epitope on the antigen. In certain embodiments, the two or more epitopes can be overlapping or non-overlapping. In certain embodiments, the multispecific antibody can be contacted with the two or more antigens or the two or more epitopes simultaneously or consecutively. For example, and not by way of limitation, the multispecific antibody can be contacted with one of the two or more antigens or two or more epitopes, followed by the contact of the multispecific antibody with the second of the two or more antigens or the two or more epitopes. Prior to contacting the multispecific antibody with the antigens or epitopes, the multispecific antibody can be purified and/or isolated, *e.g.,* from the cells. For example, and not by way of limitation, the multispecific antibody can be purified by chromatographic methods, *e.g.,* by protein A chromatography.

In certain embodiments, the method can further include identifying the multispecific antibody that binds to the two or more antigens or the two or more epitopes. In certain embodiments, binding can be determined by any assay known in the art and disclosed herein. For example, and not by way of limitation, binding can be determined by ELISA or by flow cytometry, *e.g.,* fluorescence activated cell sorting (FACS).

In certain embodiments, the method for screening for a multispecific antibody can comprise generating a library that includes a plurality of multispecific antibodies disclosed herein. For example, and not by way of limitation, the library can include a plurality of cells, where each cell expresses a single multispecific antibody disclosed herein. In certain embodiments, each cell of the library expresses a first antigen-binding polypeptide, a second antigen-binding polypeptide and a CL domain. In certain embodiments, nucleic acids, or vectors comprising such nucleic acids, that encode the first antigen-binding polypeptide, the second antigen-binding polypeptide and/or the CL domain can be introduced into the cell, *e.g.,* by transformation or transfection. In certain embodiments, each of the first antigen-binding polypeptide, the second antigen-binding polypeptide and/or the CL domain are encoded by separate nucleic acids or separate vectors comprising such nucleic acids. In certain embodiments, the first antigen-binding polypeptide and the second antigen-binding polypeptide are encoded by a first nucleic acid (or a first vector comprising the first nucleic acid) and the CL domain is encoded by a second nucleic acid (or second vector comprising the second nucleic acid). For example, and not by way of limitation, a first nucleic acid of the presently disclosed subject matter can comprise the open reading frame (ORF) that encodes the first antigen-binding polypeptide and the ORF that encodes the second antigen-binding polypeptide. In certain embodiments, the second nucleic acid can comprise the ORF that encodes the CL domain. In certain embodiments, the CL domain is not encoded by the same nucleic acid (or the vector comprising the nucleic acid) that encodes the first antigen-binding polypeptide and/or the second antigen-binding polypeptide.

The method can further include assaying the plurality of multispecific antibodies within the library for binding to two or more epitopes on an antigen, *e.g.,* a first and second epitope on an antigen. Alternatively or additionally, the method can comprise assaying the plurality of multispecific antibodies within the library for binding to two or more antigens, *e.g.,* a first antigen and a second antigen. In certain embodiments, binding can be determined by any assay known in the art and disclosed herein. The method can further include identifying the multispecific antibody that binds to the two or more antigens or the two or more epitopes on an antigen. For example, and not by way of limitation, a biepitopic antibody can be obtained using the disclosed methods by identifying a multispecific antibody that binds to two epitopes on an antigen.

In certain embodiments, a method for screening for a multispecific antibody, disclosed herein, can further include determining the activity of the multispecific antibody, *e.g.,* by a luciferase assay. For example, and not by way of limitation, the method can include determining if the multispecific antibody exhibits antagonistic activity. In certain embodiments, the method can include determining if the multispecific antibody exhibits agonistic activity. In certain embodiments, the method can further include comparing the activity of the multispecific antibody to the activity of the parental monospecific antibodies from which it is derived to identify a multispecific antibody that exhibits greater activity, *e.g.,* antagonistic or agonistic activity, than the parental monospecific antibodies, individually or combined. Activity of a multispecific antibody can be determined by any activity assay known in the art and disclosed herein.

The presently disclosed subject matter further provides methods for identifying biepitopic antibodies, using the screening methods disclosed herein. For example, and not by way of limitation, the disclosed screening method can be used to identify biepitopic antibodies that exhibit antagonistic or agonistic activity. In certain embodiments, the disclosed screening method can be used to identify biepitopic antibodies that exhibit agonistic activity. In certain embodiments, the biepitopic antibodies identified using the disclosed methods exhibit greater agonistic activity than the parental monospecific antibodies, individually or combined, from which the biepitopic antibody is derived. In certain embodiments, a biepitopic antibody that is identified using the disclosed methods exhibits greater agonistic activity than a monospecific antibody that has identical VL and VH sequences. In certain embodiments, a biepitopic antibody of the present disclosure exhibits greater agonistic activity than a monospecific antibody that has identical VL, VH, CH1, CH2 and/or CH3 sequences, *e.g.,* as one of the antigen-binding polypeptides of the biepitopic antibody.

In certain embodiments, a method for identifying a biepitopic agonist antibody can comprise expressing a multispecific antibody, *e.g.,* a bispecific or biepitopic antibody, in one or more cells. For example, and not by way of limitation, the multispecific antibody can comprise a first antigen-binding polypeptide, a second antigen-binding polypeptide and a CL domain, as disclosed herein. In certain embodiments, the first antigen-binding polypeptide binds to a first epitope on an antigen and the second antigen-binding polypeptide bind to a second epitope on the antigen. In certain embodiments, the first antigen-binding polypeptide has the same binding specificity and/or the same VL and VH sequences of a first monospecific antibody and the second antigen-binding polypeptide has the same binding specificity and/or the same VL and VH sequences of a first monospecific antibody.

The method can further comprise contacting the multispecific antibody, *e.g.,* a bispecific or biepitopic antibody, obtained from the one or more cells with two epitopes present on the same antigen of interest, e.g., a first and a second epitope on the antigen. For example, and not by way of limitation, the multispecific antibody can be contacted with one of the two epitopes, followed by the contact of the multispecific antibody with the second of the two epitopes. As indicated above, the multispecific antibody, *e.g.,* a bispecific or biepitopic antibody, can be purified prior to contact with the two epitopes. In certain embodiments, the method can further include identifying the multispecific antibody that binds to the two epitopes of interest. Binding to the epitopes can be determined by any assay known in the art and disclosed herein.

In certain embodiments, the method can further comprise determining the activity of the identified biepitopic antibody that binds to the two epitopes. For example, and not by way of limitation, the method can include determining whether the biepitopic antibody exhibits agonistic activity. Agonistic activity of a biepitopic antibody can be determined by any activity assay known in the art and disclosed herein. In certain embodiments, the agonistic activity of a biepitopic antibody can be determined by contacting the antigen, *e.g.,* a receptor, with the biepitopic antibody and analyzing whether the biepitopic antibody results in the activation of a signaling pathway associated with the receptor. For example, and not by way of limitation, agonistic activity can be determined by using an *in vitro* reporter-based assay, *e.g.,* a luciferase assay, where the activation of the receptor, *e.g.,* antigen, results in the expression of reporter, *e.g.,* luciferase. Alternatively or additionally, agonistic activity can be determined by using an *in vitro* cell assay, where the activation of the receptor, *e.g.,* antigen, results in the activation of the signaling pathway regulated by the receptor by analyzing the expression, activity and/or phosphorylation of the downstream targets, *e.g.,* proteins, of the signaling pathway of the receptor.

In certain embodiments, the method can further include comparing the agonistic activity of the biepitopic antibody to the agonistic activity of the monospecific parental antibodies (*e.g.,* from which the biepitopic antibody was derived), individually or combined, and to identify a biepitopic antibody that exhibits greater agonistic activity than the activity of the monospecific parental antibodies. For example, and not by way of limitation, the method can further include determining the agonistic activity of the monospecific parental antibodies of the biepitopic agonistic antibody (*e*.*g*., alone or in combination) using the same technique as the technique used to determine the agonistic activity of the biepitopic antibody to compare the agonistic activities of the biepitopic antibodies and the monospecific antibodies.

In certain embodiments, the VL, VH, CH1, CH2 and/or CH3 domains of the first antigen-binding polypeptide can have the same sequences of the VL, VH, CH1, CH2 and/or CH3 domains of a first parental monoclonal antibody. In certain embodiments, the VL, VH, CH1, CH2 and/or CH3 domains of the second antigen-binding polypeptide can have the same sequences of the VL, VH, CH1, CH2 and/or CH3 domains of a second parental monoclonal antibody. The disclosed methods can allow the identification of multispecific antibodies, *e*.*g*., bispecific antibodies and biepitopic antibodies, by the pairing of two antigen-binding polypeptides having the binding specificities of two parental monoclonal, *e.g.,* monospecific, antibodies. In certain embodiments, the multispecific antibodies identified by the disclosed screening assay exhibit higher binding affinity and/or activity, *e.g.,* agonistic activity, than the parental antibodies, *e.g.,* monospecific antibodies, individually or combined. As a result, the presently disclosed methods provide a throughput method for pairwise screening of VL and VH regions of monospecific antibodies to identify multispecific, *e.g.,* bispecific or biepitopic antibodies. In certain embodiments, the multispecific antibodies, *e.g.,* bispecific or biepitopic antibodies, identified by the disclosed screening methods can be formatted into a different antibody format. For example, and not by way of limitation, the multispecific antibodies, *e.g.,* bispecific or biepitopic antibodies, identified by the disclosed methods can be reformatted into a full antibody format, *e.g.,* of the IgG isotype.

In certain embodiments, the antigen to which the multispecific antibody, bispecific or biepitopic antibody, binds is a receptor. In certain embodiments, the antigen is in a biological complex. For example, and not by way of limitation, the receptor is present within a biological complex, *e.g.,* in a complex with one or more co-receptors and/or proteins.

### B. Binding assays and other assays

In certain embodiments, an antibody of the present disclosure can be tested for its antigen binding activity by known methods, such enzyme-linked immunosorbent assay (ELISA), a radioimmunoassay (RIA), or a Western Blot Assay. Each of these assays generally detects the presence of protein-antibody complexes of particular interest by employing a labeled reagent (*e.g.,* an antibody) specific for the complex of interest.

In certain embodiments, antigen (*e*.*g*., KLB)-antibody complexes can be detected using, *e.g.,* an enzyme-linked antibody or antibody fragment which recognizes and specifically binds to the antigen (*e*.*g*., KLB)-antibody complexes. Alternatively, the complexes can be detected using any of a variety of other immunoassays. For example, the antibody can be radioactively labeled and used in a radioimmunoassay (RIA) (*see, for example,* Weintraub, B., Principles of Radioimmunoassays, Seventh Training Course on Radioligand Assay Techniques, The Endocrine Society, March, 1986). The radioactive isotope can be detected by such means as the use of a Geiger counter or a scintillation counter or by autoradiography.

In certain embodiments, competition assays can be used to identify an antibody that competes with a multispecific antibody, *e.g.,* bispecific antibody, of the presently disclosed subject matter. Detailed exemplary methods for mapping an epitope to which an antibody binds are provided in Morris (1996) "Epitope Mapping Protocols," in Methods in Molecular Biology vol. 66 (Humana Press, Totowa, NJ). In a non-limiting example of a competition assay, immobilized antigen (*e*.*g*., KLB) can be incubated in a solution comprising a first labeled antibody that binds to the antigen (*e*.*g*., KLB) and a second unlabeled antibody that is being tested for its ability to compete with the first antibody for binding to the antigen (*e*.*g*., KLB) . The second antibody may be present in a hybridoma supernatant. As a control, immobilized antigen (*e*.*g*., KLB) is incubated in a solution comprising the first labeled antibody but not the second unlabeled antibody. After incubation under conditions permissive for binding of the first antibody to antigen (*e.g.,* KLB), excess unbound antibody is removed, and the amount of label associated with immobilized antigen (*e*.*g*., KLB) is measured. If the amount of label associated with immobilized antigen (*e*.*g*., KLB) is substantially reduced in the test sample relative to the control sample, then that indicates that the second antibody is competing with the first antibody for binding to the antigen *(e.g.,* KLB). *See* Harlow and Lane (1988) Antibodies: A Laboratory Manual ch.14 (Cold Spring Harbor Laboratory, Cold Spring Harbor, NY).

In certain embodiments, binding of multispecific antibodies, *e*.*g*., bispecific antibodies, to cells, *e.g.,* 293T cells expressing the antigen of the antibodies can be determined using flow cytometry. For example, but not by way of limitation, cell lines expressing an antigen or epitope (*e.g.,* of KLB) can be mixed with various concentrations of bispecific antibodies in PBS containing 0.1% BSA and 10% fetal calf serum, and incubated at 37°C for 1 hour. After washing, the cells can be reacted with Fluorescein-labeled anti-human IgG antibody under the same conditions as the primary antibody staining. The samples can then be analyzed by FACScan instrument using light and side scatter properties to gate on single cells. An alternative assay using fluorescence microscopy may be used (in addition to or instead of) the flow cytometry assay. Cells can be stained exactly as described above and examined by fluorescence microscopy. This method allows visualization of individual cells and analyze localization of the antigen in the cell.

Multispecific antibodies, *e*.*g*., bispecific antibodies, of the present disclosure can can be further tested for reactivity with the epitope or antigen of the antibodies by Western blotting. For example, but not by way of limitation, cell extracts from cells expressing the antigen of interest (*e*.*g*., KLB) can be prepared and subjected to sodium dodecyl sulfate polyacrylamide gel electrophoresis. After electrophoresis, the separated antigens are transferred to nitrocellulose membranes, blocked with 10% fetal calf serum, and probed with the monoclonal antibodies to be tested. Bispecific antibody binding can be detected using anti-human IgG alkaline phosphatase and developed with BCIP/NBT substrate tablets (Sigma Chem. Co., St. Louis, MO).

### C. Activity assays

The present disclosure provides assays for identifying multispecific antibodies, e.g., bispecific antibodies, having biological activity. For example, and not by way of limitation, the present disclosure provides assays for identifying multispecific antibodies, *e*.*g*., anti-KLB antibodies, having biological activity. For example, and not by way of limitation, biological activity for an anti-KLB antibody may include, *e*.*g*., activating a KLB/FGFR1c receptor complex. Antibodies having such biological activity *in vivo* and/or *in vitro* are also provided. In certain embodiments, the assays can include binding bispecific antibodies of the present disclosure to cells, *e.g.,* 293T cells expressing KLB, and analyzing the activity and/or phosphorylation states of one or more downstream targets of the KLB-FGFR1 receptor complex, *e*.*g*., ERK. In certain embodiments, the assay can include the administration of a bispecific antibody of the present disclosure to a subject, *e.g.,* a non-human animal, and analyzing the effect the antibody has on the glucose level in the subject.

### D. Methods of Production

The multispecific, bispecific and biepitopic antibodies, disclosed herein, can be produced using any available or known technique in the art. For example, but not by way of limitation, antibodies can be produced using recombinant methods and compositions, *e.g.,* as described in U.S. Patent No. 4,816,567. Detailed procedures to generate bispecific antibodies, *e.g.,* anti-KLB bispecific antibodies, are described in the Examples below.

In certain embodiments, techniques for making bispecific, biepitopic and/or multispecific antibodies include, but are not limited to, recombinant co-expression of two immunoglobulin heavy chain-light chain pairs, *e.g.,* in the VLfH format, having different specificities *(see* Milstein and Cuello, Nature 305: 537 (1983)), PCT Patent Application No. WO 93/08829, and Traunecker et al., EMBO J. 10: 3655 (1991)), and heterodimerization domains, *e.g.,* produced using "knob-in-hole" engineering (see, *e.g.,* U.S. Patent No. 5,731,168). The "knob-in-hole" technique aims at forcing the pairing of two different antibody heavy chains by introducing mutations into the CH3 domains to modify the contact interface. On one heavy chain one or more original amino acids can be replaced by amino acids with short side chains to create a "hole" or "cavity." In certain embodiments, the hole mutation(s) can comprise one or more of T366S, L368A and/or Y407V (EU numbering). Conversely, one or more amino acids with large side chains were introduced into the other CH3 domain of the second antibody heavy chain, to create a "knob." In certain embodiments, the knob mutation can comprise T366W (EU numbering). By coexpressing these two heavy chains (and two identical light chains, which are appropriate for both heavy chains), high yields of heterodimer formation ("knob-hole") versus homodimer formation ("hole-hole" or "knob-knob") is observed *(see* Ridgway, Protein Eng. 9 (1996) 617-621; and WO 96/027011). Knob-in-hole technology has also been used where the two heavy chains are each paired with a different or cognate light chain. For example, and not by way of limitation, the two heavy chain/light chain pairs can each be produced and folded in separate cells, purified and assembled to form a knob-in-hole bispecific antibody *in vitro. See, e.g.,* WO 2012/106587, WO 2011/133886 and WO 2013/055958. *See,* also, Example 2 of the present disclosure. Additional mutations that can be introduced into the Fc region of the disclosed antibodies to promote heterodimerization of two heavy chains are disclosed below.

In certain embodiments, other heterodimerization domains having a strong preference for forming heterodimers over homodimers can be incorporated into the disclosed multispecific antibodies, *e.g.,* for producing such antibodies *in vitro* or *in vivo.* Non-limiting examples of such heterodimerization domains are disclosed in WO 2007/147901 (describing ionic interactions), WO 2009/089004 (describing electrostatic steering effects), WO 2010/034605 (describing coiled coils). *See,* also, Pack and Plueckthun, Biochemistry 31:1579-1584 (1992) describing leucine zipper and Pack et al., Bio/Technology 11:1271-1277 (1993) describing the helix-turn-helix motif. In certain embodiments, the multispecific antibodies disclosed herein can comprise one or more heterodimerization domains to generate heterodimers, *e.g.,* to facilitate interaction between a first antigen-binding polypeptide and a second antigen-binding polypeptide of a multispecific antibody. Additional non-limiting examples of techniques that can be used to generate heterodimerization domains within the antibodies, *e.g.,* within the first and/or second antigen-binding polypeptides of the antibodies, of the present disclosure include F405L in the first antigen-binding polypeptide chain and K409R in the second antigen-binding polypeptide chain (Labrijn et al. Efficient generation of stable bispecific IgG1 by controlled Fab-arm exchange. Proc. Nat'l. Acad. Sci. USA 110:5145-5150 (2013)); T350V, L351Y, F405A and Y407V in the first antigen-binding polypeptide chain and T350V, T366L, K392L and T394W in the second antigen-binding polypeptide chain (Kreudenstein et al. Improving biophysical properties of a bispecific antibody scaffold to aid developability: quality by molecular design. MAbs 5:646-654 (2013)); K409D and K392D in the first antigen-binding polypeptide chain and D399K and E356K in the second antigen-binding polypeptide chain (Gunasekaran et al. Enhancing antibody Fc heterodimer formation through electrostatic steering effects: applications to bispecific molecules and monovalent IgG. J. Biol. Chem. 285:19637-19646 (2010)); D221E, P228E and L368E in the first antigen-binding polypeptide chain and D221R, P228R and K409R in the second antigen-binding polypeptide chain (Strop et al. Generating Bispecific Human IgG1 and IgG2 Antibodies from Any Antibody Pair. J Mol Biol 420:204-219 (2012)); IgG/A chimera (Davis, J. H. et al. SEEDbodies: fusion proteins based on strand-exchange engineered domain (SEED) CH3 heterodimers in an Fc analogue platform for asymmetric binders or immunofusions and bispecific antibodies. Protein Eng. Des. Sel 23:195-202 (2010)); H435R in one of the first or second antigen-binding polypeptide chains (US 2014/0248664A1); Y349C, K360E and K409W in the first antigen-binding polypeptide chain and S354C, Q347R, D399V and F405T in the second antigen-binding polypeptide chain (Choi et al. Crystal structures of immunoglobulin Fc heterodimers reveal the molecular basis for heterodimer formation. Mol. Immunol. 65:377-383 (2015)); and S364H and F405A in the first antigen-binding polypeptide chain and Y349T and T394F in the second antigen-binding polypeptide chain (Moore et al. A novel bispecific antibody format enables simultaneous bivalent and monovalent co-engagement of distinct target antigens. MAbs 3:546-557 (2011)).

Multispecific, biepitopic and bispecific antibodies of the present disclosure can also be made by engineering electrostatic steering effects for making antibody Fc-heterodimeric molecules (WO 2009/089004A1); cross-linking two or more antibodies or fragments (see, *e.g.,* US Patent No. 4,676,980, and Brennan et al., Science, 229: 81 (1985)); using leucine zippers to produce bispecific antibodies (see, *e*.*g*., Kostelny et al., J. Immunol., 148(5):1547-1553 (1992)); using "diabody" technology for making bispecific antibody fragments (see, *e.g.,* Hollinger et al., Proc. Natl. Acad. Sci. USA, 90:6444-6448 (1993)); and using single-chain Fv (sFv) dimers (*see, e.g.,* Gruber et al., J. Immunol., 152:5368 (1994)); and preparing trispecific antibodies as described, *e.g.,* in Tutt et al. J. Immunol. 147: 60 (1991).

Bispecific and multispecific molecules of the present disclosure can also be made using chemical techniques *(see, e.g.,* Kranz (1981) Proc. Natl. Acad. Sci. USA 78:5807), "polydoma" techniques *(see, e.g.,* U.S. Patent 4,474,893), or recombinant DNA techniques. Bispecific and multispecific molecules of the present disclosure can also be prepared by conjugating the constituent binding specificities, *e.g.,* a first epitope and a second epitope binding specificities, using methods known in the art and as described herein. For example, each binding specificity of the bispecific and multispecific molecule can be generated separately and then conjugated to one another. When the binding specificities are proteins or peptides, a variety of coupling or cross-linking agents can be used for covalent conjugation. Examples of cross-linking agents include protein A, carbodiimide, N-succinimidyl-S-acetyl-thioacetate (SATA), N-succinimidyl-3-(2-pyridyldithio)propionate (SPDP), and sulfosuccinimidyl 4-(N-maleimidomethyl) cyclohaxane-1-carboxylate (sulfo-SMCC) *(see, e.g.,* Karpovsky (1984) J. Exp. Med. 160:1686; Liu (1985) Proc. Natl. Acad. Sci. USA 82:8648). Other methods include those described by Paulus (Behring Ins. Mitt. (1985) No. 78, 118-132; Brennan (1985) Science 229:81-83), Glennie (1987) J. Immunol. 139: 2367-2375). When the binding specificities are antibodies (*e.g.,* two humanized antibodies), they can be conjugated via sulfhydryl bonding of the C-terminus hinge regions of the two heavy chains. The hinge region can be modified to contain an odd number of sulfhydryl residues, *e.g.,* one, prior to conjugation.

In certain embodiments, both binding specificities can be encoded in the same vector and expressed and assembled in the same host cell. This method is particularly useful where the bispecific and multispecific molecule is a MAb x MAb, MAb x Fab, Fab x F(ab')₂ or ligand x Fab fusion protein. A multispecific antibody, *e.g.,* bispecific antibody, of the present disclosure can be a single chain molecule, such as a single chain bispecific antibody, a single chain bispecific molecule comprising one single chain antibody and a binding determinant, or a single chain bispecific molecule comprising two binding determinants. Bispecific and multispecific molecules can also be single chain molecules or may comprise at least two single chain molecules. Methods for preparing bi- and multispecific molecules are described for example in U.S. Patent Number 5,260,203; U.S. Patent Number 5,455,030; U.S. Patent Number 4,881,175; U.S. Patent Number 5,132,405; U.S. Patent Number 5,091,513; U.S. Patent Number 5,476,786; U.S. Patent Number 5,013,653; U.S. Patent Number 5,258,498; and U.S. Patent Number 5,482,858. Engineered antibodies with three or more functional antigen binding sites (*e*.*g*., epitope binding sites) including "Octopus antibodies," are also included herein (*see, e.g.,* US 2006/0025576A1).

In certain embodiments, an animal system can be used to produce the disclosed antibodies. One animal system for preparing hybridomas is the murine system. Hybridoma production in the mouse is a very well-established procedure. Immunization protocols and techniques for isolation of immunized splenocytes for fusion are known in the art. Fusion partners (*e*.*g*., murine myeloma cells) and fusion procedures are also known (see, e.g., Harlow and Lane (1988), Antibodies, A Laboratory Manual, Cold Spring Harbor Laboratory Press, Cold Spring Harbor New York).

The presently disclosed subject matter further provides an isolated nucleic acid encoding a multispecific antibody, *e.g.,* a bispecific antibody, disclosed herein. For example, the isolated nucleic acid can encode a first antigen-binding polypeptide and/or a second antigen-binding polypeptide of a multispecific antibody. In certain embodiments, the first antigen-binding polypeptide and the second antigen-binding polypeptide are encoded by two distinct nucleic acids. In certain embodiments, the presently disclosed subject matter further provides an isolated nucleic acid encoding a CL domain, *e.g.,* a kappa CL domain.

In certain embodiments, the isolated nucleic acid can encode an amino acid sequence that includes the VL and/or an amino acid sequence comprising the VH of the antibody, *e.g.,* the light and/or heavy chains of the antibody. In certain embodiments, the isolated nucleic acid can include a nucleotide sequence that encodes a heavy chain variable region amino acid sequence having the sequence set forth in SEQ ID NOs: 34, 36, 38, 40 and 42, and/or a nucleotide sequence that encodes a light chain variable region amino acid sequence having the sequence set forth in SEQ ID NOs: 33, 35, 37, 39 and 41. In certain embodiments, the nucleic acid can be present in one or more vectors, *e.g.,* expression vectors.

As used herein, the term "vector" refers to a nucleic acid molecule capable of transporting another nucleic acid to which it has been linked. One type of vector is a "plasmid," which refers to a circular double stranded DNA loop into which additional DNA segments can be ligated. Another type of vector is a viral vector, where additional DNA segments can be ligated into the viral genome. Certain vectors are capable of autonomous replication in a host cell into which they are introduced (*e.g.,* bacterial vectors having a bacterial origin of replication and episomal mammalian vectors). Other vectors (*e*.*g*., non-episomal mammalian vectors) are integrated into the genome of a host cell upon introduction into the host cell, and thereby are replicated along with the host genome. Moreover, certain vectors, expression vectors, are capable of directing the expression of genes to which they are operably linked. In general, expression vectors of utility in recombinant DNA techniques are often in the form of plasmids (vectors). However, the disclosed subject matter is intended to include such other forms of expression vectors, such as viral vectors (*e.g.,* replication defective retroviruses, adenoviruses and adeno-associated viruses) that serve equivalent functions.

In certain embodiments, the nucleic acid encoding a multispecific antibody and/or the one or more vectors including the nucleic acid can be introduced, *e.g.,* transformed, into a host cell. In certain embodiments, a host cell can be transformed with: (1) a first vector comprising a nucleic acid that encodes an amino acid sequence comprising a first antigen-binding polypeptide; (2) a second vector comprising a nucleic acid that that encodes an amino acid sequence comprising a second antigen-binding polypeptide; and (3) a third vector comprising a nucleic acid that encodes an amino acid sequence comprising a CL domain of the antibody.

In certain embodiments, a host cell can include, *e.g.,* has been transformed with: (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody.

In certain embodiments, the methods of making a multispecific antibody can include culturing a host cell, in which one or more nucleic acids encoding the antibody or specific components of the antibody, *e.g.,* the first antigen-binding polypeptide, the second antigen-binding polypeptide and/or the CL domain, has been introduced, under conditions suitable for expression of the antibody, and optionally recovering the antibody from the host cell and/or host cell culture medium. In certain embodiments, the antibody can be recovered from the host cell by lysing the host cell. Alternatively, the antibody can be secreted from the host cell and recovered from the host cell culture medium. In certain embodiments, the antibody is recovered from the host cell, *e.g.,* a host cell lysate, or the host cell culture medium, through chromatography techniques.

For recombinant production of an antibody of the present disclosure, nucleic acid encoding an antibody, *e.g.,* as described above, is isolated and inserted into one or more vectors for further cloning and/or expression in a host cell. Such nucleic acid may be readily isolated and sequenced using conventional procedures (*e*.*g*., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and antigen-binding polypeptides in bacteria, see, *e.g.,* U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in *E. coli.*) After expression, the antibody may be isolated from the bacterial cell paste in a soluble fraction and can be further purified. In certain embodiments, the host cell is *E. coli.*

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. *See* Gerngross, Nat. Biotech. 22:1409-1414 (2004), and Li et al., Nat. Biotech. 24:210-215 (2006). Suitable host cells for the expression of glycosylated antibody can also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

In certain embodiments, plant cell cultures can be utilized as host cells. *See, e.g.,* US Patent Nos. 5,959,177, 6,040,498, 6,420,548, 7,125,978, and 6,417,429 (describing PLANTIBODIES^{™} technology for producing antibodies in transgenic plants).

In certain embodiments, vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977; baby hamster kidney cells (BHK); mouse sertoli cells (TM4 cells as described, *e.g.,* in Mather, Biol. Reprod. 23:243-251 (1980); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, *e.g.,* in Mather et al., Annals N.Y. Acad. Sci. 383:44-68 (1982); MRC 5 cells; and FS4 cells. Other useful mammalian host cell lines include Chinese hamster ovary (CHO) cells, including DHFR⁻ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, *see, e.g.,* Yazaki and Wu, Methods in Molecular Biology, Vol. 248 (B.K.C. Lo, ed., Humana Press, Totowa, NJ), pp. 255-268 (2003).

### IV. METHODS OF USE

The present disclosure further provides methods for using the disclosed multispecific and bispecific antibodies. In certain embodiments, the disclosure is directed to therapeutic uses of the presently disclosed antibodies. In certain embodiments, the disclosure is directed to the use of the disclosed antibodies in diagnostic methods.

### A. Therapeutic methods

In certain embodiments, one or more antibodies of the presently disclosed subject matter can be used for treating a disease in a subject. In certain embodiments, the treatment of an individual includes administering to the individual a therapeutically effective amount of an antibody, disclosed herein. In certain embodiments, the treatment can further include administering to the subject a therapeutically effective amount of at least one additional therapeutic agent. Non-limiting examples of additional therapeutic agents, *e.g.,* a second therapeutic agent, are described below.

In certain embodiments, the disease can be a metabolic disorder. Non-limiting examples of metabolic disorders include, but are not limited to, polycystic ovary syndrome (PCOS), metabolic syndrome (MetS), obesity, non-alcoholic steatohepatitis (NASH), non-alcoholic fatty liver disease (NAFLD), hyperlipidemia, hypertension, type 2 diabetes, non-type 2 diabetes, type 1 diabetes, latent autoimmune diabetes (LAD), maturity onset diabetes of the young (MODY), and aging and related diseases such as Alzheimer's disease, Parkinson's disease and ALS.

The present disclosure provides methods of treating an immune-related disease or disorder, such as an autoimmune disease or disorder and an inflammatory disease. In certain embodiments, the method comprises administering to a subject in need thereof an antibody of the present disclosure or a pharmaceutical composition comprising the antibody. Non-limiting examples of immune-related diseases or disorders include systemic lupus erythematosis, rheumatoid arthritis, psoriatic arthritis, osteoarthritis, juvenile chronic arthritis, spondyloarthropathies, systemic sclerosis, idiopathic inflammatory myopathies, Sjögren's syndrome, systemic vasculitis, sarcoidosis, autoimmune hemolytic anemia, autoimmune thrombocytopenia, thyroiditis, diabetes mellitus, type I or type II diabetes mellitus, immune-mediated renal disease, demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious, autoimmune chronic active hepatitis, primary biliary cirrhosis, granulomatous hepatitis, and sclerosing cholangitis, inflammatory bowel disease, ulcerative colitis, Crohn's disease, gluten-sensitive enteropathy and Whipple's disease, bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonia, idiopathic pulmonary fibrosis and hypersensitivity pneumonitis, transplantation associated diseases including graft rejection and graft-versus-host-disease. In certain other embodiments, the immune-related disorder is asthma, multiple sclerosis, rheumatoid arthritis, inflammatory bowel disease, ulcerative colitis, lupus erythematosus, psoriasis, chronic obstructive pulmonary disease, or idiopathic pulmonary fibrosis.

The present disclosure further provides methods of treating a cell proliferation-related disease or disorder that comprise administering to a subject in need thereof an antibody of the present disclosure or a pharmaceutical composition comprising the antibody. In certain embodiments, the cell proliferation-related disorder can be, without limitation, colorectal cancer, renal cell cancer *(e.g.,* renal cell carcinoma), melanoma, bladder cancer, ovarian cancer, breast cancer (*e*.*g*., triple-negative breast cancer, HER2-positive breast cancer, or hormone receptor-positive cancer), and non-small-cell lung cancer (*e*.*g*., squamous non-small-cell lung cancer or non-squamous non-small-cell lung cancer). In certain embodiments, a cancer to be treated by the methods of the present disclosure includes, but is not limited to, a carcinoma, lymphoma, blastoma, sarcoma and leukemia. In certain embodiments, a cancer to be treated by the methods of the present disclosure includes, but is not limited to, squamous cell cancer, lung cancer (including small-cell lung cancer, non-small cell lung cancer, adenocarcinoma of the lung, and squamous carcinoma of the lung), melanoma, renal cell carcinoma, cancer of the peritoneum, hepatocellular cancer, gastric or stomach cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney or renal cancer, liver cancer, prostate cancer, vulval cancer, thyroid cancer, hepatic carcinoma and various types of head and neck cancer, as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors) and Meigs' syndrome. In certain embodiments, the cancer may be an early stage cancer or a late stage cancer. In certain embodiments, the cancer may be a primary tumor. In certain embodiments, the cancer may be a metastatic tumor at a second site derived from any of the above types of cancer.

In certain embodiments, a multispecific antibody, *e*.*g*., a bispecific anti-KLB antibody, for use in the disclosed methods can be present in a pharmaceutical composition. In certain embodiments, the pharmaceutical composition can include a pharmaceutically acceptable carrier. Additionally or alternatively, the pharmaceutical composition can include a second therapeutic agent. When antibodies are administered together with another therapeutic agent, the two can be administered in either order or simultaneously.

For the prevention or treatment of disease, the appropriate dosage, *e.g.,* therapeutically effective amount, of an antibody of the presently disclosed subject matter, when used alone or in combination with one or more other additional therapeutic agents, will depend on the type of disease to be treated, the type of antibody, the severity and course of the disease, whether the antibody is administered for preventive or therapeutic purposes, previous therapy, the patient's clinical history and response to the antibody, and the discretion of the attending physician.

In certain embodiments, the antibody can be administered to the patient one time or over a series of treatments. For example, but not by way of limitation, the antibody and/or pharmaceutical formulation contains an antibody, as disclosed herein, can be administered to a subject twice every day, once every day, once every two days, once every three days, once every four days, once every five days, once every six days, once a week, once every two weeks, once every three weeks, once every month, once every two months, once every three months, once every six months or once every year. In certain embodiments, the antibody and/or a pharmaceutical formulation that contains an antibody, disclosed herein, can be administered to a subject once a week or once a month.

In certain embodiments, depending on the type and severity of the disease, about 1 µg/kg to about 15 mg/kg (*e.g.,* 0.1 mg/kg-10 mg/kg) of antibody can be an initial candidate dosage for administration to the patient, whether, for example, by one or more separate administrations, or by continuous infusion. One typical daily dosage can range from about 1 µg/kg to about 100 mg/kg, depending on the factors mentioned above. In certain embodiments, the daily dosage can be greater than about 100 mg/kg. In some methods, dosage is adjusted to achieve a plasma antibody concentration of 1-1000 µg/ml and in some methods 25-300 µg/ml. Alternatively, antibody can be administered as a sustained release formulation, in which case less frequent administration is required. Dosage and frequency can vary based on the half-life of the antibody in the patient.

For repeated administrations over several days or longer, depending on the condition, the treatment would generally be sustained until a desired suppression of disease symptoms occurs. In certain embodiments, a dosage of the antibody can be in the range from about 0.05 mg/kg to about 10 mg/kg. For example, one or more doses of about 0.5 mg/kg, 2.0 mg/kg, 4.0 mg/kg or 10 mg/kg (or any combination thereof) can be administered to the patient. Such doses can be administered intermittently, *e.g.,* every week or every three weeks (*e*.*g*., such that the patient receives from about two to about twenty or, *e.g.,* about six doses of the antibody). An initial higher loading dose, followed by one or more lower doses can be administered.

In certain embodiments, the methods of the present disclosure can further include monitoring the subject and determining the effectiveness of the treatment. For example, the progress of this therapy can be monitored by conventional techniques and assays.

### B. Diagnostic and detection methods

The present disclosure provides methods for diagnosing and/or detecting a disease using a disclosed antibody, disclosed herein. In a further aspect, methods for detecting the presence and/or level of antigen in a biological sample are provided. The term "detecting" as used herein encompasses quantitative and/or qualitative detection.

Non-limiting examples of a sample include, but are not limited to, cells in culture, cell supernatants, cell lysates, serum, blood plasma, biological fluid *(e.g.,* blood, plasma, serum, stool, urine, lymphatic fluid, ascites, ductal lavage, nipple aspirate, saliva, broncho-alveolar lavage, tears and cerebrospinal fluid), and tissue samples. The source of the sample may be solid tissue (*e*.*g*., from a fresh, frozen, and/or preserved organ, tissue sample, biopsy, or aspirate), blood or any blood constituents, bodily fluids (such as, *e.g.,* urine, lymph, cerebral spinal fluid, amniotic fluid, peritoneal fluid, or interstitial fluid), or cells from the individual. In certain embodiments, the biological sample is a tissue and/or cells from a liver.

In certain embodiments, the method of diagnosis or detection of the present disclosure includes contacting biological sample with a bispecific anti-KLB antibody as described herein under conditions permissive for binding of the bispecific anti-KLB antibody to KLB, and detecting whether a complex is formed between the bispecific anti-KLB antibody and KLB. Such method may be an *in vitro* or *in vivo* method, *e.g.,* immunofluorescence or western blot. In certain embodiments, a bispecific anti-KLB antibody, disclosed herein, is used to select subjects eligible for therapy with an anti-KLB antibody, *e.g.,* where KLB is a biomarker for selection of patients.

In certain embodiments, a disclosed antibody for use in the methods of the present disclosure can be labeled. Labels include, but are not limited to, labels or moieties that are detected directly, such as fluorescent, chromophoric, electron-dense, chemiluminescent, and radioactive labels, as well as moieties, such as enzymes or ligands, that are detected indirectly, *e.g.,* through an enzymatic reaction or molecular interaction. Non-limiting examples of labels include, but are not limited to, the radioisotopes ³²P, ¹⁴C, ¹²⁵I, ³H, and ¹³¹I, fluorophores such as rare earth chelates or fluorescein and its derivatives, rhodamine and its derivatives, dansyl, umbelliferone, luceriferases, *e.g.,* firefly luciferase and bacterial luciferase *(see* U.S. Patent No. 4,737,456), luciferin, 2,3-dihydrophthalazinediones, horseradish peroxidase (HRP), alkaline phosphatase, β-galactosidase, glucoamylase, lysozyme, saccharide oxidases, *e.g.,* glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals and the like.

### V. PHARMACEUTICAL FORMULATIONS

The presently disclosed subject matter further provides pharmaceutical formulations containing a multispecific antibody, *e.g.,* bispecific antibody, as described herein, with a pharmaceutically acceptable carrier. In certain embodiments, the pharmaceutical compositions can include a combination of multiple (*e.g.,* two or more) isolated multispecific, *e.g.,* bispecific antibodies, and/or antigen-binding portions thereof of the presently disclosed subject matter.

In certain embodiments, the disclosed pharmaceutical formulations can be prepared by combining a disclosed multispecific, *e.g.,* bispecific anti-KLB antibody, having a desired degree of purity with one or more pharmaceutically acceptable carriers (Remington's Pharmaceutical Sciences 16th edition, Osol, A. Ed. (1980)), in the form of lyophilized formulations or aqueous solutions. For example, but not by way of limitation, lyophilized antibody formulations are described in US Patent No. 6,267,958. In certain embodiments, aqueous antibody formulations can include those described in US Patent No. 6,171,586 and WO2006/044908, the latter formulations including a histidine-acetate buffer.

In certain embodiments, an antibody of the present disclosure can be of a purity greater than about 80%, greater than about 90%, greater than about 91%, greater than about 92%, greater than about 93%, greater than about 94%, greater than about 95%, greater than about 96%, greater than about 97%, greater than about 98%, greater than about 99%, greater than about 99.1%, greater than about 99.2%, greater than about 99.3%, greater than about 99.4%, greater than about 99.5%, greater than about 99.6%, greater than about 99.7%, greater than about 99.8% or greater than about 99.9%.

Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e*.*g*., Zn-protein complexes); and/or non-ionic surfactants such as polyethylene glycol (PEG). Additional non-limiting examples of pharmaceutically acceptable carriers include insterstitial drug dispersion agents such as soluble neutral-active hyaluronidase glycoproteins (sHASEGP), for example, human soluble PH-20 hyaluronidase glycoproteins, such as rHuPH20 (HYLENEX^{®}, Baxter International, Inc.). Certain exemplary sHASEGPs and methods of use, including rHuPH20, are described in US Patent Publication Nos. 2005/0260186 and 2006/0104968. In certain embodiments, a sHASEGP is combined with one or more additional glycosaminoglycanases such as chondroitinases.

The carrier can be suitable for intravenous, intramuscular, subcutaneous, parenteral, spinal or epidermal administration (*e*.*g*., by injection or infusion). Depending on the route of administration, the active compound, *i.e.,* bispecific antibody can be coated in a material to protect the compound from the action of acids and other natural conditions that may inactivate the compound.

Pharmaceutical compositions of the present disclosure also can be administered in combination therapy, *i.e.,* combined with other agents. In certain embodiments, pharmaceutical compositions disclosed herein can also contain more than one active ingredients as necessary for the particular indication being treated, for example, those with complementary activities that do not adversely affect each other. In certain embodiments, the pharmaceutical formula can include a second active ingredient for treating the same disease treated by the first therapeutic. Such active ingredients are suitably present in combination in amounts that are effective for the purpose intended.

A composition of the present disclosure can be administered by a variety of methods known in the art. The route and/or mode of administration vary depending upon the desired results. The active compounds can be prepared with carriers that protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are described by e.g., Sustained and Controlled Release Drug Delivery Systems, J.R. Robinson, ed., Marcel Dekker, Inc., New York, 1978. In certain embodiments, the pharmaceutical compositions are manufactured under Good Manufacturing Practice (GMP) conditions of the U.S. Food and Drug Administration.

Sustained-release preparations containing a disclosed bispecific anti-KLB antibody can also be prepared. Suitable examples of sustained-release preparations include, but are not limited to, semipermeable matrices of solid hydrophobic polymers containing the antibody, which matrices are in the form of shaped articles, e.g., films, or microcapsules. In certain embodiments, active ingredients can be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

To administer an antibody of the present disclosure by certain routes of administration, it may be necessary to coat the compound with, or co-administer the compound with, a material to prevent its inactivation. For example, the compound may be administered to a subject in an appropriate carrier, for example, liposomes, or a diluent. Pharmaceutically acceptable diluents include saline and aqueous buffer solutions. Liposomes include water-in-oil-in-water CGF emulsions as well as conventional liposomes (Strejan et al.. (1984) J. Neuroimmunol. 7:27).

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the present disclosure is contemplated. Supplementary active compounds can also be incorporated into the compositions.

Therapeutic compositions typically must be sterile, substantially isotonic, and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it is preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin.

Sterile injectable solutions can be prepared by incorporating the bispecific antibody in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

Therapeutic compositions can also be administered with medical devices known in the art. For example, a therapeutic composition of the present disclosure can be administered with a needleless hypodermic injection device, such as the devices disclosed in, *e.g.,* U.S. Patent Nos. 5,399,163, 5,383,851, 5,312,335, 5,064,413, 4,941,880, 4,790,824, or 4,596,556. Examples of implants and modules useful in the present disclosure include: U.S. Patent No. 4,487,603, which discloses an implantable micro-infusion pump for dispensing medication at a controlled rate; U.S. Patent No. 4.,486,194, which discloses a therapeutic device for administering medicants through the skin; U.S. Patent No. 4,447,233, which discloses a medication infusion pump for delivering medication at a precise infusion rate; U.S. Patent No. 4,447,224, which discloses a variable flow implantable infusion apparatus for continuous drug delivery; U.S. Patent No. 4,439,196, which discloses an osmotic drug delivery system having multi-chamber compartments; and U.S. Patent No. 4,475,196, which discloses an osmotic drug delivery system. Many other such implants, delivery systems, and modules are known.

For the therapeutic compositions, formulations of the present disclosure include those suitable for oral, nasal, topical (including buccal and sublingual), rectal, vaginal and/or parenteral administration. The formulations can conveniently be presented in unit dosage form and may be prepared by any methods known in the art of pharmacy. The amount of bispecific antibody, which can be combined with a carrier material to produce a single dosage form, vary depending upon the subject being treated, and the particular mode of administration. The amount of the bispecific antibody which can be combined with a carrier material to produce a single dosage form generally be that amount of the composition which produces a therapeutic effect. Generally, out of one hundred percent, this amount range from about 0.01 percent to about ninety-nine percent of active ingredient, from about 0.1 percent to about 70 percent, or from about 1 percent to about 30 per cent.

Formulations of the presently disclosed subject matter which are suitable for vaginal administration also include pessaries, tampons, creams, gels, pastes, foams or spray formulations containing such carriers as are known in the art to be appropriate. Dosage forms for the topical or transdermal administration of compositions of this disclosure include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

The phrases "parenteral administration" and "administered parenterally" mean modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

These pharmaceutical compositions can also contain adjuvants such as preservatives, wetting agents, emulsifying agents and dispersing agents. Prevention of presence of microorganisms may be ensured both by sterilization procedures, *supra,* and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form can be brought about by the inclusion of agents which delay absorption such as aluminum monostearate and gelatin.

When the antibodies of the present disclosure are administered as pharmaceuticals, to humans and animals, they can be given alone or as a pharmaceutical composition containing, for example, from about 0.01% to about 99.5% (or about 0.1 to about 90%) of a bispecific antibody in combination with a pharmaceutically acceptable carrier.

### VI. ARTICLES OF MANUFACTURE AND KITS

The presently disclosed subject matter further relates to articles of manufacture and kits. For example, and not by way of limitation, an article of manufacture and/or kit of the present disclosure can comprise one or more multispecific antibodies, e.g., bispecific antibodies, disclosed herein.

In certain embodiments, the article of manufacture includes a container and a label or package insert on or associated with the container. Non-limiting examples of suitable containers include bottles, vials, syringes and IV solution bags. The containers can be formed from a variety of materials such as glass or plastic. The container can hold a composition which is by itself or combined with another composition effective for treating, preventing and/or diagnosing the condition and may have a sterile access port (for example the container may be an intravenous solution bag or a vial having a stopper pierceable by a hypodermic injection needle).

In certain embodiments, an article of manufacture and/or kit of the present disclosure contains materials useful for the treatment, prevention and/or diagnosis of the disorders described above. In certain embodiments, at least one active agent in the composition is an antibody of the presently disclosed subject matter. The label or package insert can indicate that the composition is used for treating the condition of choice.

In certain embodiments, the article of manufacture can include (a) a first container with a composition contained therein, wherein the composition includes a presently disclosed bispecific antibody of the presently disclosed subject matter; and (b) a second container with a composition contained therein, wherein the composition includes a further cytotoxic or therapeutic agent. In certain embodiments, the article of manufacture can further comprise a package insert indicating that the compositions can be used to treat a particular condition.

In certain embodiments, the article of manufacture can further include an additional container, e.g., a second or third container, including a pharmaceutically-acceptable buffer, such as, but not limited to, bacteriostatic water for injection (BWFI), phosphate-buffered saline, Ringer's solution and dextrose solution. The article of manufacture can include other materials desirable from a commercial and user standpoint, including other buffers, diluents, filters, needles, and syringes.

The following examples are merely illustrative of the presently disclosed subject matter and should not be considered as limitations in any way.

### EXAMPLES

### Example 1: Development and characterization of anti-KLB antibodies

This Example describes the generation of anti-KLB monoclonal antibodies. To produce anti-KLB monoclonal antibodies, KLB knock-out (KLB.ko) mice (Genentech, South San Francisco, CA) were immunized with 50 µg each of either pRK or pCMV vectors expressing either KLB or FGFR1c separately, or with a pCMV.hKLB.IREShFGFR1c vector expressing human KLB and FGFR1c with or without mFlt3 ligand (DNA) and mGM-CSF (DNA) (Genentech) diluted in lactated Ringer's solution via hydrodynamic tail vein (HTV) injection at 1-4 week intervals for a total of 3-13 injections, or immunized with 5 million 300.19 cells stably transfected with human KLB and FGFR1c diluted in PBS weekly via intraperitoneal (i.p.) injection for a total of 12 injections. Mice received a pre-fusion boost of either 50 µg each of KLB and FGFR1c plasmid DNA via HTV or 5 million 300.19-KLB/FGFR1c transfected cells along with 2 µg each of human and cyno KLB proteins via i.p. injection.

Spleens were harvested 3 days after the last immunization. Splenocytes from these mice, all of whose sera demonstrated strong binding to 293 cells overexpressing human KLB and/or KLB/FGFR1c complex by FACS, were fused with P3X63-Ag8U.1 mouse myeloma cells (American Type Culture Collection, Manassas, VA) via electrofusion (Cyto Pulse CEEF-50 apparatus, BTX Harvard Apparatus, Holliston, MA). After washing twice with Cytofusion Medium C (BTX Harvard Apparatus 47-0001), the isolated splenocytes and myeloma cells were mixed at a 1:1 ratio and then resuspended at 10 million cells/ml in Cytofusion Medium C. Electrofusion was performed according to the manufacturer's guidance. Fused cells were cultured in ClonaCell-HY Medium C (Stemcell Technologies, Cat# 03803) overnight at 37°C in a 7% CO₂ incubator. The following day, fused cells were centrifuged and then resuspended in 10 ml of ClonaCell-HY Medium C with anti-mouse IgG-FITC (Jackson Immunoresearch, West Grove, PA) and then gently mixed with 90 ml Methylcellulose-based ClonaCell-HY Medium D (Stemcell Technologies Cat# 03804) containing HAT components. The cells were plated into OmniTray plates (Thermo Fisher Scientific, Rochester, NY) and allowed to grow at 37°C in a 7% CO₂ incubator. After 6-7 days incubation, fluorescent colonies were selected and transferred into 96-well plates (Becton Dickinson, Cat #353075) containing 200 µL/well ClonaCell-HY Medium E (StemCell Technologies, Cat#03805) using a Clonepix FL (Molecular Devices, Sunnyvale, CA). Hybridoma culture media was changed 3 days prior to ELISA screening. Supernatants were screened by ELISA against anti-mouse IgG seven days after picking. Hybridomas demonstrating mouse IgG expression by ELISA were expanded and screened by cell-based ELISA and/or FACS for binding to 293 cells overexpressing human KLβ, mouse KLB, cyno KLB, human KLB/FGFR1c complex, cyno KLB/FGFR1c complex, human FGFR1c, and/or human KLα/ KLB; supernatants were also screened for binding competition with anti-KLB 8C5 antibody (Genentech) by FACS. All IgG positive supernatants were also screened for agonistic activity in a GAL/Elk1 luciferase assay using 293 cells overexpressing human FGFR1c and KLβ/FGFR1c complex. RNA was extracted from FACS positive and agonistic hybridoma cell lines using the RNeasy kit (Qiagen, Hilden, Germany), and cDNA was generated and amplified for sequence determination. Variable region genes of heavy and light chains were inserted into pRK plasmid vectors (Genentech) for expression. Plasmid DNA from unique clones demonstrating FACS binding and agonistic activity were expressed recombinantly in 293 cells. Supernatants were then purified by Protein A affinity chromatography as previously described (Hongo et al., Hybridoma 19:303, 2000).

Approximately 4 different hybridomas, 2C12, 4H7, 23B3 and 28B7, producing monoclonal anti-KLB antibodies that bind to a single epitope of KLB were identified using the methods described above.

A fifth monoclonal antibody, 12B8, was identified using the following experimental method. Balb/c mice were immunized with HEK293 cells stably expressing hFGFR1c and hKLB protein. Spleens were harvested after 12 weeks and hybridomas were generated. Anti-hKLB antibody producing hybridomas were identified by FACS analysis using the HEK293 cells used for immunization. Briefly, 293 cells expressing hKLB alone, hFGFR1 alone, or both, were stained with diluted hybridoma supernatant and PE-conjugated goat anti-mouse IgG antibody (Jackson Labs) in FACS buffer (0.5% BSA in PBS). The same FACS buffer was used to wash the stained cells. Stained cells were analyzed by FACScan (Becton Dickinson) and FlowJo FACS analysis software (Tree Star). cDNA encoding the IgG heavy chain and light chain were cloned into expression vectors.

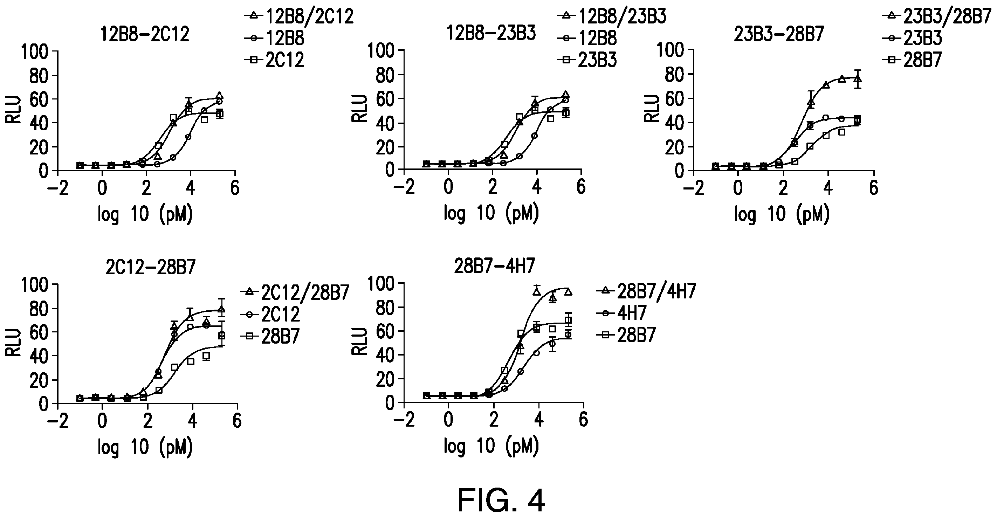

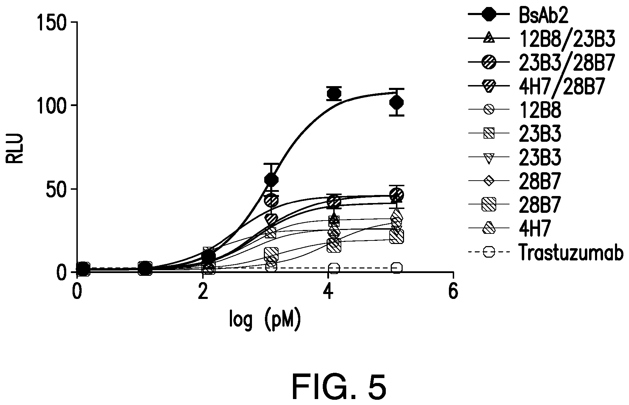

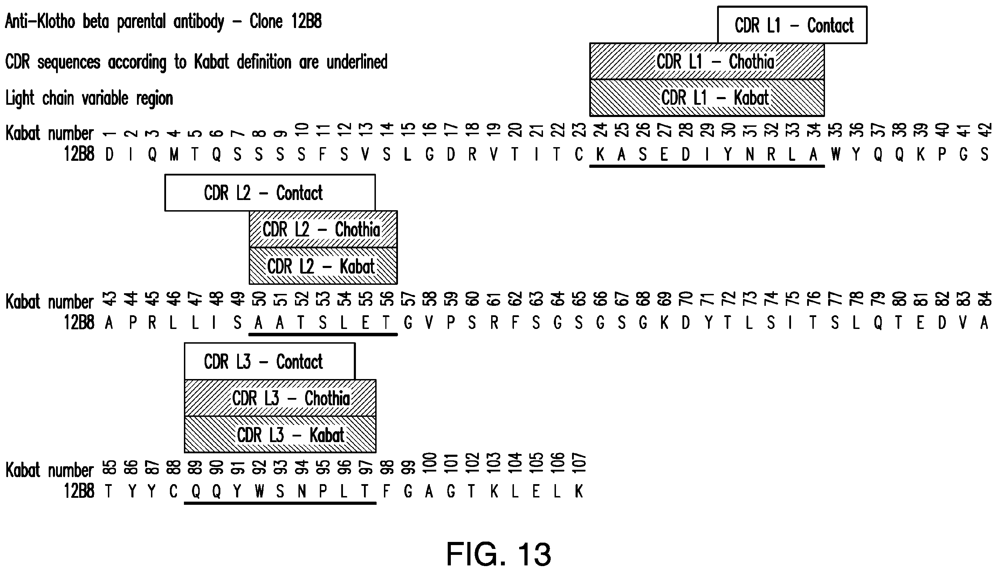

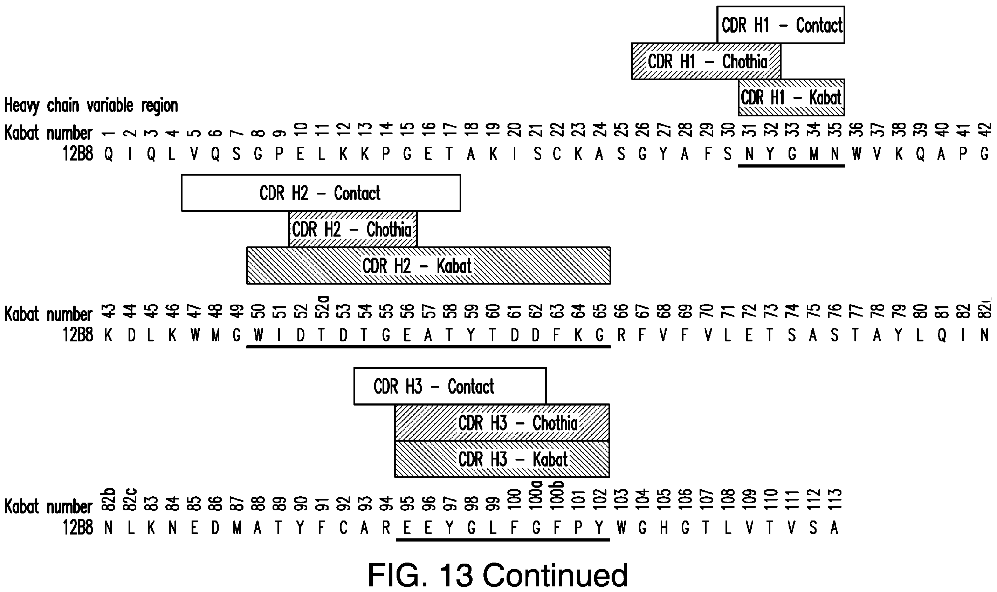

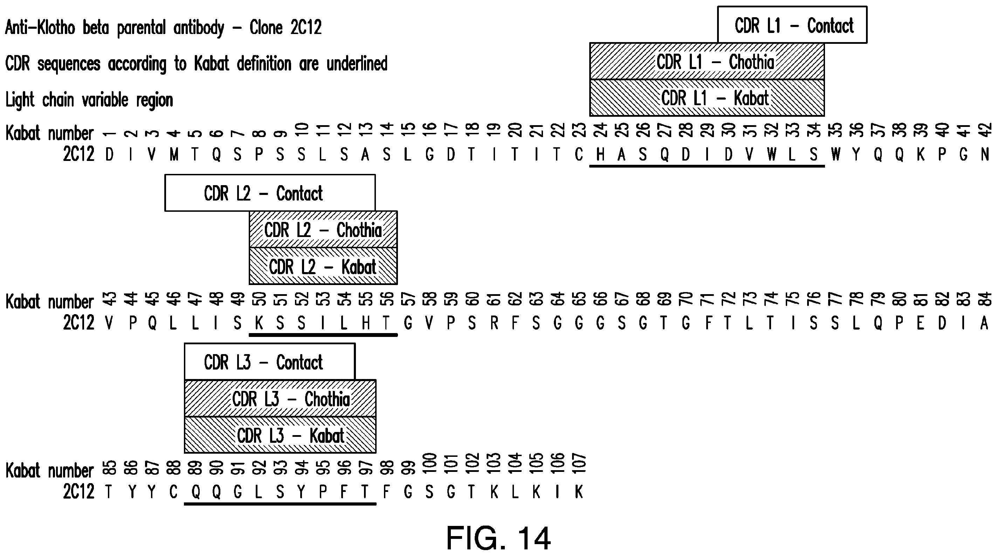

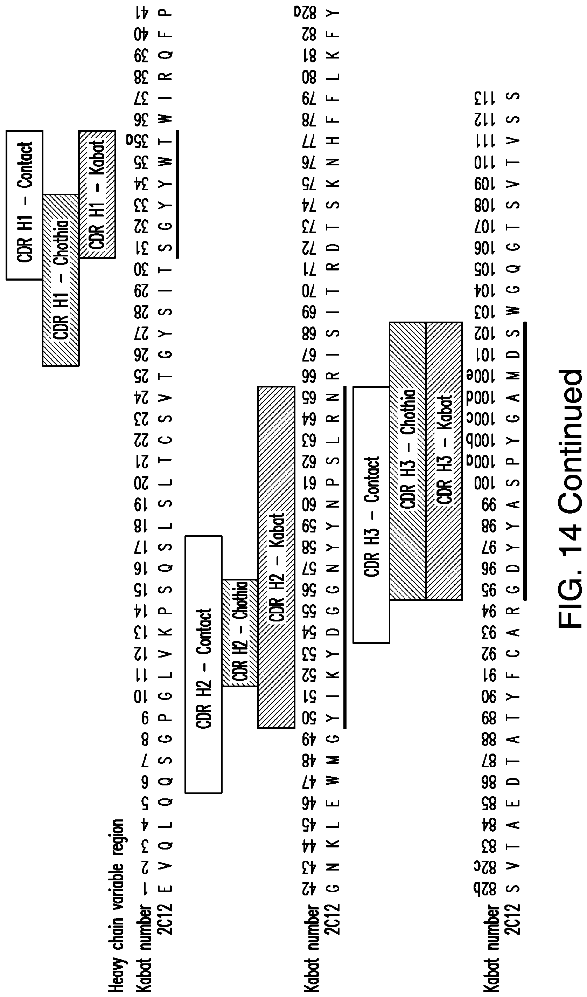

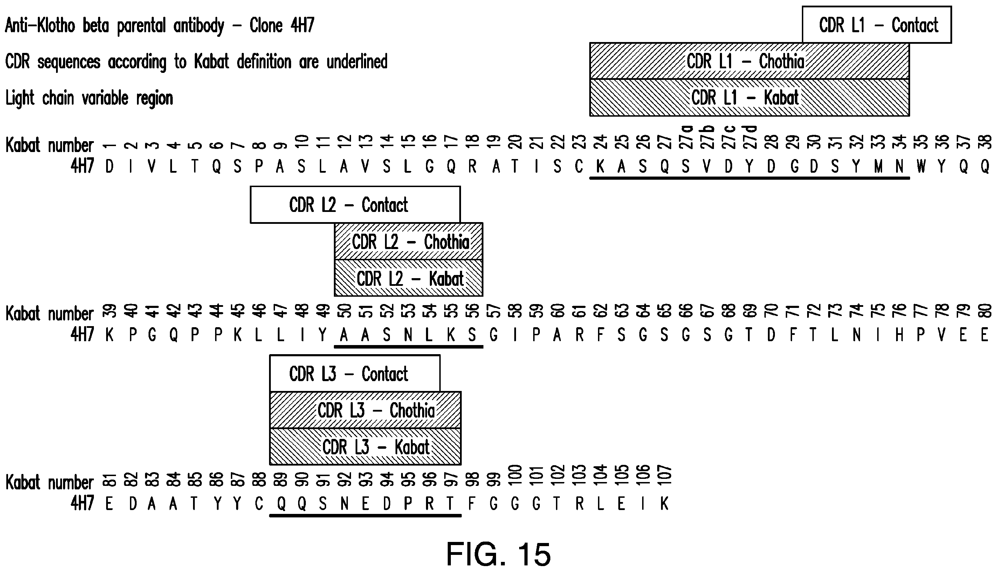

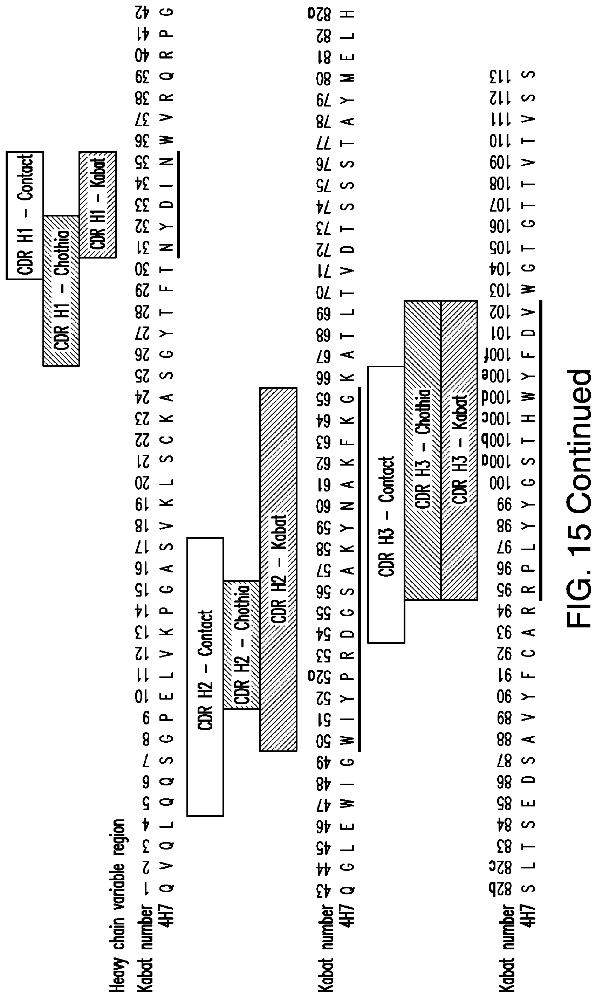

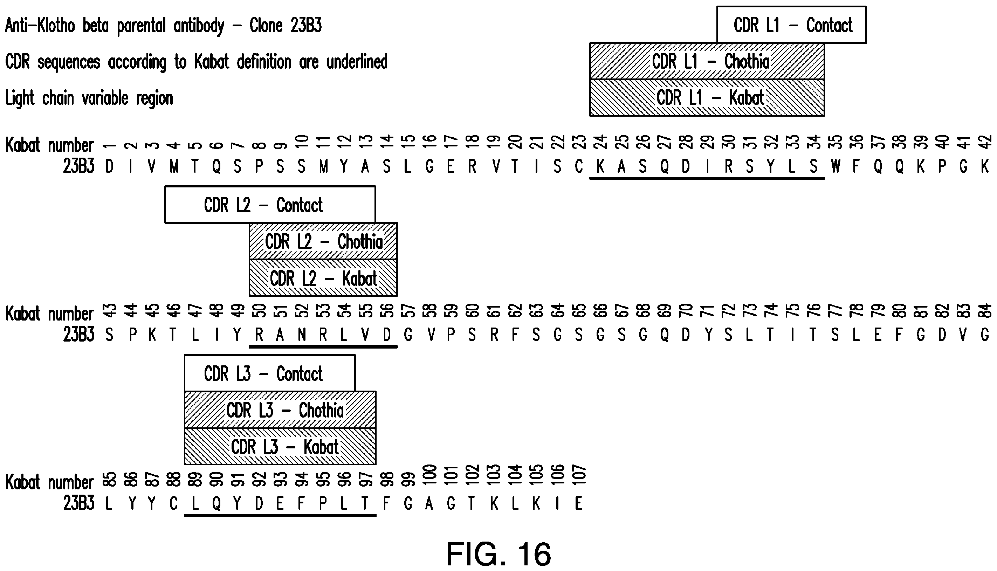

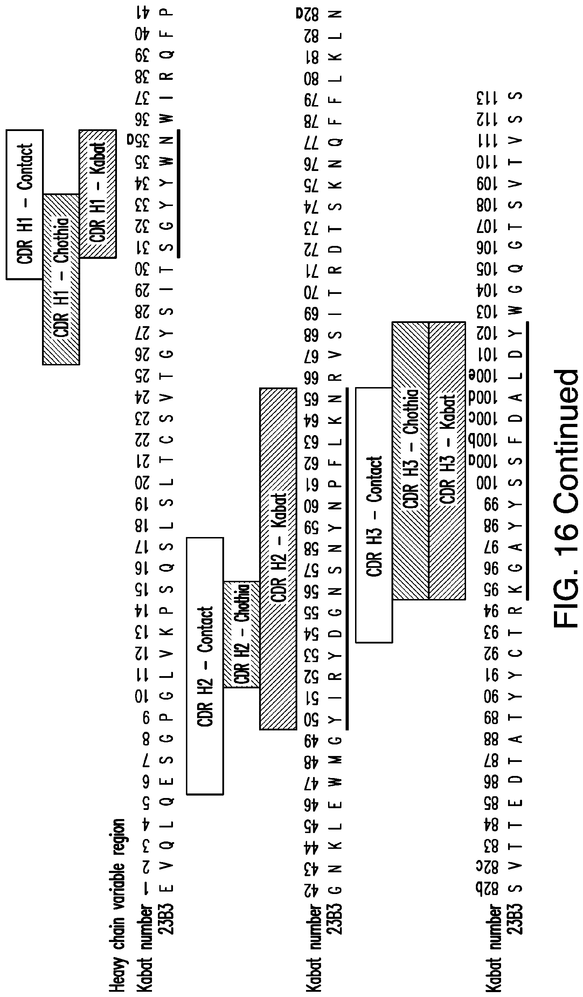

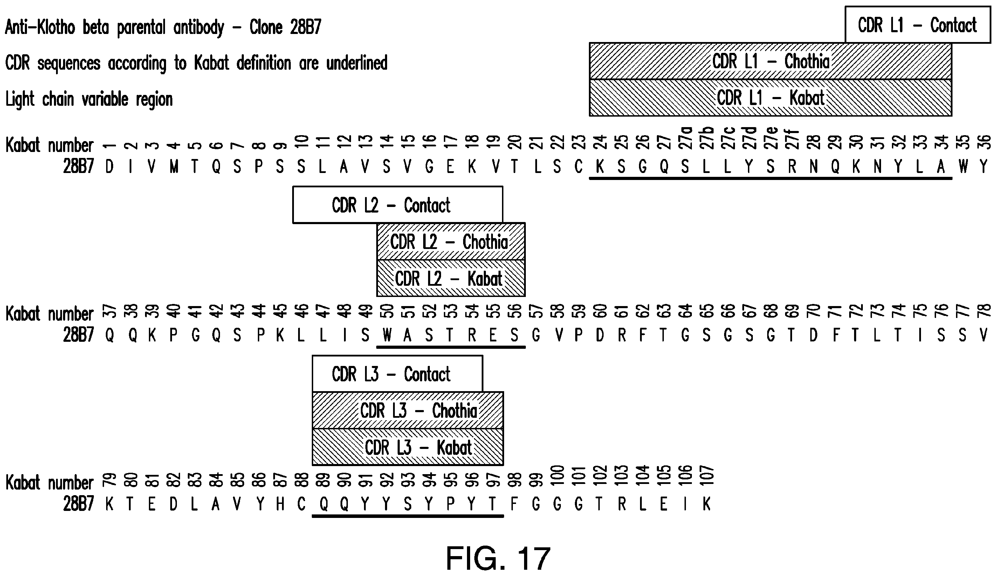

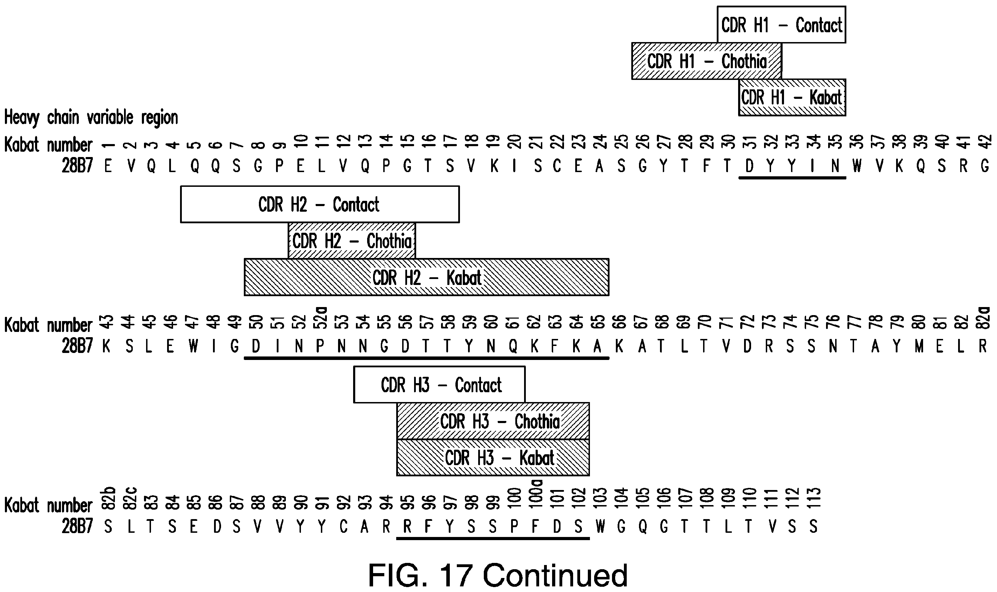

These five anti-KLB antibodies, 12B8, 2C12, 4H7, 23B3 and 28B7, were shown to function as KLB/FGFR1c agonist antibodies (Figures 4 and 5). The heavy chain variable region and light chain variable region CDR sequences for these 5 antibodies are shown in Tables 2 and 3, respectively. The full length heavy and light chain sequences of these anti-KLB antibodies are shown in Table 4 and in Figures 13-17. These antibodies are referred to herein as the parental antibodies.

**Table 2. Heavy chain variable region CDR sequences for anti-KLB antibodies.**

| **Antibody** | **CDR H1** | **CDR H2** | **CDR H3** |
|---|---|---|---|
| 12B8 | NYGMN (SEQ ID NO: 3) | WIDTDTGEATYTDDFKG (SEQ ID NO: 8) | EEYGLFGFPY (SEQ ID NO: 13) |
| 2C12 | SGYYWT (SEQ ID NO: 4) | YIKYDGGNYYNPSLRN (SEQ ID NO: 9) | GDYYASPYGAMDS (SEQ ID NO: 14) |
| 4H7 | NYDIN (SEQ ID NO: 5) | WIYPRDGSAKYNAKFKG (SEQ ID NO: 10) | RPLYYGSTHWYFDV (SEQ ID NO: 15) |
| 23B3 | SGYYWN (SEQ ID NO: 6) | YIRYDGNSNYNPFLKN (SEQ ID NO: 11) | KGAYYSSFDALDY (SEQ ID NO: 16) |
| 28B7 | DYYIN (SEQ ID NO: 7) | DINPNNGDTTYNQKFKA (SEQ ID NO: 12) | RFYSSPFDS (SEQ ID NO: 17) |

**Table 3. Light chain variable region CDR sequences for the anti-KLB antibodies.**

| **Antibody** | **CDR L1** | **CDR L2** | **CDR L3** |
|---|---|---|---|
| 12B8 | KASEDIYNRLA (SEQ ID NO: 18) | AATSLET (SEQ ID NO: 23) | QQYWSNPLT (SEQ ID NO: 28) |
| 2C12 | HASQDIDVWLS | KSSILHT | QQGLSYPFT |
| | (SEQ ID NO: 19) | (SEQ ID NO: 24) | (SEQ ID NO: 29) |
| 4H7 | KASQSVDYDGDSYMN (SEQ ID NO: 20) | AASNLKS (SEQ ID NO: 25) | QQSNEDPRT (SEQ ID NO: 30) |
| 23B3 | KASQDIRSYLS (SEQ ID NO: 21) | RANRLVD (SEQ ID NO: 26) | LQYDEFPLT (SEQ ID NO: 31) |
| 28B7 | KSGQSLLYSRNQKNYLA (SEQ ID NO: 22) | WASTRES (SEQ ID NO: 27) | QQYYSYPYT (SEQ ID NO: 32) |

**Table 4. Heavy and light chain variable regions of the identified anti-KLB antibodies.**

| **Antibody** | **Light Chain Variable Region** | **Heavy Chain Variable Region** |
|---|---|---|
| 12B8 | DIQMTQSSSSFSVSLGDRVTITC KASEDIYNRLAWYQQKPGSAP RLLISAATSLETGVPSRFSGSGS GKDYTLSITSLQTEDVATYYCQ QYWSNPLTFGAGTKLELK (SEQ ID NO: 33) | QIQLVQSGPELKKPGETAKISCKASG YAFSNYGMNWVKQAPGKDLKWMG WIDTDTGEATYTDDFKGRFVFSLETS ASTAYLQINNLKNEDMATYFCAREE YGLFGFPYWGHGTLVTVSA (SEQ ID NO: 34) |
| 2C12 | DIVMTQSPSSLSASLGDTITITC HASQDIDVWLSWYQQKPGNVP QLLISKSSILHTGVPSRFSGGGS GTGFTLTISSLQPEDIATYYCQQ GLSYPFTFGSGTKLKIK (SEQ ID NO: 35) | EVQLQQSGPGLVKPSQSLSLTCSVTG YSITSGYYWTWIRQFPGNKLEWMGY IKYDGGNYYNPSLRNRISITRDTSKN HFFLKFYSVTAEDTATYFCARGDYY ASPYGAMDSWGQGTSVTVSS (SEQ ID NO: 36) |
| 4H7 | DIVLTQSPASLAVSLGQRATISC KASQSVDYDGDSYMNWYQQK PGQPPKLLIYAASNLKSGIPARF SGSGSGTDFTLNIHPVEEEDAA TYYCQQSNEDPRTFGGGTRLEI K (SEQ ID NO: 37) | QVQLQQSGPELVKPGASVKLSCKAS GYTFTNYDINWVRQRPGQGLEWIG WIYPRDGSAKYNAKFKGKATLTVDT SSSTAYMELHSLTSEDSAVYFCARRP LYYGSTHWYFDVWGTGTTVTVSS (SEQ ID NO: 38) |
| 23B3 | DIVMTQSPSSMYASLGERVTIS CKASQDIRSYLSWFQQKPGKSP KTLIYRANRLVDGVPSRFSGSG SGQDYSLTITSLEFGDVGLYYC LQYDEFPLTFGAGTKLKIE (SEQ ID NO: 39) | EVQLQESGPGLVKPSQSLSLTCSVTG YSITSGYYWNWIRQFPGNKLEWMG YIRYDGNSNYNPFLKNRVSITRDTSK NQFFLKLNSVTTEDTATYYCTRKGA YYSSFDALDYWGQGTSVTVSS (SEQ ID NO: 40) |
| 28B7 | DIVMTQSPSSLAVSVGEKVTLS CKSGQSLLYSRNQKNYLAWYQ QKPGQSPKLLISWASTRESGVP DRFTGSGSGTDFTLTISSVKTED LAVYHCQQYYSYPYTFGGGTR LEIK (SEQ ID NO: 41) | EVQLQQSGPELVQPGTSVKISCEASG YTFTDYYINWVKQSRGKSLEWIGDI NPNNGDTTYNQKFKAKATLTVDRSS NTAYMELRSLTSEDSVVYYCARRFY SSPFDSWGQGTTLTVSS (SEQ ID NO: 42) |

### Example 2: Screening of bispecific anti-KLB antibodies

In this Example, screening for bispecific anti-KLB antibodies was performed and the KLB/FGFR1c agonist activity of the bispecific anti-KLB antibodies was tested in 293T cells.

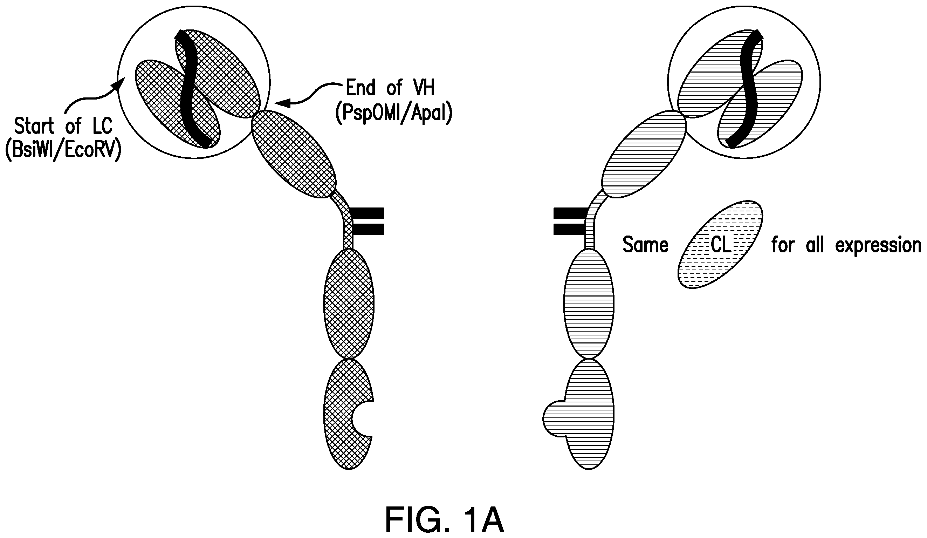

Bispecific antibodies that bind to two binding sites, e.g., epitopes, on KLB were generated using the anti-KLB antibody sequences of the parental antibodies listed above in Tables 1 and 2. See Table 5 for a summary of the bispecific antibodies that were generated. The bispecific antibodies were produced using the "knob-into-hole" heterodimerization technique with a single light chain variable region linked to a full heavy chain in a VLfH (variable light full heavy) format *(see* Figure 1). *See* Ridgway et al., Protein Engineering, Vol. 9:7, p617-621 (1996); Atwell et al. J. Mol. Biol. 270, 26-35 (1997); Spiess et al., Nat. Biotech. 31, 753-759 (2013).

To screen for bispecific antibodies that exhibited KLB/FGFR1c agonist activity, each light chain and heavy chain was expressed in the VLfH format. As shown in Figure 1, the VLfH format includes a full length heavy chain linked to a variable light chain domain through a linker comprising amino acids having the sequence set forth in SEQ ID NO: 68. To generate polypeptides having a light chain variable domain and full length heavy chain in the VLfH format, cDNA encoding ATGAYA (SEQ ID NO: 83) - a light chain variable domain up to R108 (according to the EU numbering system) - GGGGSGGGGSGGGGSGGGGS (linker; SEQ ID NO: 68) - heavy chain variable domain including up to CH1 residue F126 (according to the EU numbering system) and containing BsiWI restriction site at the 5' end and a PspOMI/ApaI restriction at the 3' end was commercially synthesized. The cDNA was digested with restriction enzymes BsiWI and PspOMI for sub-cloning into a standard pRK vector coding for the full heavy chain with various isotypes and hinge variants including knob and hole mutations in CH3. The resulting translated polypeptide comprises the following format: MGWSCIILFLVATATGAYA (signal peptide; SEQ ID NO: 69) - light chain variable domain up to R108 (according to the EU numbering system) - GGGGSGGGGSGGGGSGGGGS (linker; SEQ ID NO: 68) - full heavy chain.

The same light chain constant chain (CL) domain was used for each pair of VLfH polypeptides. The CL domain was amplified by polymerase chain reaction (PCR) from a plasmid containing cDNA for the human kappa (κ) light chain comprising the following nucleotide sequence set forth in SEQ ID NO: 66 using the following primers: 5'-TTTCCCTTTATCGATTGAATTCCACCATGGGATGGTCATGTATC ATCCTTTTTCTAGTAGCAACTGCAACTGGAGTACATTCAACTGTGGCTGCACC ATCTGTCTTC-3' (SEQ ID NO: 63); 5'-TTTCCCTTTAAGCTTAACACTCTCCCCTGTTGAAGC TCTTTGT-3' (SEQ ID NO: 64). SEQ ID NO: 66 is provided below. 
CGAACTGTGGCTGCACCATCTGTCTTCATCTTCCCGCCATCTGATGAGCAGTT
GAAATCTGGAACTGCTTCTGTTGTGTGCCTGCTGAATAACTTCTATCCCCGTG
AGGCCAAAGTACAGTGGAAGGTGGATAACGCCCTCCAATCGGGTAACTCCC
AGGAGAGTGTCACAGAGCAGGACAGCAAGGACAGCACCTACAGCCTCAGCA
GCACCCTGACGCTGAGCAAAGCAGACTACGAGAAACACAAAGTCTACGCCT
GCGAAGTCACCCATCAGGGCCTGAGCTCGCCCGTCACAAAGAGCTTCAACAG
GGGAGAGTGT (SEQ ID NO: 66).
 SEQ ID NO: 66 encodes an amino acid sequence set forth in SEQ ID NO: 65, provided below.



(SEQ ID NO: 65).

Amplified DNA was then digested with restriction enzymes ClaI and HindIII for sub-cloning into a standard pRK vector containing the same restriction enzyme sites as unique sites before the start of the open reading frame (ORF) and after the stop codon, resulting in polypeptide having the amino acid sequence set forth in SEQ ID NO: 67, provided below. MGWSCIILFLVATATGVHSTVAAPSVFIFPPSDEQLKSGTASVVCLLNNFYPREA KVQWKVDNALQSGNSQESVTEQDSKDSTYSLSSTLTLSKADYEKHKVYACEVT HQGLSSPVTKSFNRGEC (SEQ ID NO: 67). The start of the kappa constant light is T109 (according to the EU numbering system), but not limited to T109 and includes the R108 and the prior elbow residues. This constant light chain (CL) DNA was cloned only once and reused for pairing with VLfH DNA disclosed above.

Equal amounts of the VLfH and CL pRK DNAs were mixed for expression in various volumes of transient transfection cultures of CHO (Wong et al. (2010) Biotechnol. Bioeng., 106:751-763) or HEK293T (Bos et al. (2014) Journal of Biotechnology, 180:10-16) cells as previously described to promote heterodimerization of bispecific antibodies. The CL domain was expressed as a distinct polypeptide from the VLfH polypeptides. It has been previously shown that the CL domain folds autonomously and does not interact with BiP during folding (Hellman et al. (1999) J. Cell Biol. 144:21-30). In addition, the CL domain can be efficiently secreted by itself because it does not have an ER retention signal that stalls secretion until it is paired with the heavy chain (HC). Light chain (LC) and surrogate light chain (λ5) (Bankovich et al. (2007) Science 316:291-294) can interact with the HC via two domains. In addition to the CL:CH1 interface, VL (for LC) and first beta strand (for λ5) provide additional stability during the interaction. It is currently unknown if association kinetics for isolated CL are fast and tight enough to successfully (i) displace chaperones such as BiP on CH1, (ii) stably pair with CH1 and (iii) stabilize CH1 to enable the final proline isomerization in CH1 to enter the final folding state (Feige et al. (2009) Mol Cell. 34:569-579). Without the contribution of the interaction of the variable domain, the CL:CH1 interaction may have been too transient to allow proper folding of CH1 and rescue the folding defect of the VL-HC fusion.

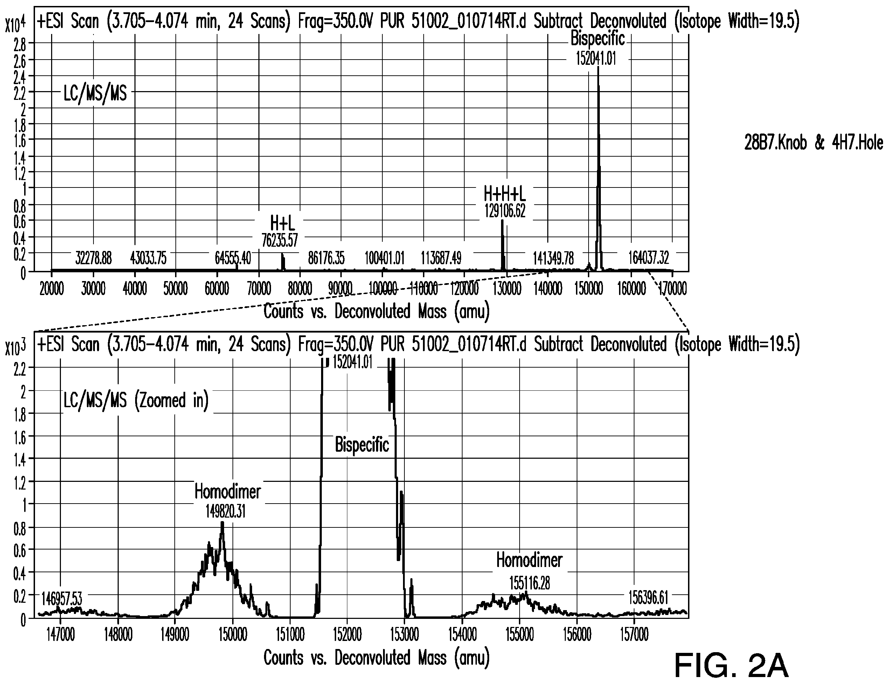

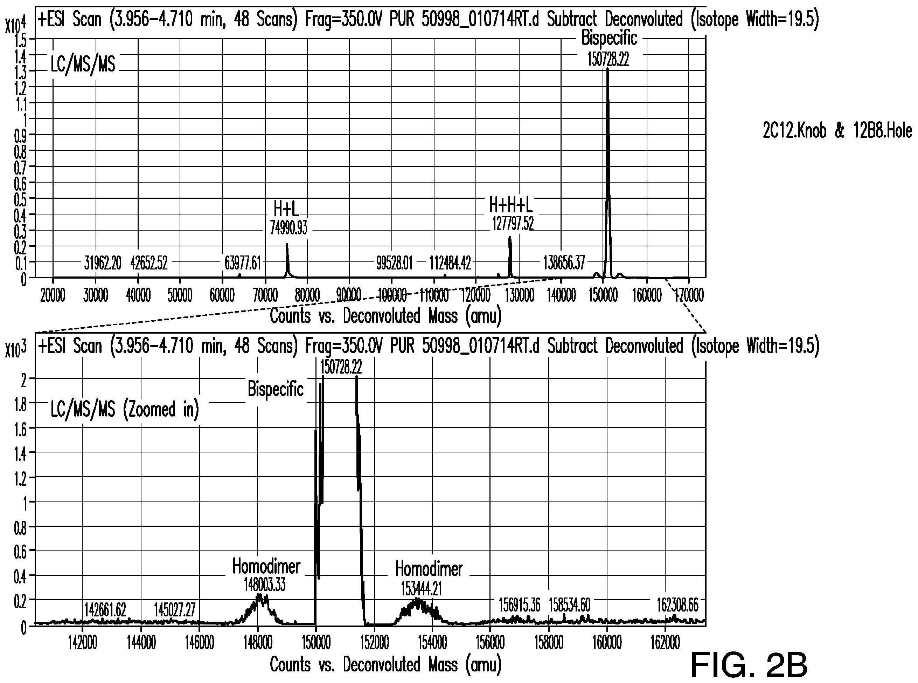

To determine whether the coexpression of two VLfH polypeptides resulted in the heterodimerization, mass spectrometry was performed. Mass spectrometric data was acquired using an Agilent 6230 TOF LC-MS system and 1290 Infinity HPLC (Agilent Technology, Santa Clara, CA, USA). The bispecific antibodies were purified using a 4.6 mm x 50 mm PLRP-S reversed-phase column (Agilent Technology). Intact masses were obtained by Maximum Entropy Deconvolution using MassHunter software (Qualitative Analysis B.04.00). As shown in Figure 2A-B, coexpression of the VLfHs resulted in the heterodimerization of the two VLfH polypeptides. The disclosed method resulted in greater than 95% heterodimerization and less than 5% homodimerization.

**Table 5. Bispecific anti-KLB antibodies**

| | | | | |
|---|---|---|---|---|
| 12B8 - 2C12 | 12B8 - 28B7 | 2C12 - 23B3 | 4H7 - 28B7 | 23B3 - 28B7 |
| 12B8 - 4H7 | 2C12 - 4H7 | 2C12 - 28B7 | 4H7 - 23B3 | 12B8 - 23B3 |

To determine the agonistic activity of the VLfH-formatted bispecific antibodies, a luciferase assay was performed. HEK293T cells were cultured in Dulbecco's Modified Eagle Medium (DMEM) + 10% fetal bovine serum (FBS), GlutaMax (Life Technologies) and Antibiotic-Antimycotic (Life Technologies), and transiently-transfected with expression vectors encoding Renilla luciferase (pRL-SV40, Promega), FGFR1c, a transcriptional activator (pFA2-Elk1, Stratagene), and a firefly luciferase reporter driven by GAL4 binding sites (pFR-Luc, Stratagene), using FuGENE^{®} HD Transfection Reagent (Roche). On the following day, the transfected cells were cultured for an additional 6-8 h in serum-free media and each of the bispecific anti-KLB antibodies and the corresponding parental monoclonal antibodies were tested at increasing concentrations. Recombinant human FGF21 (R&D) was used as a reference in some luciferase experiments. Cells were lysed with passive lysis buffer (Promega) and either incubated at 4°C for one hour, or at -20°C overnight. The cellular luciferase activity was determined using DUAL-GLO^{®} Luciferase Assay System (Promega) and EnVision^{®} Multilabel Reader (PerkinElmer). Firefly luciferase activity was normalized to the co-expressed Renilla luciferase activity, and the results were plotted as relative light units (RLU) as a function of ligand concentration.

In HEK293TdelFGFR1 cells, the FGFR1 gene was inactivated with the CRISPR/Cas9 technology, using guide RNAs aacttcactgtcttggcagccgg and gatctccaggtacaggggcgagg. Cells were transfected with a Cas9-expression plasmid driven by the CMV promoter, as well as plasmids expressing each guide RNA under the control of the U6 promoter. Three days after transfection, colonies were sorted at one cell per well in 96-well plates containing conditioned medium, consisting of 75% DMEM and 25% supernatant from transfected cultures that had been previously filtered thought a 22 micron filter. Colonies were screened for the absence of FGFR1c surface expression by FACS. FGFR1-deficient clones were further confirmed to bear a deletion in the FGFR1 locus, using genomic DNA PCR using the primers ATGCTCTCCCCTCCTCGG (SEQ ID NO: 84) and AGGCCCCTGTGCAATAGATG (SEQ ID NO: 85). One FGFR1-negative clone was expanded and used for luciferase reporter assays.

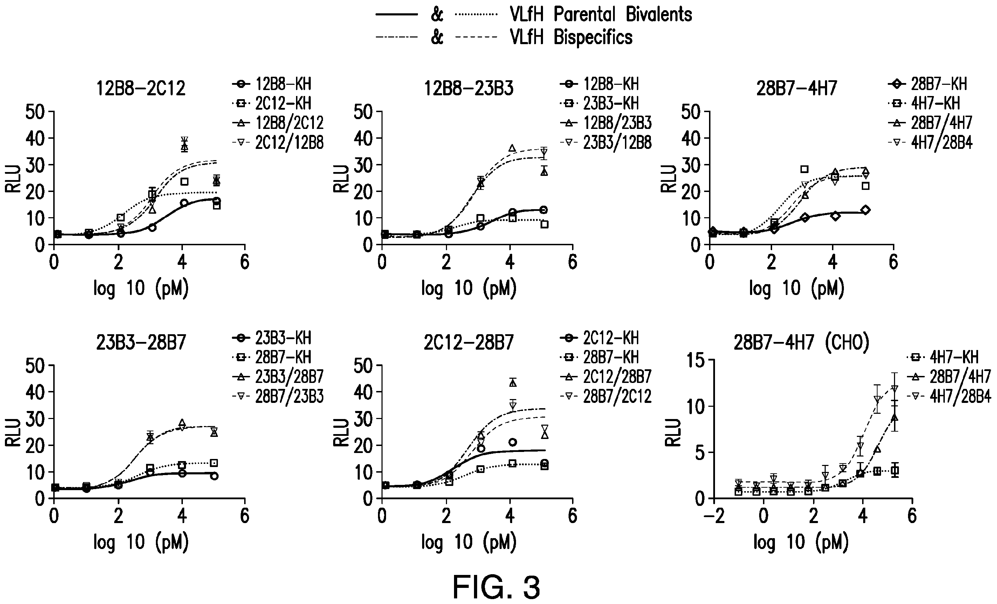

As shown in Figure 3, VLfH-formatted bispecific antibodies (tcBsIgG) exhibited agonistic activity. For example, the VLfH-formatted bispecific antibody 12B8/23B3 exhibited higher agonist activity than the VLfH-formatted parental antibodies, 12B8 and 23B3, alone (Figure 3). In addition, the VLfH-formatted bispecific antibody 12B8/23B7 exhibited higher agonist activity than the VLfH-formatted parental antibodies, 12B8 and 23B7, alone (Figure 3). These results show that the VLfH-based screening method to identify bispecific antibodies does not significantly affect agonistic activity and can be used to as a general screen for bispecific antibodies. The five biepitopic antibodies and the parental antibodies were subsequently produced in the same tcBsIgG format, but with two-column purification to remove the HMW species and other potential impurities. These monodisperse tcBsIgG preparations retained superior agonistic activity compared to the monospecific parents (data not shown), confirming that an additional purification was not necessary during the primary screening process.

### Example 3: Characterization of bispecific anti-KLB antibodies

The bispecific antibodies identified in Example 2, were reformatted into bispecific IgG antibodies and analyzed for KLB/FGFR1c agonist activity using the luciferase described in Example 2. As shown in Figures 4 and 5, the VLfH-formatted bispecific antibodies (tcBsIgG) that were identified as exhibiting agonistic activity also showed activity when reformatted into IgG antibodies, indicating that the screening method described in Example 2 did not result in any false positives. For example, the bispecific antibody 12B8/23B3 exhibited higher agonist activity than the parental antibodies, 12B8 and 23B3, alone. Similar results were observed for the bispecific anti-KLB antibodies, 12B8/2C12, 23B3/28B7, 2C12/28B7 and 4H7/28B7 (Figures 4 and 5). These results indicate that the disclosed bispecific antibodies exhibit higher agonist activity than their respective parental monospecific antibodies alone.

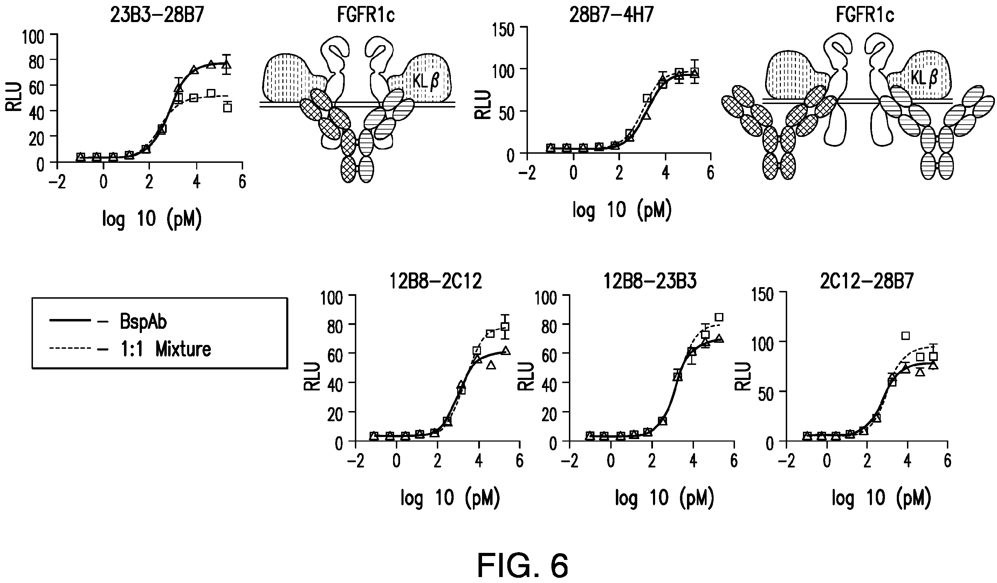

The effectiveness of the bispecific anti-KLB antibodies were analyzed in comparison to the activity of a 1:1 mixture of the corresponding pair of parental antibodies. For example, the activity of the bispecific antibody 23B3 - 28B7 was compared to the activity of a 1:1 mixture of 23B3 to 28B7. As shown in Figure 6, one pair of the complex-specific anti-KLB bispecific antibodies, 23B3/28B7, required locked bispecificity, *i.e.,* the agonistic effect of 23B3 and 28B7 binding sites is greater if these variable regions are part of the same bispecific antibody than if they act as part of two distinct monoclonal antibodies.

### Example 4: Agonist activity of KLB bispecific antibodies requires FGFR1c

In this Example, the disclosed bispecific antibodies were further analyzed to determine whether their agonist activity was dependent on the expression FGFR1c and/or KLB.

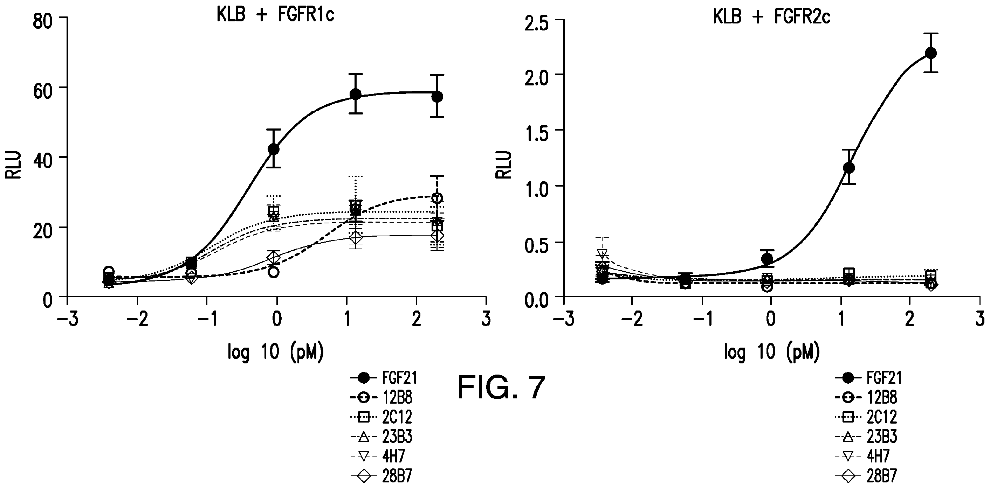

When tested in the GAL4-Elk1-based luciferase assay in HEK293T cells expressing FGFR1c with KLB, or expressing FGFR2c with KLB, it was observed that the agonist activity of the anti-KLB antibodies required the expression of KLB and required the expression of FGFR1c (Figure 7). The bispecific antibodies were observed to induce luciferase activity in a dose-dependent manner in cells expressing recombinant FGFR1c and hKLB, but not in cells coexpressing FGFR2c, a receptor very closely related to FGFR1c, and KLB, indicating that these bispecific antibodies act as a KLB-dependent FGFR agonist (Figure 7).

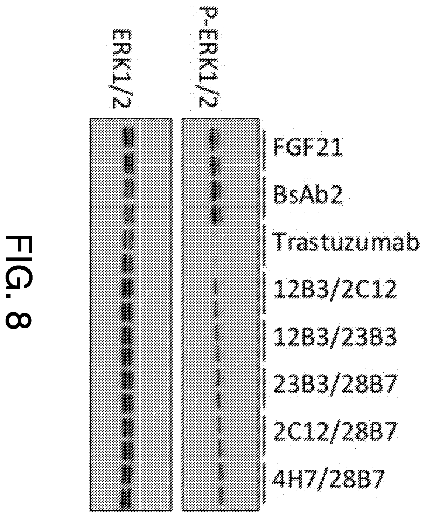

Furthermore, bispecific anti-KLB antibodies exhibited activity to induce phosphorylation of the MAPK signaling intermediates such as ERK in primary human adipocytes, which represent the relevant cell type for the anti-diabetic activity of FGF21 (Figure 8). FGF21, anti-KLB/anti-FGFR1c bispecific antibody (BsAb2), a bispecific antibody with an FGFR1c arm and a KLB arm (lead antibody), and trastuzumab, an isotype-matched antibody, were used as controls.

To further understand the mode of action for the bispecific anti-KLB antibodies, their ability to initiate the MAPK phosphorylation cascade in HEK293T cells expressing KLB and FGFR1c was quantified using a luciferase-based reporter assay.

Antibody binding was determined by flow cytometry. Cells were plated at 2 x 10⁴ cells/well in 96-well tissue culture plates and grown overnight. The following day, each well was transfected 100 ng of plasmid DNA using the FugeneHD transfection reagent (Roche), according to the manufacturer's specifications. 48 hours thereafter, cells were dissociated, washed in cold PBS supplemented with 3% fetal calf serum (FCS), and incubated with 2 µg/ml of the specified anti-KLB antibody for 45 minutes on ice, washed again, then incubated with 4 µg/ml anti-human IgG-Alexa 488 (Life Technologies) for 45 minutes on ice. Cells were analyzed by flow cytometry using a FACSCalibur analyzer (Becton Dickinson). As shown in Table 6 below, the bispecific antibodies exhibited high EC₅₀ compared to the lead anti-KLB/anti-FGFR1c bispecific antibody, BsAb2. For example, the bispecific anti-KLB antibody 23B3/28B7 exhibited a very high EC₅₀ value of 0.63 nM, comparable to BsAb2, using the luciferase-based reporter assay *(see* Table 6).

**Table 6**

| **Antibody** | **EC₅₀ (nM)** |
|---|---|
| BsAb2 | 0.75 nM |
| 12B8/2C12 | 1.36 nM |
| 12B8/23B3 | 0.95 nM |
| 23B3/28B7 | 0.63 nM |
| 2C12/28B7 | 0.58 nM |
| 4H7/28B7 | 1.21 nM |

### Example 5: Epitope mapping of anti-KLB antibodies

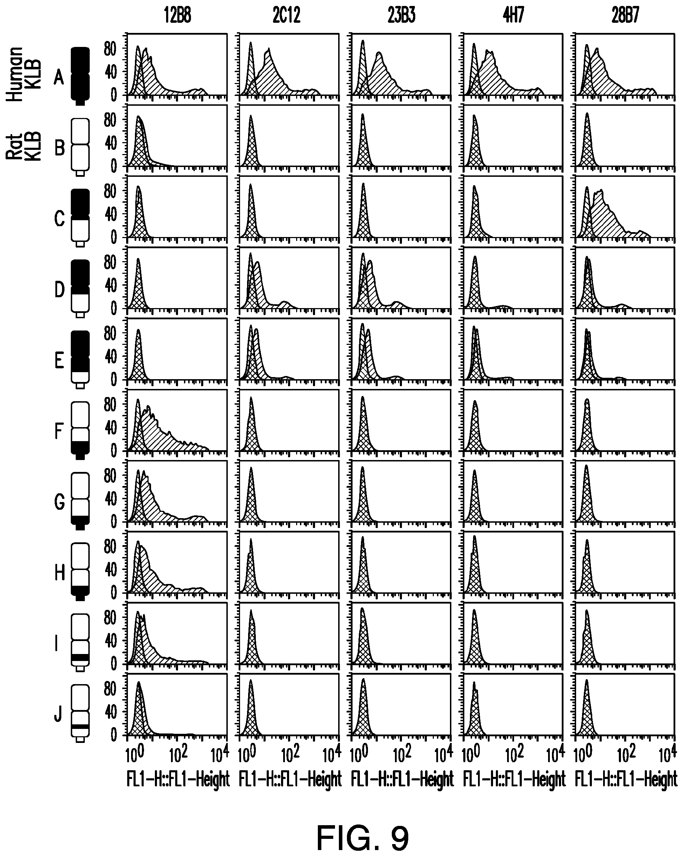

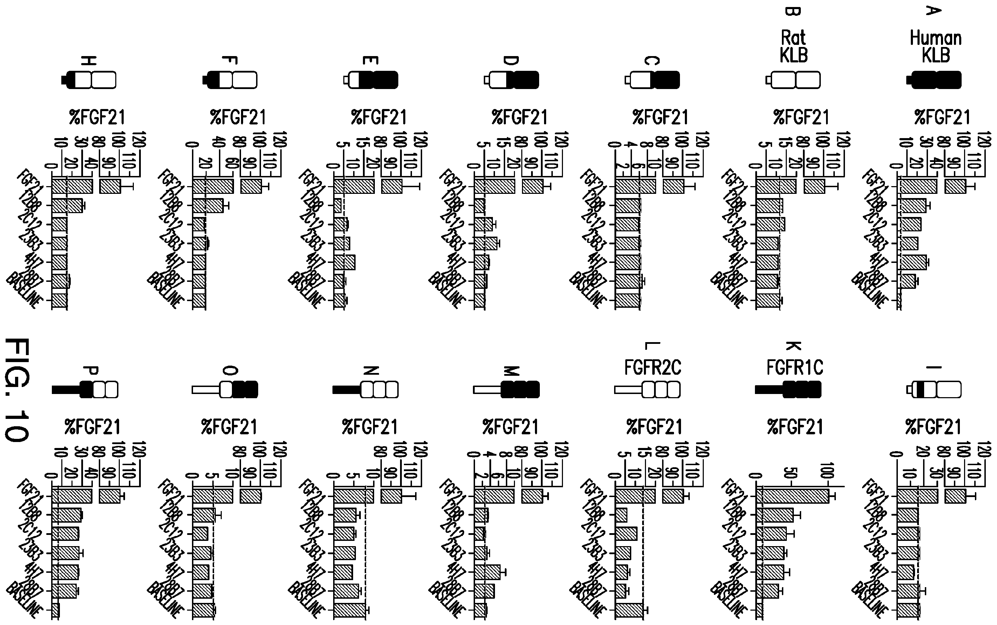

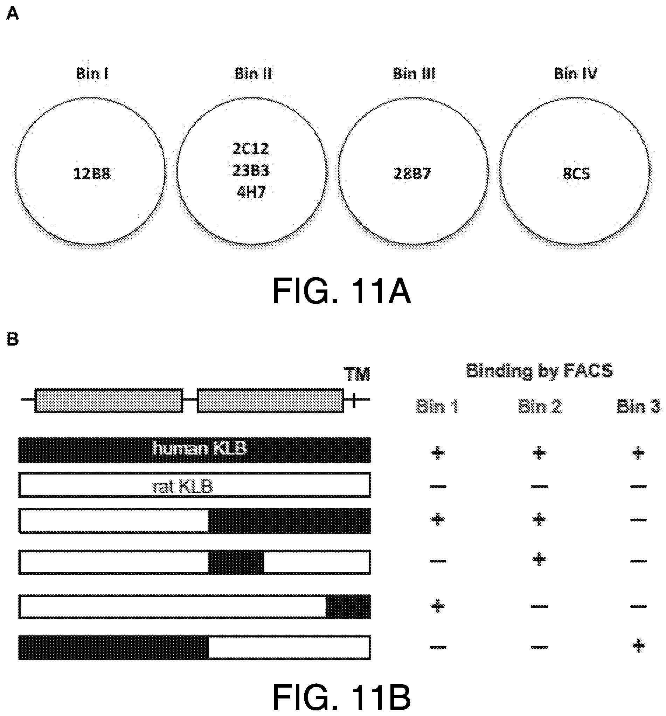

In this Example, epitope mapping of the anti-KLB antibodies identified in Example 1 were performed by FACS (Figure 9), by a luciferase-based reporter assay (Figure 10) and by epitope binning (Figures 11A-11B).

Chimeric human/rat KLB proteins (Figures 9 and 10) or chimeric human FGFR1c/FGFR2c proteins (Figure 10), described below, were expressed transiently on the surface of HEK293T or HEK293TdelFGFR1 cells, stained with the indicated antibodies, and analyzed by flow cytometry (Figure 9) or by luciferase-based reporter assay (Figure 10). In Figures 9 and 10, KLB and FGFR chimeras are represented as cartoons, with human KLB or FGFR1c sequence shown in black, and rat KLB or human FGFR2c sequences shown in white.

Based on these analyses, it can be inferred that the monoclonal antibody 12B8 binds at least part of the following amino acid sequence of KLB

IQFYNKVISSRGFPFENSSSRCSQTQENTECTVCLFLVQKKPLIFLGCCFFSTLVLL
LSIAIFQRQKRRKFWKAKNLQHIPLKKGKRVVS (SEQ ID NO: 43).

The monoclonal antibodies 2C12, 23B3 and 4H7 bind at least part of the following amino acid sequence of KLB:

ADSHWRAAERFLQFEIAWFAEPLFKTGDYPAAMREYIASKHRRGLSSSALPRLT
EAERRLLKGTVDFCALNHFTTRFVMHEQLAGSRYDSDRDI (SEQ ID NO: 44).

The monoclonal antibody 28B7 binds at least part of the following amino acid sequence of KLB:

FSGDGRAIWSKNPNFTPVNESQLFLYDTFPKNFFWGIGTGALQVEGSWK
KDGKGPSIWDHFIHTHLKNVSSTNGSSDSYIFLEKDLSALDFIGVSFYQFSISWPR
LFPDGIVTVANAKGLQYYSTLLDALVLRNIEPIVTLYHWDLPLALQEKYGGWKN
DTIIDIFNDYATYCFQMFGDRVKYWITIHNPYLVAWHGYGTGMHAPGEKGNLA
AVYTVGHNLIKAHSKVWHNYNTHFRPHQKGWLSITLGSHWIEPNRSENTMDIF
KCQQSMVSVLGWFANPIHGDGDYPEGMRKKLFSVLPIFSEAEKHEMRGTADFF
AFSFGPNNFKPLNTMAKMGQNVSLNLREALNWIKLEYNNPRILIAENGWFTDSR
VKTEDTTAIYMMKNFLSQVLQAIRLDEIRVFGYTAWSLLDGFEWQDAYTIRRGL
FYVDFNSKQKERKPKSSAHYYKQIIRENGFSLKESTPDVQGQFPCDFSWGVTESV
LKPESVASSPQFSDPHLYVWNATGNRLLHRVEGVRLKTRPAQCTDFVNIKKQLE
MLARMKVTHYRFALDWASVLPTGNLSAVNRQA (SEQ ID NO: 45).

Similarly, the following FGFR1c sequence is required for the monoclonal antibodies 12B8, 23B3, 4H7 and 28B7 to exert agonistic activity:

KTVALGSNVEFMCKVYSDPQPHIQWLKHIEVNGSKIGPDNLPYVQILKTAGVNT
TDKEMEVLHLRNVSFEDAGEYTCLAGNSIGLSHHSAWLTVLEALEERPAVMT
(SEQ ID NO: 46).

The following human/rat KLB and human FGFR1c/FGFR2c proteins and chimeras were cloned into vectors and expressed on the surface of HEK293T for FACS analysis or HEK293TdelFGFR1 for the luciferase assay. The human KLB sequence is depicted in bold, while homologous sequence in the proximity of human/rat sequence junctions are depicted in italics. Similarly, FGFR2c is depicted in bold, while junction sequences that are homologous between FGFR1c and FGFR2c are depicted in italics.

### Human KLB:

FSGDGRAIWSKNPNFTPVNESQLFLYDTFPKNFFWGIGTGALQVEGSWKKD
GKGPSIWDHFIHTHLKNVSSTNGSSDSYIFLEKDLSALDFIGVSFYQFSISWPR
LFPDGIVTVANAKGLQYYSTLLDALVLRNIEPIVTLYHWDLPLALQEKYGG
WKNDTIIDIFNDYATYCFQMFGDRVKYWITIHNPYLVAWHGYGTGMHAPG
EKGNLAAVYTVGHNLIKAHSKVWHNYNTHFRPHQKGWLSITLGSHWIEPN
RSENTMDIFKCQQSMVSVLGWFANPIHGDGDYPEGMRKKLFSVLPIFSEAE
KHEMRGTADFFAFSFGPNNFKPLNTMAKMGQNVSLNLREALNWIKLEYNN
PRILIAENGWFTDSRVKTEDTTAIYMMKNFLSQVLQAIRLDEIRVFGYTAWS
LLDGFEWQDAYTIRRGLFYVDFNSKQKERKPKSSAHYYKQIIRENGFSLKES
TPDVQGQFPCDFSWGVTESVLKPESVASSPQFSDPHLYVWNATGNRLLHRV
EGVRLKTRPAQCTDFVNIKKQLEMLARMKVTHYRFALDWASVLPTGNLSA
VNRQALRYYRCVVSEGLKLGISAMVTLYYPTHAHLGLPEPLLHADGWLNP
STAEAFQAYAGLCFQELGDLVKLWITINEPNRLSDIYNRSGNDTYGAAHNLL
VAHALAWRLYDRQFRPSQRGAVSLSLHADWAEPANPYADSHWRAAERFLQ
FEIAWFAEPLFKTGDYPAAMREYIASKHRRGLSSSALPRLTEAERRLLKGTV
DFCALNHFTTRFVMHEQLAGSRYDSDRDIQFLQDITRLSSPTRLAVIPWGVR
KLLRWVRRNYGDMDIYITASGIDDQALEDDRLRKYYLGKYLQEVLKAYLID
KVRIKGYYAFKLAEEKSKPRFGFFTSDFKAKSSIQFYNKVISSRGFPFENSSSR
CSQTQENTECTVCLFLVQKKPLIFLGCCFFSTLVLLLSIAIFQRQKRRKFWK
AKNLQHIPLKKGKRVVS (SEQ ID NO: 47).

### Homologous rat region corresponding to KLB protein A:

FSGDGKAIWDKKQYVSPVNPGQLFLYDTFPKNFSWGVGTGAFQVEGSWKADG
RGPSIWDRYVDSHLRGVNSTDRSTDSYVFLEKDLLALDFLGVSFYQFSISWPRLF

PNGTVAAVNAKGLQYYRALLDSLVLRNIEPIVTLYHWDLPLTLQEEYGGWKNA
TMIDLFNDYATYCFQTFGDRVKYWITIHNPYLVAWHGFGTGMHAPGEKGNLTA
VYTVGHNLIKAHSKVWHNYDKNFRPHQKGWLSITLGSHWIEPNRTENMEDVIN
CQHSMSSVLGWFANPIHGDGDYPEFMKTSSVIPEFSEAEKEEVRGTADFFAFSFG
PNNFRPSNTVVKMGQNVSLNLRQVLNWIKLEYDNPRILISENGWFTDSYIKTEDT
TAIYMMKNFLNQVLQAIKFDEIQVFGYTAWTLLDGFEWQDAYTTRRGLFYVDF
NSEQKERKPKSSAHYYKQIIQDNGFPLQESTPDMKGQFPCDFSWGVTESVLKPEF
TVSSPQFTDPHLYVWNVTGNRLLYRVEGVRLKTRPSQCTDYVSIKKRVEMLAK
MKVTHYQFALDWTSILPTGNLSKINRQVLRYYRCVVSEGLKLGISPMVTLYHPT
HSHLGLPMPLLSSGGWLNTNTAKAFQDYAGLCFKELGDLVKLWITINEPNRLSD
MYNRTSNDTYRAAHNLMIAHAQVWHLYDRQYRPVQHGAVSLSLHSDWAEPA
NPYVESHWKAAERFLQFEIAWFADPLFKTGDYPLAMKEYIASKKQRGLSSSVLP
RFTLKESRLVKGTIDFYALNHFTTRFVIHKQLNTNCSVADRDVQFLQDITRLSSPS
RLAVTPWGMRKLLGWIRRNYRDMDIYVTANGIDDLALEDDQIRKYYLEKYVQE
ALKAYLIDKVKIKGYYAFKLTEEKSKPRFGFFTSDFKAKSSVQFYSKLISSSGFSS
ENRSPACGQPPEDTECAICSFLTQKKPLIFFGCCFISTLAALLSITIFHHRKRRKFQK
ARNLQNIPLKKGHSRVFS (SEQ ID NO: 48).

### Human/rat KLB chimera:

FSGDGRAIWSKNPNFTPVNESQLFLYDTFPKNFFWGIGTGALQVEGSWKKD
GKGPSIWDHFIHTHLKNVSSTNGSSDSYIFLEKDLSALDFIGVSFYQFSISWPR
LFPDGIVTVANAKGLQYYSTLLDALVLRNIEPIVTLYHWDLPLALQEKYGG
WKNDTIIDIFNDYATYCFQMFGDRVKYWITIHNPYLVAWHGYGTGMHAPG
EKGNLAAVYTVGHNLIKAHSKVWHNYNTHFRPHQKGWLSITLGSHWIEPN
RSENTMDIFKCQQSMVSVLGWFANPIHGDGDYPEGMRKKLFSVLPIFSEAE
KHEMRGTADFFAFSFGPNNFKPLNTMAKMGQNVSLNLREALNWIKLEYNN
PRILIAENGWFTDSRVKTEDTTAIYMMKNFLSQVLQAIRLDEIRVFGYTAWS
LLDGFEWQDAYTIRRGLFYVDFNSKQKERKPKSSAHYYKQIIRENGFSLKES
TPDVQGQFPCDFSWGVTESVLKPESVASSPQFSDPHLYVWNATGNRLLHRV
EGVRLKTRPAQCTDFVNIKKQLEMLARMKVTHYRFALDWASVLPTGNLSA
VNRQALRYYRCVVSEGLKLGISAMVTLYHPTHSHLGLPMPLLSSGGWLNTNTAKA
FQDYAGLCFKELGDLVKLWITINEPNRLSDMYNRTSNDTYRAAHNLMIAHAQV
WHLYDRQYRPVQHGAVSLSLHSDWAEPANPYVESHWKAAERFLQFEIAWFAD
PLFKTGDYPLAMKEYIASKKQRGLSSSVLPRFTLKESRLVKGTIDFYALNHFTTR

FVIHKQLNTNCSVADRDVQFLQDITRLSSPSRLAVTPWGMRKLLGWIRRNYRD
MDIYVTANGIDDLALEDDQIRKYYLEKYVQEALKAYLIDKVKIKGYYAFKLTEE
KSKPRFGFFTSDFKAKSSVQFYSKLISSSGFSSENRSPACGQPPEDTECAICSFLTQ
KKPLIFFGCCFISTLAALLSITIFHHRKRRKFQKARNLQNIPLKKGHSRVFS (SEQ
ID NO: 49).

### Human/rat KLB chimera:

FSGDGRAIWSKNPNFTPVNESQLFLYDTFPKNFFWGIGTGALQVEGS
WKKDGKGPSIWDHFIHTHLKNVSSTNGSSDSYIFLEKDLSALDFIGVSFYQFS
ISWPRLFPDGIVTVANAKGLQYYSTLLDALVLRNIEPIVTLYHWDLPLALQE
KYGGWKNDTIIDIFNDYATYCFQMFGDRVKYWITIHNPYLVAWHGYGTGM
HAPGEKGNLAAVYTVGHNLIKAHSKVWHNYNTHFRPHQKGWLSITLGSH
WIEPNRSENTMDIFKCQQSMVSVLGWFANPIHGDGDYPEGMRKKLFSVLPI
FSEAEKHEMRGTADFFAFSFGPNNFKPLNTMAKMGQNVSLNLREALNWIK
LEYNNPRILIAENGWFTDSRVKTEDTTAIYMMKNFLSQVLQAIRLDEIRVFG
YTAWSLLDGFEWQDAYTIRRGLFYVDFNSKQKERKPKSSAHYYKQIIRENG
FSLKESTPDVQGQFPCDFSWGVTESVLKPESVASSPQFSDPHLYVWNATGNR
LLHRVEGVRLKTRPAQCTDFVNIKKQLEMLARMKVTHYRFALDWASVLPT
GNLSAVNRQALRYYRCVVSEGLKLGISAMVTLYYPTHAHLGLPEPLLHADG
WLNPSTAEAFQAYAGLCFQELGDLVKLWITINEPNRLSDIYNRSGNDTYGA
AHNLLVAHALAWRLYDRQFRPSQRGAVSLSLHADWAEPANPYVESHWKAAE
RFLQFEIAWFADPLFKTGDYPLAMKEYIASKKQRGLSSSVLPRFTLKESRLVKGT
IDFYALNHFTTRFVIHKQLNTNCSVADRDVQFLQDITRLSSPSRLAVTPWGMRKL
LGWIRRNYRDMDIYVTANGIDDLALEDDQIRKYYLEKYVQEALKAYLIDKVKIK
GYYAFKLTEEKSKPRFGFFTSDFKAKSSVQFYSKLISSSGFSSENRSPACGQPPED
TECAICSFLTQKKPLIFFGCCFISTLAALLSITIFHHRKRRKFQKARNLQNIPLKKG
HSRVFS (SEQ ID NO: 50).

### Human/rat KLB chimera:

FSGDGRAIWSKNPNFTPVNESQLFLYDTFPKNFFWGIGTGALQVEGSWKKD
GKGPSIWDHFIHTHLKNVSSTNGSSDSYIFLEKDLSALDFIGVSFYQFSISWPR
LFPDGIVTVANAKGLQYYSTLLDALVLRNIEPIVTLYHWDLPLALQEKYGG
WKNDTIIDIFNDYATYCFQMFGDRVKYWITIHNPYLVAWHGYGTGMHAPG
EKGNLAAVYTVGHNLIKAHSKVWHNYNTHFRPHQKGWLSITLGSHWIEPN
RSENTMDIFKCQQSMVSVLGWFANPIHGDGDYPEGMRKKLFSVLPIFSEAE

KHEMRGTADFFAFSFGPNNFKPLNTMAKMGQNVSLNLREALNWIKLEYNN
PRILIAENGWFTDSRVKTEDTTAIYMMKNFLSQVLQAIRLDEIRVFGYTAWS
LLDGFEWQDAYTIRRGLFYVDFNSKQKERKPKSSAHYYKQIIRENGFSLKES
TPDVQGQFPCDFSWGVTESVLKPESVASSPQFSDPHLYVWNATGNRLLHRV
EGVRLKTRPAQCTDFVNIKKQLEMLARMKVTHYRFALDWASVLPTGNLSA
VNRQALRYYRCVVSEGLKLGISAMVTLYYPTHAHLGLPEPLLHADGWLNP
STAEAFQAYAGLCFQELGDLVKLWITINEPNRLSDIYNRSGNDTYGAAHNLL
VAHALAWRLYDRQFRPSQRGAVSLSLHADWAEPANPYADSHWRAAERFLQ
FEIAWFAEPLFKTGDYPAAMREYIASKHRRGLSSSALPRLTEAERRLLKGTV
DFCALNHFTTRFVMHEQLAGSRYDSDRDIQFLQDITRLSSPSRLAVTPWGMRK
LLGWIRRNYRDMDIYVTANGIDDLALEDDQIRKYYLEKYVQEALKAYLIDKVKI
KGYYAFKLTEEKSKPRFGFFTSDFKAKSSVQFYSKLISSSGFSSENRSPACGQPPE
DTECAICSFLTQKKPLIFFGCCFISTLAALLSITIFHHRKRRKFQKARNLQNIPLKK
GHSRVFS (SEQ ID NO: 51).

### Human/rat KLB chimera:

FSGDGKAIWDKKQYVSPVNPGQLFLYDTFPKNFSWGVGTGAFQVEGSWKADG
RGPSIWDRYVDSHLRGVNSTDRSTDSYVFLEKDLLALDFLGVSFYQFSISWPRLF
PNGTVAAVNAKGLQYYRALLDSLVLRNIEPIVTLYHWDLPLTLQEEYGGWKNA
TMIDLFNDYATYCFQTFGDRVKYWITIHNPYLVAWHGFGTGMHAPGEKGNLTA
VYTVGHNLIKAHSKVWHNYDKNFRPHQKGWLSITLGSHWIEPNRTENMEDVIN
CQHSMSSVLGWFANPIHGDGDYPEFMKTSSVIPEFSEAEKEEVRGTADFFAFSFG
PNNFRPSNTVVKMGQNVSLNLRQVLNWIKLEYDNPRILISENGWFTDSYIKTEDT
TAIYMMKNFLNQVLQAIKFDEIQVFGYTAWTLLDGFEWQDAYTTRRGLFYVDF
NSEQKERKPKSSAHYYKQIIQDNGFPLQESTPDMKGQFPCDFSWGVTESVLKPEF
TVSSPQFTDPHLYVWNVTGNRLLYRVEGVRLKTRPSQCTDYVSIKKRVEMLAK
MKVTHYQFALDWTSILPTGNLSKINRQVLRYYRCVVSEGLKLGISPMVTLYHPT
HSHLGLPMPLLSSGGWLNTNTAKAFQDYAGLCFKELGDLVKLWITINEPNRLSD
MYNRTSNDTYRAAHNLMIAHAQVWHLYDRQYRPVQHGAVSLSLHSDWAEPA
NPYVESHWKAAERFLQFEIAWFADPLFKTGDYPLAMKEYIASKKQRGLSSSVLP
RFTLKESRLVKGTIDFYALNHFTTRFVIHKQLNTNCSVADRDVQFLQDITRLSSPT
RLAVIPWGVRKLLRWVRRNYGDMDIYITASGIDDQALEDDRLRKYYLGKY
LQEVLKAYLIDKVRIKGYYAFKLAEEKSKPRFGFFTSDFKAKSSIQFYNKVIS

SRGFPFENSSSRCSQTQENTECTVCLFLVQKKPLIFLGCCFFSTLVLLLSIAIF
QRQKRRKFWKAKNLQHIPLKKGKRVVS (SEQ ID NO: 52).

### Human/rat KLB chimera:

FSGDGKAIWDKKQYVSPVNPGQLFLYDTFPKNFSWGVGTGAFQVEGSWKADG
RGPSIWDRYVDSHLRGVNSTDRSTDSYVFLEKDLLALDFLGVSFYQFSISWPRLF
PNGTVAAVNAKGLQYYRALLDSLVLRNIEPIVTLYHWDLPLTLQEEYGGWKNA
TMIDLFNDYATYCFQTFGDRVKYWITIHNPYLVAWHGFGTGMHAPGEKGNLTA
VYTVGHNLIKAHSKVWHNYDKNFRPHQKGWLSITLGSHWIEPNRTENMEDVIN
CQHSMSSVLGWFANPIHGDGDYPEFMKTSSVIPEFSEAEKEEVRGTADFFAFSFG
PNNFRPSNTVVKMGQNVSLNLRQVLNWIKLEYDNPRILISENGWFTDSYIKTEDT
TAIYMMKNFLNQVLQAIKFDEIQVFGYTAWTLLDGFEWQDAYTTRRGLFYVDF
NSEQKERKPKSSAHYYKQIIQDNGFPLQESTPDMKGQFPCDFSWGVTESVLKPEF
TVSSPQFTDPHLYVWNVTGNRLLYRVEGVRLKTRPSQCTDYVSIKKRVEMLAK
MKVTHYQFALDWTSILPTGNLSKINRQVLRYYRCVVSEGLKLGISPMVTLYHPT
HSHLGLPMPLLSSGGWLNTNTAKAFQDYAGLCFKELGDLVKLWITINEPNRLSD
MYNRTSNDTYRAAHNLMIAHAQVWHLYDRQYRPVQHGAVSLSLHSDWAEPA
NPYVESHWKAAERFLQFEIAWFADPLFKTGDYPLAMKEYIASKKQRGLSSSVLP
RFTLKESRLVKGTIDFYALNHFTTRFVIHKQLNTNCSVADRDVQFLQDITRLSSPS
RLAVTPWGMRKLLGWIRRNYRDMDIYVTANGIDDQALEDDRLRKYYLGKYL
QEVLKAYLIDKVRIKGYYAFKLAEEKSKPRFGFFTSDFKAKSSIQFYNKVISS
RGFPFENSSSRCSQTQENTECTVCLFLVQKKPLIFLGCCFFSTLVLLLSIAIFQ
RQKRRKFWKAKNLQHIPLKKGKRVVS (SEQ ID NO: 53).

### Human/rat KLB chimera:

FSGDGKAIWDKKQYVSPVNPGQLFLYDTFPKNFSWGVGTGAFQVEGSWKADG
RGPSIWDRYVDSHLRGVNSTDRSTDSYVFLEKDLLALDFLGVSFYQFSISWPRLF
PNGTVAAVNAKGLQYYRALLDSLVLRNIEPIVTLYHWDLPLTLQEEYGGWKNA
TMIDLFNDYATYCFQTFGDRVKYWITIHNPYLVAWHGFGTGMHAPGEKGNLTA
VYTVGHNLIKAHSKVWHNYDKNFRPHQKGWLSITLGSHWIEPNRTENMEDVIN
CQHSMSSVLGWFANPIHGDGDYPEFMKTSSVIPEFSEAEKEEVRGTADFFAFSFG
PNNFRPSNTVVKMGQNVSLNLRQVLNWIKLEYDNPRILISENGWFTDSYIKTEDT
TAIYMMKNFLNQVLQAIKFDEIQVFGYTAWTLLDGFEWQDAYTTRRGLFYVDF
NSEQKERKPKSSAHYYKQIIQDNGFPLQESTPDMKGQFPCDFSWGVTESVLKPEF

TVSSPQFTDPHLYVWNVTGNRLLYRVEGVRLKTRPSQCTDYVSIKKRVEMLAK
MKVTHYQFALDWTSILPTGNLSKINRQVLRYYRCVVSEGLKLGISPMVTLYHPT
HSHLGLPMPLLSSGGWLNTNTAKAFQDYAGLCFKELGDLVKLWITINEPNRLSD
MYNRTSNDTYRAAHNLMIAHAQVWHLYDRQYRPVQHGAVSLSLHSDWAEPA
NPYVESHWKAAERFLQFEIAWFADPLFKTGDYPLAMKEYIASKKQRGLSSSVLP
RFTLKESRLVKGTIDFYALNHFTTRFVIHKQLNTNCSVADRDVQFLQDITRLSSPS
RLAVTPWGMRKLLGWIRRNYRDMDIYVTANGIDDLALEDDQIRKYYLEKYVQE
ALKAYLIDKVKIKGYYAFKLTEEKSKPRFGFFTSDFKAKSSIQFYNKVISSRGFPF
ENSSSRCSQTQENTECTVCLFLVQKKPLIFLGCCFFSTLVLLLSIAIFQRQKR
RKFWKAKNLQHIPLKKGKRVVS (SEQ ID NO: 54).

### Human/rat KLB chimera:

FSGDGKAIWDKKQYVSPVNPGQLFLYDTFPKNFSWGVGTGAFQVEGSWKADG
RGPSIWDRYVDSHLRGVNSTDRSTDSYVFLEKDLLALDFLGVSFYQFSISWPRLF
PNGTVAAVNAKGLQYYRALLDSLVLRNIEPIVTLYHWDLPLTLQEEYGGWKNA
TMIDLFNDYATYCFQTFGDRVKYWITIHNPYLVAWHGFGTGMHAPGEKGNLTA
VYTVGHNLIKAHSKVWHNYDKNFRPHQKGWLSITLGSHWIEPNRTENMEDVIN
CQHSMSSVLGWFANPIHGDGDYPEFMKTSSVIPEFSEAEKEEVRGTADFFAFSFG
PNNFRPSNTVVKMGQNVSLNLRQVLNWIKLEYDNPRILISENGWFTDSYIKTEDT
TAIYMMKNFLNQVLQAIKFDEIQVFGYTAWTLLDGFEWQDAYTTRRGLFYVDF
NSEQKERKPKSSAHYYKQIIQDNGFPLQESTPDMKGQFPCDFSWGVTESVLKPEF
TVSSPQFTDPHLYVWNVTGNRLLYRVEGVRLKTRPSQCTDYVSIKKRVEMLAK
MKVTHYQFALDWTSILPTGNLSKINRQVLRYYRCVVSEGLKLGISPMVTLYHPT
HSHLGLPMPLLSSGGWLNTNTAKAFQDYAGLCFKELGDLVKLWITINEPNRLSD
MYNRTSNDTYRAAHNLMIAHAQVWHLYDRQYRPVQHGAVSLSLHSDWAEPA
NPYVESHWKAAERFLQFEIAWFADPLFKTGDYPLAMKEYIASKKQRGLSSSVLP
RFTLKESRLVKGTIDFYALNHFTTRFVIHKQLNTNCSVADRDVQFLQDITRLSSPT
RLAVIPWGVRKLLRWVRRNYGDMDIYITASGIDDQALEDDRLRKYYLGKY
LQEVLKAYLIDKVRIKGYYAFKLAEEKSKPRFGFFTSDFKAKSSVQFYSKLISSS
GFSSENRSPACGQPPEDTECAICSFLTQKKPLIFFGCCFISTLAALLSITIFHHRKRR
KFQKARNLQNIPLKKGHSRVFS (SEQ ID NO: 55).

### Human/rat KLB chimera:

FSGDGKAIWDKKQYVSPVNPGQLFLYDTFPKNFSWGVGTGAFQVEGSWKADG
RGPSIWDRYVDSHLRGVNSTDRSTDSYVFLEKDLLALDFLGVSFYQFSISWPRLF
PNGTVAAVNAKGLQYYRALLDSLVLRNIEPIVTLYHWDLPLTLQEEYGGWKNA
TMIDLFNDYATYCFQTFGDRVKYWITIHNPYLVAWHGFGTGMHAPGEKGNLTA
VYTVGHNLIKAHSKVWHNYDKNFRPHQKGWLSITLGSHWIEPNRTENMEDVIN
CQHSMSSVLGWFANPIHGDGDYPEFMKTSSVIPEFSEAEKEEVRGTADFFAFSFG
PNNFRPSNTVVKMGQNVSLNLRQVLNWIKLEYDNPRILISENGWFTDSYIKTEDT
TAIYMMKNFLNQVLQAIKFDEIQVFGYTAWTLLDGFEWQDAYTTRRGLFYVDF
NSEQKERKPKSSAHYYKQIIQDNGFPLQESTPDMKGQFPCDFSWGVTESVLKPEF
TVSSPQFTDPHLYVWNVTGNRLLYRVEGVRLKTRPSQCTDYVSIKKRVEMLAK
MKVTHYQFALDWTSILPTGNLSKINRQVLRYYRCVVSEGLKLGISPMVTLYHPT
HSHLGLPMPLLSSGGWLNTNTAKAFQDYAGLCFKELGDLVKLWITINEPNRLSD
MYNRTSNDTYRAAHNLMIAHAQVWHLYDRQYRPVQHGAVSLSLHSDWAEPA
NPYVESHWKAAERFLQFEIAWFADPLFKTGDYPLAMKEYIASKKQRGLSSSVLP
RFTLKESRLVKGTIDFYALNHFTTRFVIHKQLNTNCSVADRDVQFLQDITRLSSPT
RLAVIPWGVRKLLRWVRRNYGDMDIYITASGIDDLALEDDQIRKYYLEKYVQ
EALKAYLIDKVKIKGYYAFKLTEEKSKPRFGFFTSDFKAKSSVQFYSKLISSSGFS
SENRSPACGQPPEDTECAICSFLTQKKPLIFFGCCFISTLAALLSITIFHHRKRRKFQ
KARNLQNIPLKKGHSRVFS (SEQ ID NO: 56).

### Human FGFR1c:

RPSPTLPEQAQPWGAPVEVESFLVHPGDLLQLRCRLRDDVQSINWLRDGVQ
LAESNRTRITGEEVEVQDSVPADSGLYACVTSSPSGSDTTYFSVNVSDALPSS
EDDDDDDDSSSEEKETDNTKPNPVAPYWTSPEKMEKKLHAVPAAKTVKFK
CPSSGTPNPTLRWLKNGKEFKPDHRIGGYKVRYATWSIIMDSVVPSDKGNY
TCIVENEYGSINHTYQLDVVERSPHRPILQAGLPANKTVALGSNVEFMCKVY
SDPQPHIQWLKHIEVNGSKIGPDNLPYVQILKTAGVNTTDKEMEVLHLRNV
SFEDAGEYTCLAGNSIGLSHHSAWLTVLEALEERPAVMTSPLYLEIIIYCTGA
FLISCMVGSVIVYKMKSGTKKSDFHSQMAVHKLAKSIPLRRQVTVSADSSAS
MNSGVLLVRPSRLSSSGTPMLAGVSEYELPEDPRWELPRDRLVLGKPLGEG
CFGQVVLAEAIGLDKDKPNRVTKVAVKMLKSDATEKDLSDLISEMEMMKM
IGKHKNIINLLGACTQDGPLYVIVEYASKGNLREYLQARRPPGLEYCYNPSH
NPEEQLSSKDLVSCAYQVARGMEYLASKKCIHRDLAARNVLVTEDNVMKIA
DFGLARDIHHIDYYKKTTNGRLPVKWMAPEALFDRIYTHQSDVWSFGVLL

WEIFTLGGSPYPGVPVEELFKLLKEGHRMDKPSNCTNELYMMMRDCWHA
VPSQRPTFKQLVEDLDRIVALTSNQEYLDLSMPLDQYSPSFPDTRSSTCSSGE
DSVFSHEPLPEEPCLPRHPAQLANGGLKRR (SEQ ID NO: 57).

### Human FGFR2c:

RPSFSLVEDTTLEPEEPPTKYQISQPEVYVAAPGESLEVRCLLKDAAVISWTKDG
VHLGPNNRTVLIGEYLQIKGATPRDSGLYACTASRTVDSETWYFMVNVTDAISS
GDDEDDTDGAEDFVSENSNNKRAPYWTNTEKMEKRLHAVPAANTVKFRCPAG
GNPMPTMRWLKNGKEFKQEHRIGGYKVRNQHWSLIMESVVPSDKGNYTCVVE
NEYGSINHTYHLDVVERSPHRPILQAGLPANASTVVGGDVEFVCKVYSDAQPHI
QWIKHVEKNGSKYGPDGLPYLKVLKAAGVNTTDKEIEVLYIRNVTFEDAGEYT
CLAGNSIGISFHSAWLTVLPAPGREKEITASPDYLEIAIYCIGVFLIACMVVTVILC
RMKNTTKKPDFSSQPAVHKLTKRIPLRRQVTVSAESSSSMNSNTPLVRITTRLSST
ADTPMLAGVSEYELPEDPKWEFPRDKLTLGKPLGEGCFGQVVMAEAVGIDKDK
PKEAVTVAVKMLKDDATEKDLSDLVSEMEMMKMIGKHKNIINLLGACTQDGPL
YVIVEYASKGNLREYLRARRPPGMEYSYDINRVPEEQMTFKDLVSCTYQLARG
MEYLASQKCIHRDLAARNVLVTENNVMKIADFGLARDINNIDYYKKTTNGRLP
VKWMAPEALFDRVYTHQSDVWSFGVLMWEIFTLGGSPYPGIPVEELFKLLKEG
HRMDKPANCTNELYMMMRDCWHAVPSQRPTFKQLVEDLDRILTLTTNEEYLD
LSQPLEQYSPSYPDTRSSCSSGDDSVFSPDPMPYEPCLPQYPHINGSVKT (SEQ ID
NO: 58).

### FGFR1c/FGFR2c chimera:

RPSPTLPEQAQPWGAPVEVESFLVHPGDLLQLRCRLRDDVQSINWLRDGVQ
LAESNRTRITGEEVEVQDSVPADSGLYACVTSSPSGSDTTYFSVNVSDALPSS
EDDDDDDDSSSEEKETDNTKPNPVAPYWTSPEKMEKKLHAVPAAKTVKFK
CPSSGTPNPTLRWLKNGKEFKPDHRIGGYKVRYATWSIIMDSVVPSDKGNY
TCIVENEYGSINHTYQLDVVERSPHRPILQAGLPANKTVALGSNVEFMCKVY
SDPQPHIQWLKHIEVNGSKIGPDNLPYVQILKTAGVNTTDKEMEVLHLRNV
SFEDAGEYTCLAGNSIGLSHHSAWLTVLEALEERPAVMTSPDYLEIAIYCIGV
FLIACMVVTVILCRMKNTTKKPDFSSQPAVHKLTKRIPLRRQVTVSAESSSSMNS
NTPLVRITTRLSSTADTPMLAGVSEYELPEDPKWEFPRDKLTLGKPLGEGCFGQV
VMAEAVGIDKDKPKEAVTVAVKMLKDDATEKDLSDLVSEMEMMKMIGKHKNI
INLLGACTQDGPLYVIVEYASKGNLREYLRARRPPGMEYSYDINRVPEEQMTFK

DLVSCTYQLARGMEYLASQKCIHRDLAARNVLVTENNVMKIADFGLARDINNI
DYYKKTTNGRLPVKWMAPEALFDRVYTHQSDVWSFGVLMWEIFTLGGSPYPGI
PVEELFKLLKEGHRMDKPANCTNELYMMMRDCWHAVPSQRPTFKQLVEDLDR
ILTLTTNEEYLDLSQPLEQYSPSYPDTRSSCSSGDDSVFSPDPMPYEPCLPQYPHIN
GSVKT (SEQ ID NO: 59).

### FGFR1c/FGFR2c chimera:

RPSFSLVEDTTLEPEEPPTKYQISQPEVYVAAPGESLEVRCLLKDAAVISWTKDG
VHLGPNNRTVLIGEYLQIKGATPRDSGLYACTASRTVDSETWYFMVNVTDAISS
GDDEDDTDGAEDFVSENSNNKRAPYWTNTEKMEKRLHAVPAANTVKFRCPAG
GNPMPTMRWLKNGKEFKQEHRIGGYKVRNQHWSLIMESVVPSDKGNYTCVVE
NEYGSINHTYHLDVVERSPHRPILQAGLPANASTVVGGDVEFVCKVYSDAQPHI
QWIKHVEKNGSKYGPDGLPYLKVLKAAGVNTTDKEIEVLYIRNVTFEDAGEYT
CLAGNSIGISFHSAWLTVLPAPGREKEITASPLYLEIIIYCTGAFLISCMVGSVIV
YKMKSGTKKSDFHSQMAVHKLAKSIPLRRQVTVSADSSASMNSGVLLVRPS
RLSSSGTPMLAGVSEYELPEDPRWELPRDRLVLGKPLGEGCFGQVVLAEAI
GLDKDKPNRVTKVAVKMLKSDATEKDLSDLISEMEMMKMIGKHKNIINLL
GACTQDGPLYVIVEYASKGNLREYLQARRPPGLEYCYNPSHNPEEQLSSKD
LVSCAYQVARGMEYLASKKCIHRDLAARNVLVTEDNVMKIADFGLARDIH
HIDYYKKTTNGRLPVKWMAPEALFDRIYTHQSDVWSFGVLLWEIFTLGGSP
YPGVPVEELFKLLKEGHRMDKPSNCTNELYMMMRDCWHAVPSQRPTFKQ
LVEDLDRIVALTSNQEYLDLSMPLDQYSPSFPDTRSSTCSSGEDSVFSHEPLP
EEPCLPRHPAQLANGGLKRR (SEQ ID NO: 60).

### FGFR1c/FGFR2c chimera:

RPSPTLPEQAQPWGAPVEVESFLVHPGDLLQLRCRLRDDVQSINWLRDGVQ
LAESNRTRITGEEVEVQDSVPADSGLYACVTSSPSGSDTTYFSVNVSDALPSS
EDDDDDDDSSSEEKETDNTKPNPVAPYWTSPEKMEKKLHAVPAAKTVKFK
CPSSGTPNPTLRWLKNGKEFKPDHRIGGYKVRYATWSIIMDSVVPSDKGNY
TCIVENEYGSINHTYQLDVVERSPHRPILQAGLPANASTVVGGDVEFVCKVY
SDAQPHIQWIKHVEKNGSKYGPDGLPYLKVLKAAGVNTTDKEIEVLYIRNV
TFEDAGEYTCLAGNSIGISFHSAWLTVLPAPGREKEITASPDYLEIAIYCIGVFL
IACMVVTVILCRMKNTTKKPDFSSQPAVHKLTKRIPLRRQVTVSAESSSSMNSNT
PLVRITTRLSSTADTPMLAGVSEYELPEDPKWEFPRDKLTLGKPLGEGCFGQVV

MAEAVGIDKDKPKEAVTVAVKMLKDDATEKDLSDLVSEMEMMKMIGKHKNII
NLLGACTQDGPLYVIVEYASKGNLREYLRARRPPGMEYSYDINRVPEEQMTFKD
LVSCTYQLARGMEYLASQKCIHRDLAARNVLVTENNVMKIADFGLARDINNID
YYKKTTNGRLPVKWMAPEALFDRVYTHQSDVWSFGVLMWEIFTLGGSPYPGIP
VEELFKLLKEGHRMDKPANCTNELYMMMRDCWHAVPSQRPTFKQLVEDLDRI
LTLTTNEEYLDLSQPLEQYSPSYPDTRSSCSSGDDSVFSPDPMPYEPCLPQYPHIN
GSVKT (SEQ ID NO: 61).

### FGFR1c/FGFR2c chimera:

RPSFSLVEDTTLEPEEPPTKYQISQPEVYVAAPGESLEVRCLLKDAAVISWTKDG
VHLGPNNRTVLIGEYLQIKGATPRDSGLYACTASRTVDSETWYFMVNVTDAISS
GDDEDDTDGAEDFVSENSNNKRAPYWTNTEKMEKRLHAVPAANTVKFRCPAG
GNPMPTMRWLKNGKEFKQEHRIGGYKVRNQHWSLIMESVVPSDKGNYTCVVE
NEYGSINHTYHLDVVERSPHRPILQAGLPANKTVALGSNVEFMCKVYSDPQPHI
QWLKHIEVNGSKIGPDNLPYVQILKTAGVNTTDKEMEVLHLRNVSFEDAGE
YTCLAGNSIGLSHHSAWLTVLEALEERPAVMTSPLYLEIIIYCTGAFLISCMV
GSVIVYKMKSGTKKSDFHSQMAVHKLAKSIPLRRQVTVSADSSASMNSGVL
LVRPSRLSSSGTPMLAGVSEYELPEDPRWELPRDRLVLGKPLGEGCFGQVV
LAEAIGLDKDKPNRVTKVAVKMLKSDATEKDLSDLISEMEMMKMIGKHKN
IINLLGACTQDGPLYVIVEYASKGNLREYLQARRPPGLEYCYNPSHNPEEQL
SSKDLVSCAYQVARGMEYLASKKCIHRDLAARNVLVTEDNVMKIADFGLA
RDIHHIDYYKKTTNGRLPVKWMAPEALFDRIYTHQSDVWSFGVLLWEIFTL
GGSPYPGVPVEELFKLLKEGHRMDKPSNCTNELYMMMRDCWHAVPSQRP
TFKQLVEDLDRIVALTSNQEYLDLSMPLDQYSPSFPDTRSSTCSSGEDSVFSH
EPLPEEPCLPRHPAQLANGGLKRR (SEQ ID NO: 62).

Epitope binning experiments (Figures 11A-11B) were performed by BioLayer Inferometry in 8-channel or 16-channel mode using anti-mouse Fc or anti-human Fc capture biosensors on the Octet Red384 system (Pall Life Sciences, Menlo Park, CA). The assay consisted of a seven-step binding cycle: 1) anti-mouse Fc biosensors were dipped into running buffer (1x kinetics buffer, ForteBio 18-5032) for 1 min to establish a baseline, 2) 20 µg/ml mouse IgG2a (reference) antibody was captured for 10 minutes, 3) another baseline was established for 1.5 minutes, 4) 100nM of human KLB was loaded for 10 minutes, a third baseline was established for 1.5 minutes, and 6) 5 µg/ml of the human IgG1 (test) antibodies were allowed to associate for 10 minutes and 7) dissociate for 10 minutes. Epitope binning with anti-human Fc capture biosensors was done similarly. The summary in Figure 11 indicates the bins determined for each antibody.

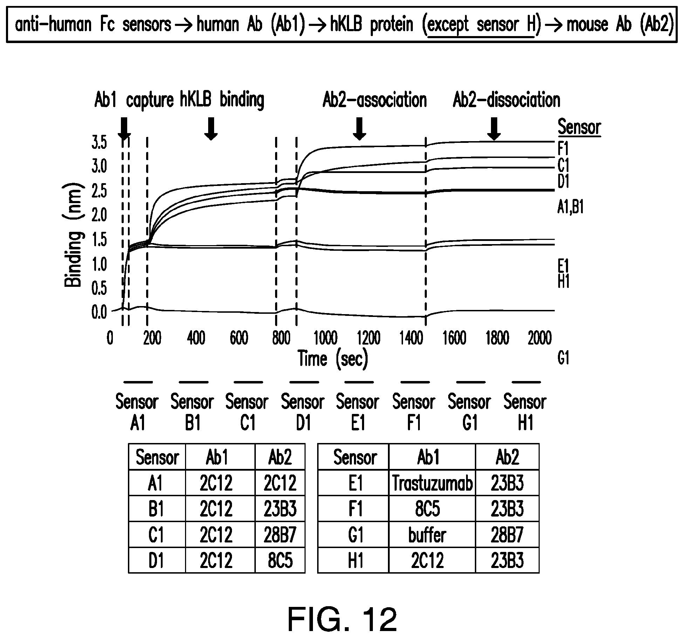

Figure 12 illustrates an example biolayer inferometry experiment. In this example, 2C12 (human IgG1) competes with 23B3, but not with 28B7 or 8C5 (mouse IgG) for binding to recombinant KLB.

### Example 6: Tri-chain bispecific IgG: An antibody platform for rapid

### bispecific antibody screening

### Materials and Methods

### Characterization of purified antibodies by capillary electrophoresis

Samples were analyzed on a Caliper GX II microfluidic system (PerkinElmer Biotechnology, Waltham, MA, USA). All samples were prepared as described previously (Kim, 2016).The chip was prepared according to the manufacturer's instructions provided in the LabChip GXII User Guide.

### Characterization of purified antibodies by LC-MS/MS

Mass spectrometric data was acquired using an Agilent 6230 TOF LC-MS system and 1290 Infinity HPLC (Agilent Technology, Santa Clara, CA). The IgG were separated with a 4.6mm x 50mm PLRP-S reversed phase column (Agilent Technology, Santa Clara, CA). Intact masses were obtained by Maximun Entropy Deconvolution using MassHunter software (Qualitative Analysis B.04.00).

### Biacore Kinetics analysis

For binding affinity determination of tcIgG and traditional IgG, surface plasmon resonance (SPR) measurements using a BIAcore T200 and ProteinA sensor chip was used and dilutions of monomeric human KLB as analyte were injected over the immobilized tcIgG or traditional IgG at 25 °C to determine monovalent affinities. Association rates (kon) and dissociation rates (koff) were calculated using a simple one-to-one Langmuir binding model. The equilibrium dissociation constant (Kd) was calculated as the ratio koff/kon.

### Western Blot

Human primary subcutaneous pre-adipocytes were acquired from Lonza (Walkersville, MD). Cells were grown and differentiated according to supplier's protocol. Briefly, cells were grown in preadipocyte basal medium-2 containing FBS, L-glutamine and GA-1000. Once confluent, cells were differentiated in growth media containing dexamethasone, indomethacin, and 3-isobutyl-1-methylxanthine (IBMX). For ERK signaling analysis, cells were differentiated for 10 days, grown in serum-free medium for 3 hours, and then further cultured for an additional hour with the indicated antibodies. Cell extracts were generated by lysing cells in 2x LDS buffer (Invitrogen, USA) containing protease and phosphatase inhibitor tablets (Roche, USA). Samples were used for western blot analysis by standard methods. Antibodies used for western blot analysis were from Cell Signaling Technology (Danvers, MA): pERK1/2 (T202/204) (catalog #4370), ERK1/2 (catalog #4695), and HSP90 (catalog #4874).

### Results

### V_{L}-HC fusion and C_{L} expression in trans solves cognate light chain pairing problem for BsIgG

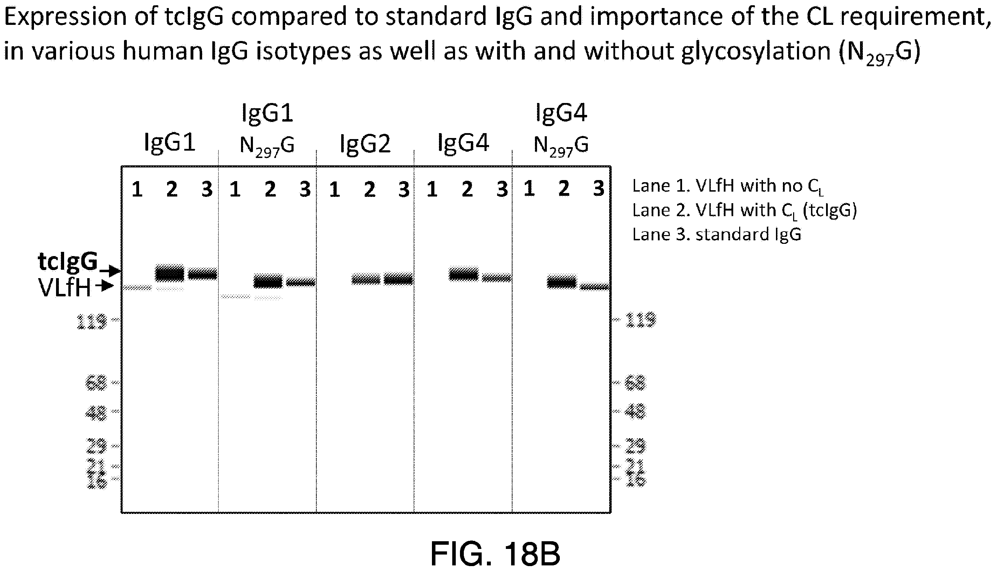

A schematic diagram for the novel tri-chain BsIgG (tcBsIgG) format is shown in Fig. 18A. In this novel platform, the heterodimerization of the heavy chains was accomplished by the previously described "knobs-into-holes" mutations (Ridgway, 1996; Atwell, 1997). In order to design V_{L}-HC fusion (VLfH) and achieve correct heavy-light chain pairing, a short (Gly₄Ser)4-linker was used to tether the C-terminal end of the antibody V_{L} domain to the N-terminus of the heavy chain, similar to the design of an antibody scFv (Figure 18A, left). The expression of VLfH alone without the C_{L} in HECK293 cells resulted in only trace amount of or no VLfH expression based on capillary electrophoresis (CE-SDS) after protein A affinity column purification recovery (Figure 18B, lane 1). The previous observation that the folding of the antibody CH1 domain necessitates pairing with the C_{L} domain was attributed to this result (Feige, 2009). The pairing with the C_{L} domain was proposed to serve as final quality control step and ensures that only properly folded antibody is secreted (Feige, 2014). To test this, the inventors sought to determine if an isolated C_{L} domain can complement the folding of the CH1 domain and allow productive secretion of the VLfH hybrid protein (Figure 18A, right) resulting in tri-chain IgG (tcIgG). Anti-KLB antibody, clone 28B7 monoepitopic tcIgG were made in the isotypes of IgG1, 2 and 4 as well as the aglycosylated (N₂₉₇G) version of IgG1 and IgG4. After co-transfection of a separate plasmid encoding for the C_{L} domain, antibody was successfully recovered in yields that were comparable to the intact, parental antibody (Figure 18B). While BsIgG of human IgG1 isotype were the predominant antibody class for the current clinical development, other isotypes were also commonly leveraged utilized as a monospecific antibody to modulate antibody effector function and antibody activity. Thus, whether the tcIgG technology could translate to other therapeutically relevant human isotypes IgG2 and IgG4 were evaluated. The requirement of the C_{L} domain was consistent across expression of VLfH in IgG2 and IgG4 and the common aglycosylated version of the IgG1 and IgG4 with the N297G mutation behaved similarly (Figure 18B). Overall yields after C_{L} co-expression were comparable to the respective wild-type IgG isotype controls. By electrophoretic analysis, it was demonstrated that the inter-chain disulfide bond between C_{L} and CH1 was efficiently formed. Thus, for productive folding and secretion of an antibody, the antibody V_{L} and C_{L} domains do not need to be covalently connected as a single polypeptide chain.

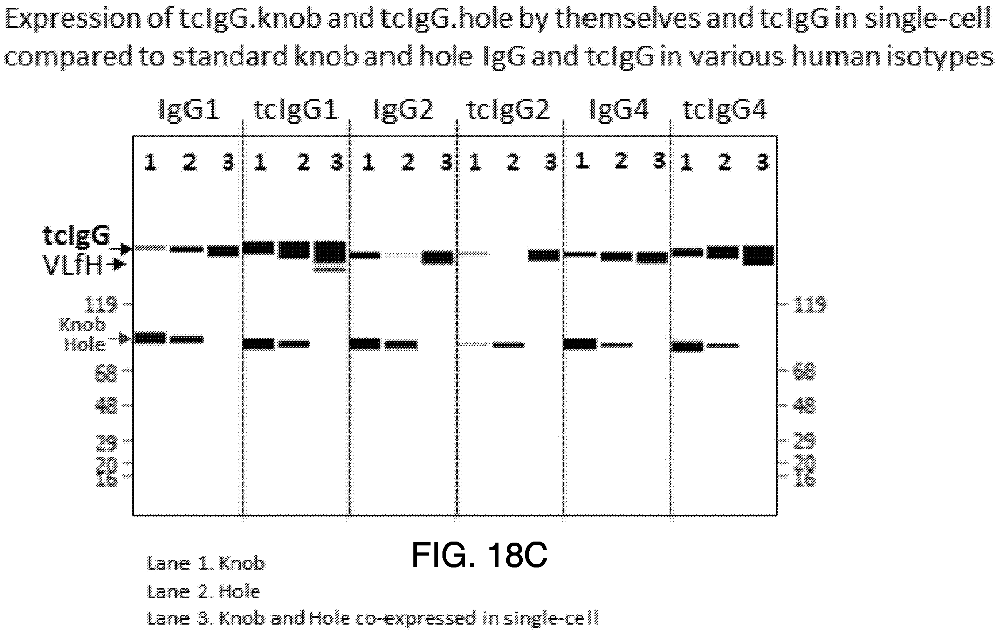

Next, whether the tcIgG format is compatible with the knobs-into-holes mutations was assessed for potential production of bispecific antibodies. The expression and assembly of knob and hole half IgGs were compared either by themselves or when co-expressed. As observed previously, expression of just the knob or hole by itself resulted predominantly in half-antibody and some covalent homodimer (Figure 18C). No major differences in expression or assembly were observed between a standard IgG and the tcIgG format. The co-expression of knob and hole half-antibodies in the same cell resulted in efficient assembly of the 150 KDa species in all three major human isotypes of IgG1, IgG2 and IgG4 (Figure 18C). The observed yields and product quality of human tcIgG2 and tcIgG4 as both monospecific bivalent tcIgG as well as bispecific tcIgG were comparable to their respective IgG isotypes.

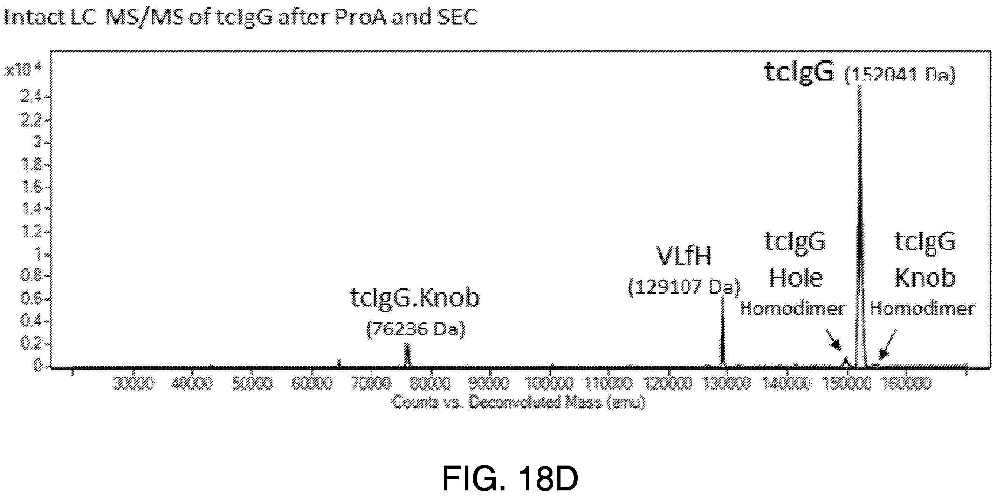

To ensure that the single-cell expression of the tcIgG knob and tcIgG hole does not impact heterodimer formation of the antibody heavy chains, the purified antibody was analyzed by mass spectrometry (Figure 18D). Only minor homodimer contaminants were detected. Because their abundance is comparable to the traditional BsIgG, the inventors conclude that the tethered VLfH format with an isolated C_{L} domain, here termed the tri-chain IgG (tcIgG) format, does not negatively impact the heterodimer formation.

### Production of anti-KLB biepitopic antibodies in the tcBsIgG format

In order to produce biepitopic antibodies derived from the five anti-KLB monoclonal antibodies described above, each antibody was first cloned into the VLfH knob and VLfH hole vectors. This enabled the production of 25 different antibodies in the tcIgG format (Table 8), including 10 possible biepitopic combinations in both heavy chain orientations (i.e. knob/hole and hole/knob). Having both of the knob-hole orientations for each antibody combination provided independent replicates. In addition, parental monospecific antibodies were generated in the tcIgG knob and tcIgG.hole co-expression format (Table 8, grey cells) to serves as a benchmark and included a positive (Table 8; (+) Exp Ctrl) and a negative (Table 8; (-) Exp Ctrl) knob and hole tcIgG expression controls to be paired with the five anti-KLB monoclonal antibodies.

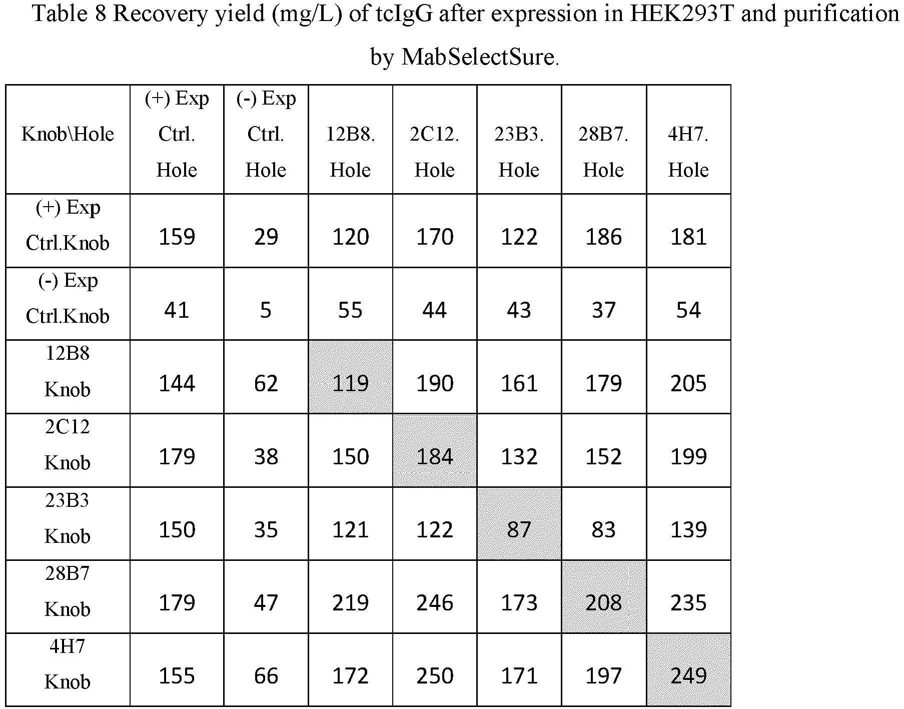

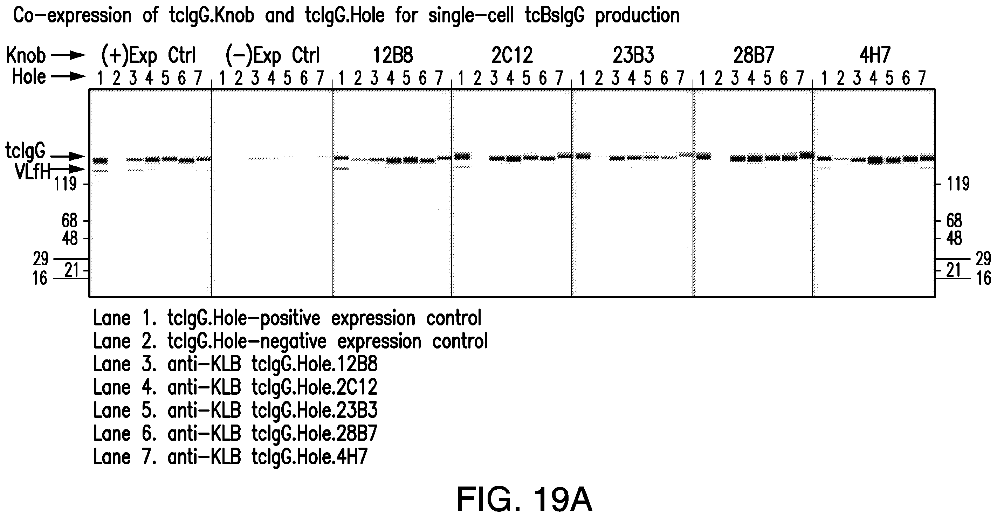

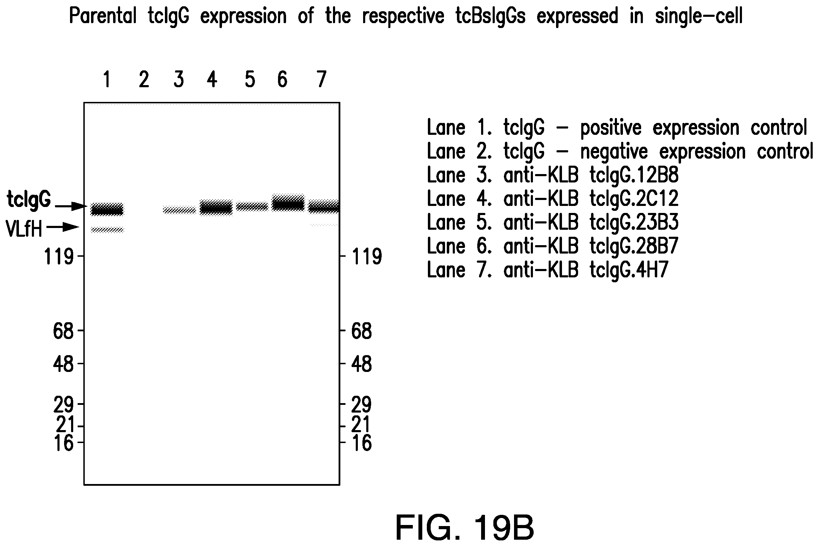

All 25 tcIgG antibodies were characterized by caliper electrophoresis and demonstrated comparable assembly efficiency. The predominant band for all antibodies was around ~ 150 kDa indicating correct domain pairing and disulfide bond formation (Figure 19A). The inventors noticed that the inefficient expression of a parental antibody also limited the expression yield of the resulting biepitopic antibodies. This is a consequence of the efficient heterodimerization by the knobs-into-holes mutations. For example, the negative expression control tcIgG had low yields when expressed as bivalent as well as when co-expressed with any other antibody (Figure 19A and 19B). At the same time this ensured minimal homodimer formation as verified by mass spectrometric analysis.

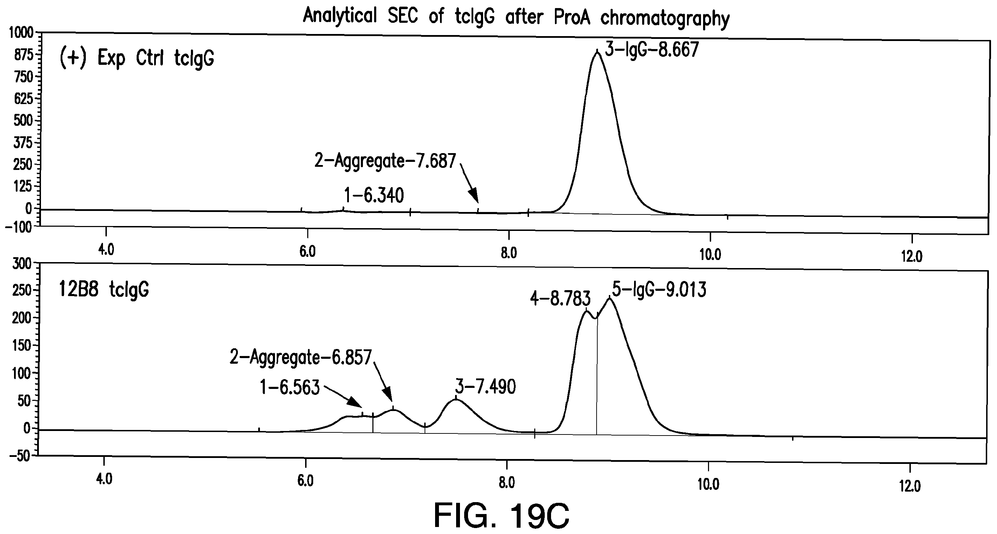

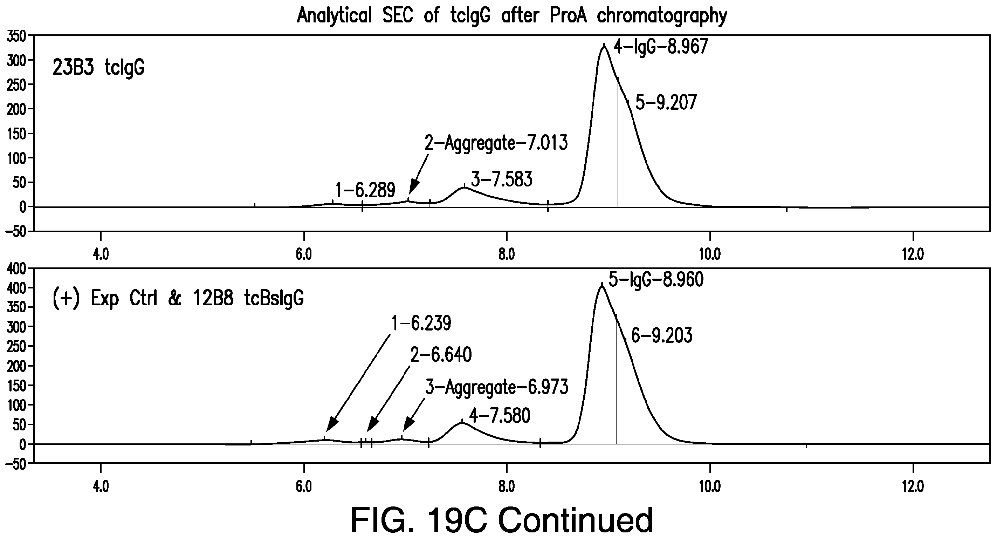

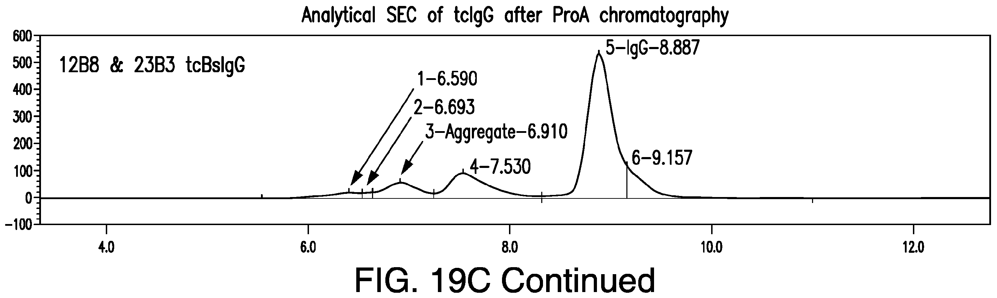

To further characterize the tcIgGs, they were analyzed by analytical size-exclusion chromatography (SEC). After HTP expression and single column purification by protein A chromatography the tcIgGs had high molecular weight (HMW) species ranging from 1% to 70 % which appeared to be related to the properties of the parental tcIgGs that is also correlated to their parental standard IgGs. The positive expression control tcIgG co-expressed as knob and hole tcIgG had no HMW species but the two relatively low expressing anti-KLB tcIgGs, 12B8 and 23B3 had much higher amounts of HMW species (Figure 19C). Interestingly, the amounts of the HMW species were averaged out when a well behaved tcIgG half antibody was co-expressed with poorly behaved tcIgG half antibody as in the case with the co-expression of the positive expression control tcIgG and 12B8 tcIgG as well as the co-expression of the poorly behaved 12B8 tcIgG and slightly better behaved 23B3 tcIgG (Figure 19C). Although HMW could affect the activity of each antibody, the inventors decided to proceed with characterization of the 25 antibodies in the luciferase reporter assay. An additional column step would reduce the throughput and the inventors decided to later validate the activity with more purified materials.

### In vitro screening of anti-KLB tcBsIgG biepitopic antibodies identifies 5 superior pairs that translate to linkerless BsIgG

In vitro screening of anti-KLB tcBsIgG biepitopic antibodies was described in Example 2. Five superior pairs that translate to linkerless BsIgG were identified: 12B8/23B3, 12B8/2C12, 23B3/28B7, 2C12/28B7 and 4H7/28B7 (Figures 3-6).

To ensure the linker in the tcIgG format did not interfere with the binding affinity of the biepitopic tcIgG during the screen, binding kinetics of antibodies 4H7 and 28B7 with the highest relative agonistic activity was assessed by Biacore as both tcIgG1 as well as standard IgG1. No significant differences were observed in binding kinetics for the two formats, indicating that the tcIgG format preserves the binding kinetics of the parental antibody (Table 9).

**Table 9 Binding kinetics of anti-KLB antibodies 28B7 and 4H7 as IgG and tcIgG format.**

| Values are mean ± range of two independent Biacore experiments. | | | |
|---|---|---|---|
| Antibody clone and format | Kₒₙ/10⁵ (M⁻¹ s⁻¹) | k_{off}/10⁻⁴ | K_{d} (nM) |
| 28B7 IgG | 1.044 .044for | 2.76 ± 0.08 | 2.65 ± 0.14 |
| 28B7 tcIgG | 1.172 ± 0.019 | 2.44 ± 0.09 | 2.06 ± 0.11 |
| 4H7 IgG | 5.710 ± 0.176 | 4.44 ± 0.13 | 0.78 ± 0.05 |
| 4H7 tcIgG | 3.322 ± 0.092 | 4.27 ± 0.07 | 1.29 ± 0.06 |

### Impact of antibody isotype on antibody agonist activity

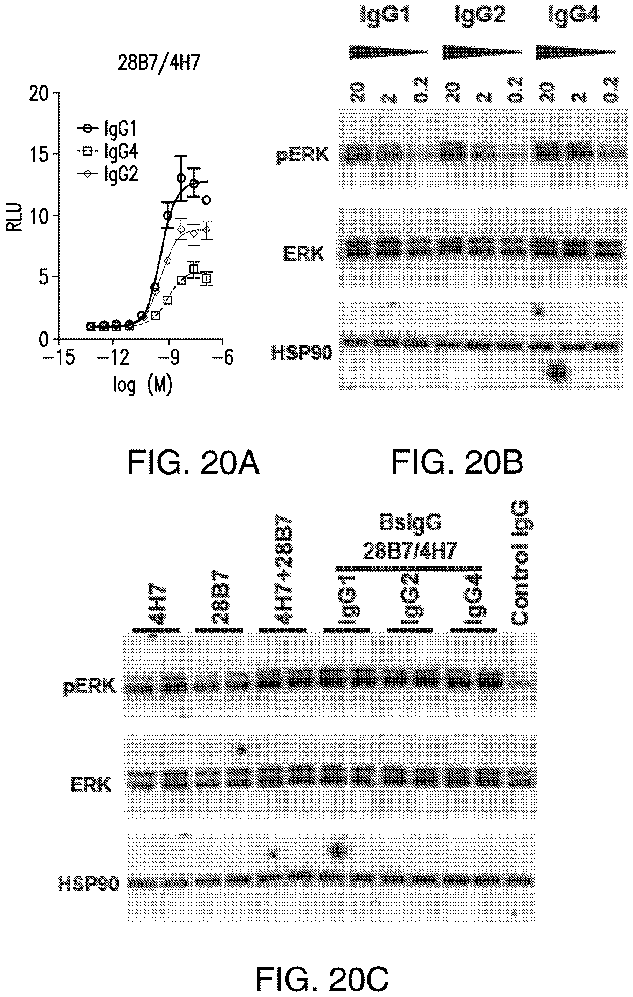

It has been reported previously that the antibody isotype can modulate the agonist and ligand mimetic activity of antibodies (White, 2014; Sampei, 2014). Consequently, the inventors explored whether the antibody isotype can affect the activity of the 4H7/28B7 biepitopic antibody, which had the highest agonistic property relative to other biepitopic pairs. The antibody as human IgG1, IgG2 and IgG4 isotypes were produced (in-vitro assembly) and compared the activity of the different isotypes in the luciferase reporter assay (Figure 20A). For the human IgG2 construct, the second hinge cysteine (C233 Kabat numbering, the same as C220S EU numbering) was mutated to serine to enable most efficient in-vitro assembly. In this assay, the activity of the 4H7/28B7 biepitopic antibody as IgG1 isotype was approximately twice of that of IgG2 isotype, while IgG4 isotype had an intermediate level of activity. The EC50 of these three antibodies were similar, consistent with the idea that the antibody isotype does not affect binding affinity.

Next, the evaluation extended to assessing ERK1/2 phosphorylation in human primary adipocytes. For this assay, differentiated primary human adipocytes were treated with various monoclonal antibodies, and the level of ERK1/2 phosphorylation was assessed by western blotting. The results of this assay using primary cells were consistent with the luciferase reporter assay; the 4H7/28B7 biepitopic antibody acts as an agonist in any one of IgG1, IgG2 and IgG4 isotype background (Figure 20B and 20C). Furthermore, 4H7 and 28B7 antibodies exhibited superior activity when combined as a 1:1 mixture or a single molecule biepitopic antibody as compared with the single parental molecule activity.

### Discussion

It has been demonstrated previously that bispecific and biepitopic antibodies can serve as potent ligand mimetics. However, finding these antibodies is often difficult, necessitating screening of up to thousands of combinations to identify a unique functional combination (Zhang, 2012; Kitazawa, 2012; Kolumam, 2015). The tcBsIgG format described here provides a novel system to simplify expression and production of BsIgG, enabling high throughput screening of a large panel of BsIgG combinations. Using this strategy, it was demonstrated that the activity of agonist antibodies can be further potentiated by co-formulation or combination into a biepitopic antibody. The biepitopic combinations that exhibited enhanced activity could not be fully anticipated from the characterization of parental antibodies, demonstrating the utility of the tcBsIgG system. The observation could be extended to other agonist antibodies, for example that target OX40, CD27, or GITR and lead to immunostimulatory activies.

The tcBsIgG format contains alteration in the antibody structure, in particular the addition of a linker and of a neoepitope at the CL N-terminus that may elicit immunogenic reactions *in vivo.* However, these issues are of no concern for *in vitro* screening activities. Once a few promising antibody pairs are identified, a selected number of BsIgG can be reformatted in a conventional BsIgG format for downstream preclinical and clinical development. To this point, it is critical for any screening format that results obtained can be recapitulated in a conventional IgG format. The tcIgG format described herein preserves the overall architecture of the antibody, and enables translation of results to a linker-less BsIgG that is more desirable for clinical applications. This is an advantage over other technologies such as fusions of scFv to a hetero-dimeric Fc (Moore, 2011) that may provide a similar throughput in producing bispecific molecules like the tcBsIgG format, but alter the geometry and distance between the target arms. In addition, another advantage of the tcBsIgG format over other BsIgG formats is that a fewer total number of plasmids (i.e. only one plasmid for each half antibody) needs to be cloned to produce a matrix of bispecific antibodies. Beyond screening of antibodies in drug development, the tcIgG format has a potential to produce bispecific antibodies for diagnostic applications. The ability to produce the tcIgG in a single cell provides a cost-efficient way to produce BsIgG.

Another advantage of the tcIgG format is its applicability to IgG2 and IgG4 isotypes. Antibody isotype-specific activities have been reported. For example, it has been reported previously that human IgG2 antibodies against CD40 displayed superagonist activity over human IgG1 and IgG4 isotypes (White, 2014). In addition, it has been reported that the amplitude of Factor VIII ligand mimetic activity of a bispecific antibody towards Factors IXa/X was dependent on the antibody class (Sampei, 2014). Based on these observations, the agonist activity of the 4H7/28B7 lead biepitopic IgG as human IgG1, IgG2 and IgG4 isotype was compared. While significant differences were observed between antibody isotypes, human IgG1 displayed the best agonist activity. This may be partially attributed to the fact that the anti-KLB pairs as human IgG1 were initially screened. Potentially, a different pair and isotype may have been selected if the initial screen was done using BsIgG of all isotypes. The compatibility of the tsIgG format with other human isotypes would enable this screening. In addition, it enables the leverage of different engagement with Fc gamma receptors to form ternary complexes. While this has no relevance in the anti-KLB model system, this might be important for tumor-targeting agonist antibody discovery.

In conclusion, the tcBsIgG format provides an excellent strategy for BsIgG screening for broad applications.

### Example 7: Binding affinity tests

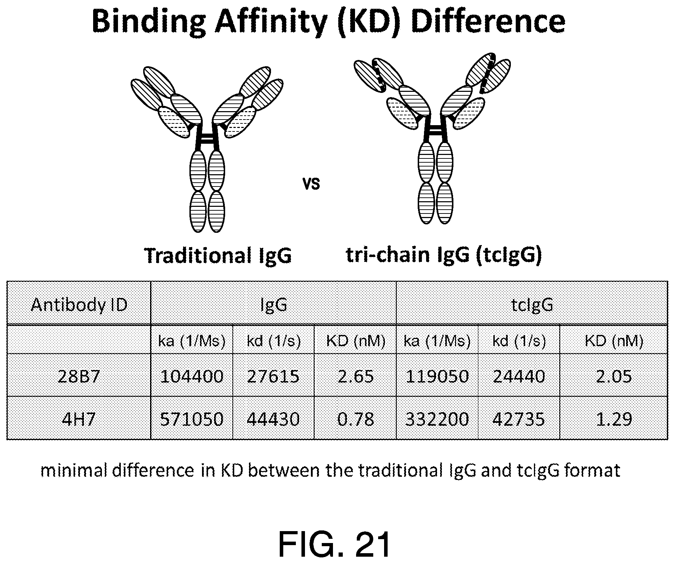

In this Example, the disclosed bispecific antibodies were further analyzed to determine their biding affinities. Binding affinity parameters (ka (1/Ms), kd (1/s) and KD (nM)) of anti-KLB antibody clones 28B7 and 4H7 were determined in both IgG format and in tcIgG format (Figure 21). Within each antibody clone, the difference of binding affinity was minimal between the traditional IgG format and the tcIgG format. For example, the KDs of the IgG format and tcIgG format of clone 28B7 were 2.65 nM and 2.05 nM, respectively. The KDs of the IgG format and the tcIgG format of clone 4H7 were 0.78 nM and 1.29 nM, respectively.

### References

Atwell, S., Ridgway, J. B., Wells, J. A. and Carter, P. (1997) J Mol Biol,270 26-35.
Feige, M. J. and Buchner, J. (2014) Biochim. Biophys. Acta,1844 2024-2031.
Feige, M. J., Groscurth, S., Marcinowski, M., Shimizu, Y., Kessler, H.,Hendershot, L.
M. and Buchner, J. (2009) Mol Cell,34 569-579.
Kim, HS. (2016) MAbs,8 1536-1547.
Kitazawa, T. et al. (2012) Nat Medicine,18 1570-1574.
Kolumam, G. et al. (2015) EBioMedicine,2 730-743*.*
Moore, G. L. et al. (2011) MAbs,3 546-557.
Ridgway, J. B., Presta, L. G. and Carter, P. (1996) Protein Eng.,9 617-621.
Sampei, Z. et al. (2014) MAbs 0-00*.*
White, A. L. et al. (2014) Cancer Cell 1-25.
Zhang, H., Wilson, I. A. and Lerner, R. A. (2012) Proc. Natl. Acad. Sci. U.S.a.,109 15728-15733.

## Claims

1. An isolated multispecific antibody consisting of
(i) a first antigen-binding polypeptide, wherein the first antigen-binding polypeptide comprises a VL domain, a linker, a VH domain, a CH1 domain, a CH2 domain and a CH3 domain positioned in an N-terminal to C-terminal direction in the following sequence VL-linker-VH-CH1-CH2-CH3;
(ii) a second antigen-binding polypeptide, wherein the second antigen-binding polypeptide comprises a VL domain, a linker, a VH domain, a CH1 domain, a CH2 domain and a CH3 domain positioned in an N-terminal to C-terminal direction in the following sequence VL-linker-VH-CH1-CH2-CH3; and
(iii) two identical CL domains, wherein one of the two CL domains is linked to the CH1 domain of the first antigen-binding polypeptide by one or more disulfide bridges, and the second of the two CL domains is linked to the CH1 domain of the second antigen-binding polypeptide by one or more disulfide bridges;
wherein the first and second antigen-binding polypeptides bind two different antigens, or the first and second antigen-binding polypeptides bind to two different epitopes of the same antigen.

2. The multispecific antibody of claim 1, wherein the linker
(i) comprises one or more glycine (G) and serine (S) residues;
(ii) has a length from about 1 to about 50 amino acids;
(iii) is about 20 amino acids in length;
(iv) comprises G₄S repeats;
(v) comprises the amino acid sequence of SEQ ID NO: 68; and/or
(vi) is cleavable.

3. The multispecific antibody of claims 1 or 2, wherein the CH3 domain of the first antigen-binding polypeptide and the CH3 domain of the second antigen-binding polypeptide meet at an interface that is altered to promote the formation of a multispecific antibody; wherein optionally
(a) one or more amino acid residues of the CH3 domain of the first antigen-binding polypeptide is replaced with one or more amino acid residues having a larger side chain volume to generate a protuberance on a surface of the CH3 domain of the first antigen-binding polypeptide that interacts with the CH3 domain of the second antigen-binding polypeptide; and (b) one or more amino acid residues of the CH3 domain of the second antigen-binding polypeptide is replaced with one or more amino acid residues having a smaller side chain volume to generate a cavity on the surface of a CH3 domain of the second antigen-binding polypeptide that interacts with the CH3 domain of the first antigen-binding polypeptide; wherein optionally the amino acid residue having a larger side chain volume is selected from the group consisting of arginine (R), phenylalanine (F), tyrosine (Y) and tryptophan (W), and/or the amino acid residue having a smaller side chain volume is selected from the group consisting of alanine (A), serine (S), threonine (T) and valine (V).

4. The multispecific antibody of any one of claims 1-3, wherein the multispecific antibody is an IgG, IgA or IgE isotype, particularly an IgG isotype, more particularly an IgG₁, IgG₂ or IgG₄ isotype.

5. The multispecific antibody of any one of claims 1-4, wherein the multispecific antibody is an agonist multispecific antibody or an antagonist multispecific antibody.

6. The multispecific antibody of any one of claims 1-5, wherein the multispecific antibody is a bispecific antibody.

7. The multispecific antibody of claim 6, wherein the bispecific antibody is an agonistic biepitopic antibody.

8. An isolated nucleic acid comprising a polynucleotide sequence that encodes the multispecific antibody of any one of claims 1-7.

9. A vector comprising the nucleic acid of claim 8.

10. A host cell that expresses the multispecific antibody of any one of claims 1-7, and/or comprises the nucleic acid of claim 8 or the vector of claim 9.

11. A method of producing a multispecific antibody comprising culturing the host cell of claim 10 under conditions sufficient for producing the multispecific antibody, and optionally further comprising recovering the multispecific antibody from the culture.

12. A pharmaceutical composition comprising the multispecific antibody of any one of claims 1-7 and a pharmaceutically acceptable carrier, and optionally further comprising a second therapeutic agent.

13. A library comprising a plurality of the multispecific antibody of any one of claims 1-7, or a plurality of polynucleotides encoding a plurality of the multispecific antibody of any one of claims 1-7.

14. A method of screening for a multispecific antibody comprising:
(a) obtaining a plurality of multispecific antibodies from the library of claim 13;
(b) assaying for binding of the plurality of multispecific antibodies to a first and second antigen or a first and second epitope of the same antigen; and
(c) identifying the multispecific antibody that binds to the first and second antigen or the first and second epitope of the same antigen.

15. A method for identifying a multispecific antibody comprising:
(a) expressing the multispecific antibody of any one of claims 1-7 in a cell;
(b) contacting the multispecific antibody of step (a) with a first antigen and a second antigen or a first epitope and a second epitope of the same antigen; and
(c) identifying the multispecific antibody that binds to the first antigen and the second antigen or the first epitope and the second epitope of the same antigen.

16. The method of claim 14 or 15, comprising
(a) identifying the multispecific antibody that binds to the first and second epitope of the same antigen to obtain a biepitopic antibody;
(b) determining whether the biepitopic antibody exhibits agonist activity to obtain a biepitopic agonist antibody; and optionally
(c) comparing the agonistic activity of the biepitopic agonist antibody to a monospecific antibody from which the biepitopic agonist antibody was derived.

17. The method of any one of claims 14-16, wherein
(i) expressing the multispecific antibody comprises introducing into the cell one or more nucleic acids encoding the multispecific antibody;
(ii) the multispecific antibody is contacted with the first and second antigens or the first and second epitope of the same antigen simultaneously in step (b);
(iii) the multispecific antibody of step (b) is purified, optionally by protein A chromatography, before contacting the multispecific antibody with the first and second antigens or the first and second epitopes of the same antigen; and/or
(iv) wherein the binding of the multispecific antibody to the first and/or second antigen or first and/or second epitope is analyzed by ELISA.

18. The method of any one of claims 14-17, wherein the antigen is in a biological complex.

19. The method of any one of claims 14-18, wherein the cells are prokaryotic cells, particularly wherein the prokaryotic cells are Escherichia coli cells.

20. The method of any one of claims 14-18, wherein the cells are eukaryotic cells, particularly wherein the eukaryotic cells are yeast cells or mammalian cells, and particularly wherein the mammalian cells are Chinese hamster ovary cells.

21. An isolated biepitopic agonist antibody consisting of
(i) a first antigen-binding polypeptide, wherein the first antigen-binding polypeptide comprises a VL domain, a linker, a VH domain, a CH1 domain, a CH2 domain and a CH3 domain positioned in an N-terminal to C-terminal direction in the following sequence VL-linker-VH-CH1-CH2-CH3;
(ii) a second antigen-binding polypeptide, wherein the second antigen-binding polypeptide comprises a VL domain, a linker, a VH domain, a CH1 domain, a CH2 domain and a CH3 domain positioned in an N-terminal to C-terminal direction in the following sequence VL-linker-VH-CH1-CH2-CH3; and
(iii) two identical CL domains, wherein one of the two CL domains is linked to the CH1 domain of the first antigen-binding polypeptide by one or more disulfide bridges, and the second of the two CL domains is linked to the CH1 domain of the second antigen-binding polypeptide by one or more disulfide bridges;
wherein the first and second antigen-binding polypeptides bind to two different epitopes on the same antigen;
identified and/or produced using the method of any one of claims 14-20.

## Patentansprüche

1. Isolierter multispezifischer Antikörper, bestehend aus
(i) einem ersten antigenbindenden Polypeptid, wobei das erste antigenbindende Polypeptid eine VL-Domäne, einen Linker, eine VH-Domäne, eine CH1-Domäne, eine CH2-Domäne und eine CH3-Domäne umfasst, die in einer N-terminalen zu C-terminalen Richtung in der folgenden Reihenfolge positioniert sind VL-Linker-VH-CH1-CH2-CH3;
(ii) einem zweiten antigenbindenden Polypeptid, wobei das zweite antigenbindende Polypeptid eine VL-Domäne, einen Linker, eine VH-Domäne, eine CH1-Domäne, eine CH2-Domäne und eine CH3-Domäne umfasst, die in einer N-terminalen zu C-terminalen Richtung in der folgenden Reihenfolge positioniert sind VL-Linker-VH-CH1-CH2-CH3; und
(iii) zwei identischen CL-Domänen, wobei eine der beiden CL-Domänen über eine oder mehrere Disulfidbrücke(n) mit der CH1-Domäne des ersten antigenbindenden Polypeptids verknüpft ist und die zweite der beiden CL-Domänen über eine oder mehrere Disulfidbrücke(n) mit der CH1-Domäne des zweiten antigenbindenden Polypeptids verknüpft ist;
wobei das erste und zweite antigenbindende Polypeptid zwei verschiedene Antigene binden oder das erste und zweite antigenbindende Polypeptid an zwei verschiedene Epitope desselben Antigens binden.

2. Multispezifischer Antikörper nach Anspruch 1, wobei der Linker
(i) einen oder mehrere Glycin-(G-) und Serin-(S-)Rest(e) umfasst;
(ii) eine Länge von etwa 1 bis etwa 50 Aminosäuren hat;
(iii) etwa 20 Aminosäuren lang ist;
(iv) G₄S-Wiederholungen umfasst;
(v) die Aminosäuresequenz von SEQ ID NO:68 umfasst und/oder
(vi) spaltbar ist.

3. Multispezifischer Antikörper nach den Ansprüchen 1 oder 2, wobei sich die CH3-Domäne des ersten antigenbindenden Polypeptids und die CH3-Domäne des zweiten antigenbindenden Polypeptids an einer Grenzfläche treffen, die verändert ist, um die Bildung eines multispezifischen Antikörpers zu fördern; wobei gegebenenfalls
(a) ein oder mehrere Aminosäurerest(e) der CH3-Domäne des ersten antigenbindenden Polypeptids durch einen oder mehrere Aminosäurerest(e) mit einem größeren Seitenkettenvolumen ausgetauscht ist/sind, um eine Protuberanz auf einer Oberfläche der CH3-Domäne des ersten antigenbindenden Polypeptids zu erzeugen, die mit der CH3-Domäne des zweiten antigenbindenden Polypeptids interagiert; und (b) ein oder mehrere Aminosäurerest(e) der CH3-Domäne des zweiten antigenbindenden Polypeptids durch einen oder mehrere Aminosäurerest(e) mit einem kleineren Seitenkettenvolumen ausgetauscht ist/sind, um eine Vertiefung auf der Oberfläche einer CH3-Domäne des zweiten antigenbindenden Polypeptids zu erzeugen, die mit der CH3-Domäne des ersten antigenbindenden Polypeptids interagiert; wobei gegebenenfalls der Aminosäurerest mit einem größeren Seitenkettenvolumen aus der Gruppe ausgewählt ist, die aus Arginin (R), Phenylalanin (F), Tyrosin (Y) und Tryptophan (W) besteht, und/oder der Aminosäurerest mit einem kleineren Seitenkettenvolumen aus der Gruppe ausgewählt ist, die aus Alanin (A), Serin (S), Threonin (T) und Valin (V) besteht.

4. Multispezifischer Antikörper nach einem der Ansprüche 1-3, wobei der multispezifische Antikörper ein IgG-, IgA- oder IgE-Isotyp ist, insbesondere ein IgG-Isotyp, noch spezieller ein IgG₁-, IgG₂- oder IgG₄-Isotyp.

5. Multispezifischer Antikörper nach einem der Ansprüche 1-4, wobei der multispezifische Antikörper ein multispezifischer Agonisten-Antikörper oder ein multispezifischer Antagonisten-Antikörper ist.

6. Multispezifischer Antikörper nach einem der Ansprüche 1-5, wobei der multispezifische Antikörper ein bispezifischer Antikörper ist.

7. Multispezifischer Antikörper nach Anspruch 6, wobei der bispezifische Antikörper ein agonistischer Biepitop-Antikörper ist.

8. Isolierte Nukleinsäure, umfassend eine Polynukleotidsequenz, die für den multispezifischen Antikörper nach einem der Ansprüche 1-7 kodiert.

9. Vektor, umfassend die Nukleinsäure nach Anspruch 8.

10. Wirtszelle, die den multispezifischen Antikörper nach einem der Ansprüche 1-7 exprimiert und/oder die Nukleinsäure nach Anspruch 8 oder den Vektor nach Anspruch 9 umfasst.

11. Verfahren zum Produzieren eines multispezifischen Antikörpers, umfassend das Kultivieren der Wirtszelle nach Anspruch 10 unter Bedingungen, die zum Produzieren des multispezifischen Antikörpers ausreichen, und gegebenenfalls ferner umfassend das Gewinnen des multispezifischen Antikörpers aus der Kultur.

12. Pharmazeutische Zusammensetzung, umfassend den multispezifischen Antikörper nach einem der Ansprüche 1-7 und einen pharmazeutisch unbedenklichen Träger und gegebenenfalls ferner umfassend ein zweites Therapeutikum.

13. Bibliothek, umfassend eine Vielzahl der multispezifischen Antikörper nach einem der Ansprüche 1-7 oder eine Vielzahl von Polynukleotiden, die für eine Vielzahl der multispezifischen Antikörper nach einem der Ansprüche 1-7 kodieren.

14. Verfahren zum Screenen auf einen multispezifischen Antikörper, umfassend:
(a) Erhalten einer Vielzahl von multispezifischen Antikörpern aus der Bibliothek nach Anspruch 13;
(b) Untersuchen auf das Binden der Vielzahl von multispezifischen Antikörpern an ein erstes und zweites Antigen oder ein erstes und zweites Epitop desselben Antigens und
(c) Identifizieren des multispezifischen Antikörpers, der an das erste und zweite Antigen oder das erste und zweite Epitop desselben Antigens bindet.

15. Verfahren zum Identifizieren eines multispezifischen Antikörpers, umfassend:
(a) Exprimieren des multispezifischen Antikörpers nach einem der Ansprüche 1-7 in einer Zelle;
(b) Inkontaktbringen des multispezifischen Antikörpers von Schritt (a) mit einem ersten Antigen und einem zweiten Antigen oder einem ersten Epitop und einem zweiten Epitop desselben Antigens und
(c) Identifizieren des multispezifischen Antikörpers, der an das erste Antigen und das zweite Antigen oder das erste Epitop und das zweite Epitop desselben Antigens bindet.

16. Verfahren nach Anspruch 14 oder 15, umfassend
(a) Identifizieren des multispezifischen Antikörpers, der an das erste und zweite Epitop desselben Antigens bindet, um einen Biepitop-Antikörper zu erhalten;
(b) Bestimmen, ob der Biepitop-Antikörper Agonistenaktivität zeigt, um einen Biepitop-Agonisten-Antikörper zu erhalten; und gegebenenfalls
(c) Vergleichen der agonistischen Aktivität des Biepitop-Agonisten-Antikörpers mit einem monospezifischen Antikörper, von dem der Biepitop-Agonisten-Antikörper abgeleitet wurde.

17. Verfahren nach einem der Ansprüche 14-16, wobei
(i) das Exprimieren des multispezifischen Antikörpers das Einbringen einer oder mehrerer Nukleinsäuren, die für den multispezifischen Antikörper kodieren, in die Zelle umfasst;
(ii) der multispezifische Antikörper in Schritt (b) gleichzeitig mit dem ersten und zweiten Antigen oder dem ersten und zweiten Epitop desselben Antigens in Kontakt gebracht wird;
(iii) der multispezifische Antikörper von Schritt (b) gegebenenfalls durch Protein-A-Chromatographie gereinigt wird, bevor der multispezifische Antikörper mit dem ersten und zweiten Antigen oder dem ersten und zweiten Epitop desselben Antigens in Kontakt gebracht wird; und/oder
(iv) wobei das Binden des multispezifischen Antikörpers an das erste und/oder zweite Antigen oder das erste und/oder zweite Epitop mittels ELISA analysiert wird.

18. Verfahren nach einem der Ansprüche 14-17, wobei das Antigen in einem biologischen Komplex vorliegt.

19. Verfahren nach einem der Ansprüche 14-18, wobei die Zellen prokaryotische Zellen sind, insbesondere wobei die prokaryotischen Zellen Escherichia coli-Zellen sind.

20. Verfahren nach einem der Ansprüche 14-18, wobei die Zellen eukaryotische Zellen sind, insbesondere wobei die eukaryotischen Zellen Hefezellen oder Säugerzellen sind, und insbesondere wobei die Säugerzellen Ovarialzellen des chinesischen Hamsters sind.

21. Isolierter Biepitop-Agonisten-Antikörper, bestehend aus
(i) einem ersten antigenbindenden Polypeptid, wobei das erste antigenbindende Polypeptid eine VL-Domäne, einen Linker, eine VH-Domäne, eine CH1-Domäne, eine CH2-Domäne und eine CH3-Domäne umfasst, die in einer N-terminalen zu C-terminalen Richtung in der folgenden Reihenfolge positioniert sind VL-Linker-VH-CH1-CH2-CH3;
(ii) einem zweiten antigenbindenden Polypeptid, wobei das zweite antigenbindende Polypeptid eine VL-Domäne, einen Linker, eine VH-Domäne, eine CH1-Domäne, eine CH2-Domäne und eine CH3-Domäne umfasst, die in einer N-terminalen zu C-terminalen Richtung in der folgenden Reihenfolge positioniert sind VL-Linker-VH-CH1-CH2-CH3; und
(iii) zwei identischen CL-Domänen, wobei eine der beiden CL-Domänen über eine oder mehrere Disulfidbrücke(n) mit der CH1-Domäne des ersten antigenbindenden Polypeptids verknüpft ist und die zweite der beiden CL-Domänen über eine oder mehrere Disulfidbrücke(n) mit der CH1-Domäne des zweiten antigenbindenden Polypeptids verknüpft ist;
wobei das erste und das zweite antigenbindende Polypeptid an zwei verschiedene Epitope auf demselben Antigen binden;
der unter Anwendung des Verfahrens nach einem der Ansprüche 14-20 identifiziert und/oder produziert wird.

## Revendications

1. Anticorps multispécifique isolé constitué par
(i) un premier polypeptide de liaison à l'antigène, dans lequel le premier polypeptide de liaison à l'antigène comprend un domaine VL, un lieur, un domaine VH, un domaine CH1, un domaine CH2 et un domaine CH3 positionnés dans le sens de l'extrémité N-terminale à l'extrémité C-terminale dans la séquence suivante VL-lieur-VH-CH1-CH2-CH3 ;
(ii) un second polypeptide de liaison à l'antigène, dans lequel le second polypeptide de liaison à l'antigène comprend un domaine VL, un lieur, un domaine VH, un domaine CH1, un domaine CH2 et un domaine CH3 positionnés dans le sens de l'extrémité N-terminale à l'extrémité C-terminale dans la séquence suivante VL-lieur-VH-CH1-CH2-CH3 ; et
(iii) deux domaines CL identiques, dans lequel l'un des deux domaines CL est lié au domaine CH1 du premier polypeptide de liaison à l'antigène par un ou plusieurs ponts disulfure, et le second des deux domaines CL est lié au domaine CH1 du second polypeptide de liaison à l'antigène par un ou plusieurs ponts disulfure ;
dans lequel les premier et second polypeptides de liaison à l'antigène se lient à deux antigènes différents, ou les premier et second polypeptides de liaison à l'antigène se lient à deux épitopes différents du même antigène.

2. Anticorps multispécifique selon la revendication 1, dans lequel le lieur
(i) comprend un ou plusieurs résidus glycine (G) et sérine (S) ;
(ii) a une longueur d'environ 1 à environ 50 acides aminés ;
(iii) est d'environ 20 acides aminés de long ;
(iv) comprend des répétitions de G₄S ;
(v) comprend la séquence d'acides aminés de SEQ ID NO : 68 ; et/ou
(vi) est clivable.

3. Anticorps multispécifique selon les revendications 1 ou 2, dans lequel le domaine CH3 du premier polypeptide de liaison à l'antigène et le domaine CH3 du second polypeptide de liaison à l'antigène se rencontrent au niveau d'une interface qui est altérée pour favoriser la formation d'un anticorps multispécifique ; dans lequel éventuellement
(a) un ou plusieurs résidus d'acides aminés du domaine CH3 du premier polypeptide de liaison à l'antigène sont remplacés par un ou plusieurs résidus d'acides aminés ayant un volume de chaîne latérale plus important pour générer une protubérance sur une surface du domaine CH3 du premier polypeptide de liaison à l'antigène qui interagit avec le domaine CH3 du second polypeptide de liaison à l'antigène ; et (b) un ou plusieurs résidus d'acides aminés du domaine CH3 du second polypeptide de liaison à l'antigène sont remplacés par un ou plusieurs résidus d'acides aminés ayant un volume de chaîne latérale plus faible pour générer une cavité sur la surface d'un domaine CH3 du second polypeptide de liaison à l'antigène qui interagit avec le domaine CH3 du premier polypeptide de liaison à l'antigène ; dans lequel éventuellement le résidu d'acide aminé ayant un volume de chaîne latérale plus important est choisi dans le groupe constitué par l'arginine (R), la phénylalanine (F), la tyrosine (Y) et le tryptophane (W), et/ou le résidu d'acide aminé ayant un volume de chaîne latérale plus faible est choisi dans le groupe constitué par l'alanine (A), la sérine (S), la thréonine (T) et la valine (V).

4. Anticorps multispécifique selon l'une quelconque des revendications 1 à 3, dans lequel l'anticorps multispécifique est un isotype IgG, IgA ou IgE, en particulier un isotype IgG, plus particulièrement un isotype IgG₁, IgG₂ ou IgG₄.

5. Anticorps multispécifique selon l'une quelconque des revendications 1 à 4, dans lequel l'anticorps multispécifique est un anticorps multispécifique agoniste ou un anticorps multispécifique antagoniste.

6. Anticorps multispécifique selon l'une quelconque des revendications 1 à 5, dans lequel l'anticorps multispécifique est un anticorps bispécifique.

7. Anticorps multispécifique selon la revendication 6, dans lequel l'anticorps bispécifique est un anticorps biépitopique agoniste.

8. Acide nucléique isolé comprenant une séquence polynucléotidique qui code pour l'anticorps multispécifique selon l'une quelconque des revendications 1 à 7.

9. Vecteur comprenant l'acide nucléique selon la revendication 8.

10. Cellule hôte qui exprime l'anticorps multispécifique selon l'une quelconque des revendications 1 à 7 et/ou comprend l'acide nucléique selon la revendication 8 ou le vecteur selon la revendication 9.

11. Procédé de production d'un anticorps multispécifique comprenant la culture de la cellule hôte selon la revendication 10 dans des conditions suffisantes pour produire l'anticorps multispécifique, et éventuellement comprenant en outre la récupération de l'anticorps multispécifique de la culture.

12. Composition pharmaceutique comprenant l'anticorps multispécifique selon l'une quelconque des revendications 1 à 7 et un véhicule pharmaceutiquement acceptable, et éventuellement comprenant en outre un second agent thérapeutique.

13. Banque comprenant une pluralité des anticorps multispécifiques selon l'une quelconque des revendications 1 à 7, ou une pluralité de polynucléotides codant pour une pluralité des anticorps multispécifiques selon l'une quelconque des revendications 1 à 7.

14. Procédé de criblage pour un anticorps multispécifique comprenant :
(a) l'obtention d'une pluralité d'anticorps multispécifiques de la banque selon la revendication 13 ;
(b) le dosage de la liaison de la pluralité d'anticorps multispécifiques à un premier et un second antigène ou un premier et un second épitope du même antigène ; et
(c) l'identification de l'anticorps multispécifique qui se lie au premier et au second antigène ou au premier et au second épitope du même antigène.

15. Procédé d'identification d'un anticorps multispécifique comprenant :
(a) l'expression de l'anticorps multispécifique selon l'une quelconque des revendications 1 à 7 dans une cellule ;
(b) la mise en contact de l'anticorps multispécifique de l'étape (a) avec un premier antigène et un second antigène ou un premier épitope et un second épitope du même antigène ; et
(c) l'identification de l'anticorps multispécifique qui se lie au premier antigène et au second antigène ou au premier épitope et au second épitope du même antigène.

16. Procédé selon la revendication 14 ou 15, comprenant
(a) l'identification de l'anticorps multispécifique qui se lie au premier et au second épitope du même antigène pour obtenir un anticorps biépitopique ;
(b) la détermination si l'anticorps biépitopique présente une activité agoniste pour obtenir un anticorps agoniste biépitopique ; et éventuellement
(c) la comparaison de l'activité agoniste de l'anticorps agoniste biépitopique à un anticorps monospécifique à partir duquel l'anticorps agoniste biépitopique a été dérivé.

17. Procédé selon l'une quelconque des revendications 14 à 16, dans lequel
(i) l'expression de l'anticorps multispécifique comprend l'introduction dans la cellule d'un ou de plusieurs acides nucléiques codant pour l'anticorps multispécifique ;
(ii) l'anticorps multispécifique est mis en contact avec les premier et second antigènes ou les premier et second épitopes du même antigène simultanément dans l'étape (b) ;
(iii) l'anticorps multispécifique de l'étape (b) est purifié, éventuellement par chromatographie sur protéine A, avant mise en contact de l'anticorps multispécifique avec les premier et second antigènes et les premier et second épitopes du même antigène ; et/ou
(iv) dans lequel la liaison de l'anticorps multispécifique au premier et/ou au second antigène ou au premier et/ou au second épitope est analysée par ELISA.

18. Procédé selon l'une quelconque des revendications 14 à 17, dans lequel l'antigène est dans un complexe biologique.

19. Procédé selon l'une quelconque des revendications 14 à 18, dans lequel les cellules sont des cellules procaryotes, en particulier dans lequel les cellules procaryotes sont des cellules d'Escherichia coli.

20. Procédé selon l'une quelconque des revendications 14 à 18, dans lequel les cellules sont des cellules eucaryotes, en particulier dans lequel les cellules eucaryotes sont des cellules de levure ou des cellules de mammifère, et en particulier dans lequel les cellules de mammifère sont des cellules d'ovaires de hamster chinois.

21. Anticorps agoniste biépitopique isolé constitué par
(i) un premier polypeptide de liaison à l'antigène, dans lequel le premier polypeptide de liaison à l'antigène comprend un domaine VL, un lieur, un domaine VH, un domaine CH1, un domaine CH2 et un domaine CH3 positionnés dans le sens de l'extrémité N-terminale à l'extrémité C-terminale dans la séquence suivante VL-lieur-VH-CH1-CH2-CH3 ;
(ii) un second polypeptide de liaison à l'antigène, dans lequel le second polypeptide de liaison à l'antigène comprend un domaine VL, un lieur, un domaine VH, un domaine CH1, un domaine CH2 et un domaine CH3 positionnés dans le sens de l'extrémité N-terminale à l'extrémité C-terminale dans la séquence suivante VL-lieur-VH-CH1-CH2-CH3 ; et
(iii) deux domaines CL identiques, dans lequel l'un des deux domaines CL est lié au domaine CH1 du premier polypeptide de liaison à l'antigène par un ou plusieurs ponts disulfure, et le second des deux domaines CL est lié au domaine CH1 du second polypeptide de liaison à l'antigène par un ou plusieurs ponts disulfure ;
dans lequel les premier et second polypeptides de liaison à l'antigène se lient à deux épitopes différents sur le même antigène ;
identifié et/ou produit en utilisant le procédé selon l'une quelconque des revendications 14 à 20.
